(19) Europäisches Patentamt
European Patent Office
Office européen des brevets

(11) EP 1 155 698 A1

(12) # EUROPEAN PATENT APPLICATION
published in accordance with Art. 158(3) EPC

(43) Date of publication:
**21.11.2001 Bulletin 2001/47**

(21) Application number: **99959910.3**

(22) Date of filing: **20.12.1999**

(51) Int Cl.[7]: **A61K 31/69**, A61P 33/02, C07F 5/02

(86) International application number:
**PCT/JP99/07139**

(87) International publication number:
**WO 00/44387 (03.08.2000 Gazette 2000/31)**

(84) Designated Contracting States:
**AT BE CH CY DE DK ES FI FR GB GR IE IT LI LU MC NL PT SE**

(30) Priority: **29.01.1999 JP 2182299**

(71) Applicants:
- **Nitto Kasei Co., Ltd.**
  **Osaka-shi, Osaka 533-0031 (JP)**
- **SHIONOGI & CO., LTD.**
  **Osaka-shi, Osaka 541-0045 (JP)**

(72) Inventors:
- **IMAZAKI, Hideyuki**
  **Mishima-gun, Osaka 618-0024 (JP)**
- **FUJIKAWA, Masazumi**
  **Nara-shi, Nara 630-8024 (JP)**
- **HAYASE, Yoshio**
  **Kameyama-shi, Mie 519-0105 (JP)**
- **KAWAGUCHI, Harumoto**
  **Ayama-gun, Mie 519-1402 (JP)**

(74) Representative:
**Baverstock, Michael George Douglas et al**
**BOULT WADE TENNANT,**
**Verulam Gardens**
**70 Gray's Inn Road**
**London WC1X 8BT (GB)**

(54) **ORGANOBORON COMPOUNDS EXHIBITING ANTICOCCIDIAL ACTIVITIES**

(57) The present invention provides a pharmaceutical composition useful for animals except for humans, a pharmaceutical composition for use as an antiprotozoal agent, and a pharmaceutical composition for use as an anticoccidial agent, which comprises a compound of formula (I):

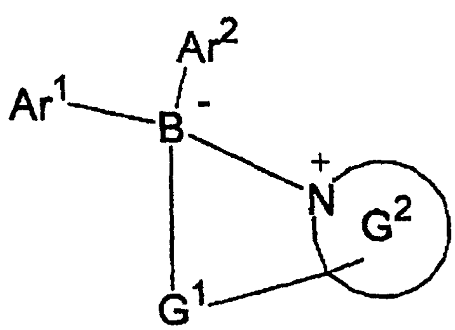

(I)

in which $Ar^1$ and $Ar^2$ are independently a cyclic group which may be optionally substituted;$G^1$ is a group of the formula: -A-, $-A-CR^1R^2-$ or $-A-CR^3R^4-CR^5R^6-$; $G^2$ is an azacyclic group which may be optionally substituted; provided that the nitrogen atom in the ring is bound to $B^-$; and the ring constituted with $B^-$, $G^1$ and $G^2$ is a 5- or 6-membered ring; or a salt thereof or a hydrate of them.

EP 1 155 698 A1

## Description

FIELD OF THE INVENTION

The invention belongs to the veterinary field, and relates to novel pharmaceutical compositions for animals except humans, antiprotozoal agents, and anticoccidial agents. Specifically, the invention relates to a composition comprising an organic boron-based compound, and the compound itself.

BACKGROUND ART

Coccidiosis is an infection disease caused by coccidia, a subclass of protozoa (Sporozoea of Apicomplexa class). A coccidia, *Eimeria tenella, Eimeria acervulina,* and *Eimeria necatrix* or the like infects mainly poultry, and induces various symptoms such as enterohemorrhage, death or growth inhibition in poultry. Outbreaks of widespread coccidiosis in poultry farms wherein poultry such as chicken and duck are commercially bred cause excessive losses, and are usually critical. Thus, there is an interest in anticoccidial agents useful for the prevention or treatment of coccidiosis.

RELATIVE ART AND PROBLEMS TO BE SOLVED BY THE INVENTION

Previously, sulfonamids, nitrofurans, quinolines, antithiamines, and benzamides have been in practical use as anticoccidial agents, and nowadays polyether antibiotics such as salinomycin and clopidol are primarily used. However, these agents are not so potent in anticoccidial effects, and are problematically toxic to hosts. Further, drug tolerant strains occur due to their long period use, and cause gradual decrease in potency. Such a situation brings about a demand to develop a new type of anticoccidial agent for poultry which is effective against drug tolerant strains and which resists drug tolerance.

As a result of an amount of research, the present inventors found organic boron-based compounds that overcome the drawbacks in the art as shown above, exhibit a good anticoccidial effect, and prevent outbreaks of coccidiosis.

For organic boron-based compounds, Farfan, Norberto et al., *J.Chem.Soc.*, Perkin Trans. 2 (1992), (4), 527-32 describes diphenyl (2-pyridylalkyloxy-O,N) borons and their preparation, and Vershbitskii, F. R. et al., *Term. Anal. Fazovye Ravnovesiya* (1985), 31-3 describes organic boron-based compounds intramolecularly coordinated. However, no use of these compounds is described therein. Further, Lin, Kai et al., *Yiyao Gongye* (1985), 16(11), 500-2 describes that (p-fluorophenyl) (o-methoxyphenyl) borinate and 8-hydroxyquinoline are reacted each other to give a boron-containing cyclic group compound, which has anti-tumor activities. However, any anticoccidial effect is not described therein.

<u>Solution for solving the problems</u>

Thus, the present invention encompasses a pharmaceutical composition useful for animals except for humans, an antiprotozoal or an anticoccidial, which comprises a compound of formula (I):

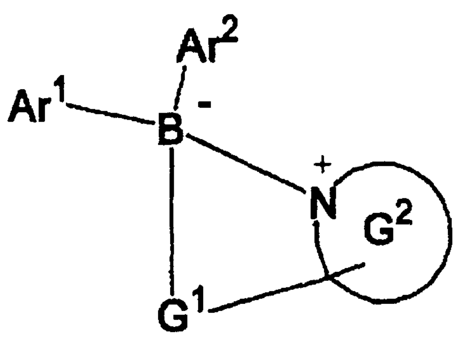

(I)

in which

$Ar^1$ and $Ar^2$ are independently a cyclic group which may be optionally substituted;
$G^1$ is a group of the formula: -A-, -A-$CR^1R^2$- or -A-$CR^3R^4$-$CR^5R^6$- wherein A is an oxygen atom or a sulfur atom, $R^1$-$R^6$ are the same or different, and are each a hydrogen atom, a halogen atom, hydroxy, an aliphatic hydrocarbon

group which may be optionally substituted, a-O- (aliphatic hydrocarbon group which may be optionally substituted), an acyloxy, a phenyloxy which may be optionally substituted, an amino group which may be optionally substituted, a sulfo group which may be optionally substituted, or an aryl which may be optionally substituted, or $R^1$ and $R^2$, $R^3$ and $R^4$ and/or $R^5$ and $R^6$ are combined together to form oxo, a methylene which may be optionally substituted, or an imino which may be optionally substituted; and

a group of the formula:

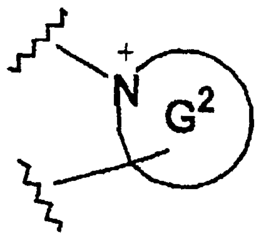

is an azacyclic group that may be optionally substituted;

provided that the smallest ring among rings constructed with $B^-$, $G^1$ and $G^2$ is a 5- or 6-membered ring; or a salt thereof or a hydrate of them.

In other aspects, the present invention encompasses the compounds as shown below of the compounds of formula (I):

1) A compound of formula (II):

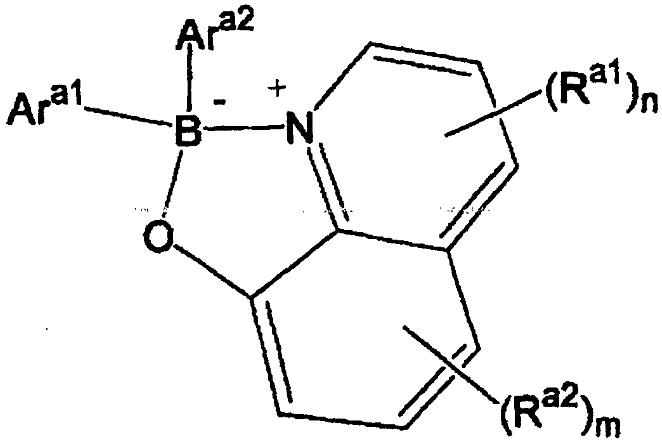

(II)

in which

$Ar^{a1}$ is an aryl which may be optionally substituted;
$Ar^{a2}$ is a fused aryl which may be optionally substituted;
$R^{a1}$ and $R^{a2}$ are independently hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted; and
n and m are the same or different, and are an integer of 0-3;

provided that when both n and m are 0, then a compound wherein both $Ar^{a1}$ and $Ar^{a2}$ are not naphthalen-2-yl substituted at the 1-position by naphthalen-1-yl; or a salt thereof or a hydrate of them;

2) A compound of formula (III):

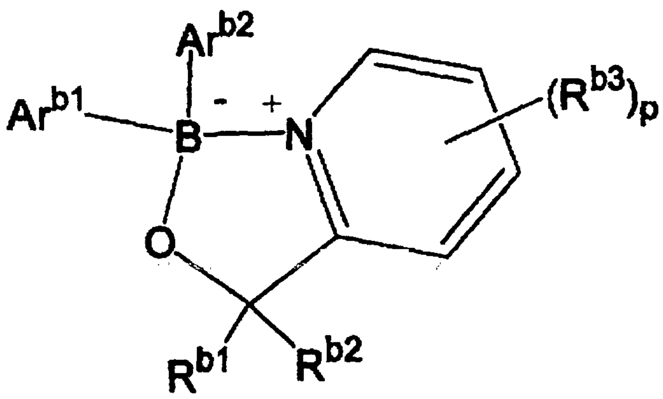

(III)

in which

$Ar^{b1}$ and $Ar^{b2}$ are the same or different, and are an aryl which may be optionally substituted;

$R^{b1}$ and $R^{b2}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{b1}$ and $R^{b2}$ are combined together to form an oxo, a methylene which may be optionally substituted or an imino which may be optionally substituted;

$R^{b3}$ is each a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be bonded with the adjacent group to form a fused ring; and

p is an integer of 0-4;

provided that when p is 0, both $R^{b1}$ and $R^{b2}$ are hydrogen, and $Ar^{b1}$ and $Ar^{b2}$ is a phenyl substituted with a halogen atom or a lower alkyl, then the phenyl of $Ar^{b1}$ and $Ar^{b2}$ is additionally substituted with a substituent other than a halogen atom or a lower alkyl; or a salt thereof or a hydrate of them;

3) A compound of formula (IV):

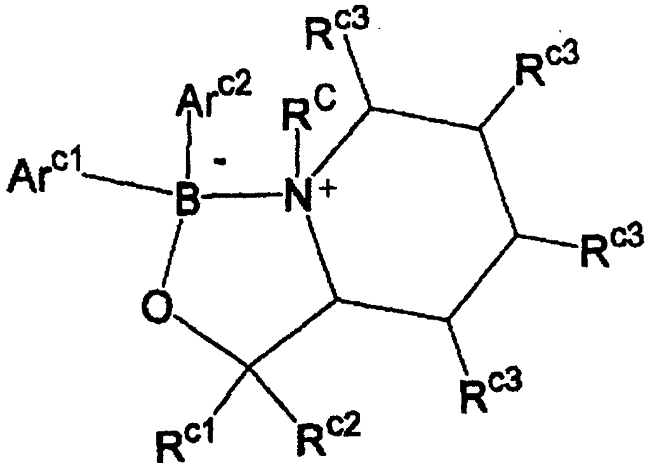

(IV)

in which

$Ar^{c1}$ and $Ar^{c2}$ are independently a cyclic group which may be optionally substituted;
$R^{c1}$ and $R^{c2}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{c1}$ and $R^{c2}$ are combined together to form an oxo, a methylene which may be optionally substituted or an imino which may be optionally substituted;
$R^{c3}$ is each a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be combined with the adjacent group to form a fused ring; and
$R^{c}$ is a hydrogen atom or a lower alkyl; or a salt thereof or a hydrate of them; and

4) A compound of formula (V):

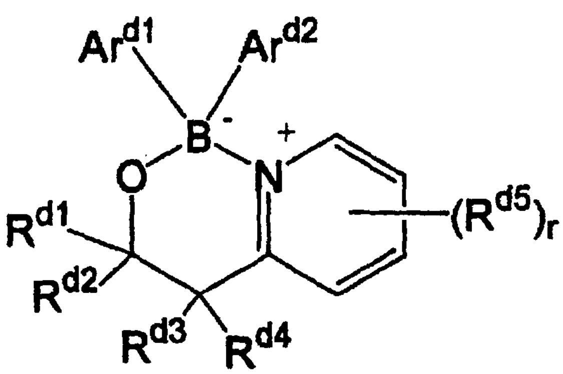

(V)

in which

$Ar^{d1}$ and $Ar^{d2}$ are the same or different, and are an aryl which may be optionally substituted;
$R^{d1}$, $R^{d2}$, $R^{d3}$, and $R^{d4}$ are independently a hydrogen atom, a halogen atom, a lower alkyl, a lower alkenyl, a halogenated lower alkyl, a halogenated lower alkenyl, an aralkyl which may be optionally substituted, hydroxy,

a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{d1}$ and $R^{d2}$ and/or $R^{d3}$ and $R^{d4}$ are combined together to form an oxo, a methylene which may be optionally substituted or an imino which may be optionally substituted;
$R^{d5}$ is each a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be combined with the adjacent group to form a fused ring; and
r is an integer of 0-4;

provided that when r is 0, all of $R^{d1}$, $R^{d2}$, $R^{d3}$ and $^{d4}$ are hydrogen, and $Ar^{d1}$ and $Ar^{d2}$ are phenyl substituted with a halogen atom or a lower alkyl, then the phenyl of $Ar^{d1}$ and $Ar^{d2}$ is additionally substituted with a substituent other than a halogen atom or a lower alkyl; or a salt thereof or a hydrate of them.

In the formulae, the term "a cyclic group which may be optionally substituted" represented by $Ar^1$ and $Ar^2$ refers to, for example, monocyclic or fused polycyclic alicyclic hydrocarbon groups (for example, a cycloalkyl which may be optionally substituted, a cycloalkenyl which may be optionally substituted, a cycloalkadienyl which may be optionally substituted, etc.), monocyclic or fused polycyclic aromatic hydrocarbon groups (for example, an aryl which may be optionally substituted, etc.), monocyclic or fused polycyclic heterocyclic groups (for example, an aromatic heterocyclic which may be optionally substituted, etc.), or spiro-cyclic hydrocarbon group or heterocyclic groups.
Preferably, $Ar^1$ and $Ar^2$ are the same.
Examples of cycloalkyl as shown above include, for example, a cycloalkyl having 3 to 20 carbon atoms such as cyclopropyl, cyclobutyl, cyclopentyl, cyclohexyl, cycloheptyl, cycloctyl, bicyclo[2.2.1]heptyl, bicyclo[2.2.2]octyl, bicyclo[3.2.1]octyl, bicyclo[3.2.2]nonyl, bicyclo[3.3.1]nonyl, bicycle[4.2.1]nonyl, bicyclo[4.3.1]decyl, and adamantyl, or the like.
Examples of cycloalkenyl as shown above include, for example, a cycloalkenyl having 4 to 20 carbon atoms such as 2-cyclopenten-1-yl, 3-cyclopenten-1-yl, 2-cyclohexen-1-yl, and 3-cyclohexen-1-yl or the like.
Examples of cycloalkadienyl as shown above include, for example, a cycloalkadienyl having 4 to 20 carbon atoms such as 2,4-cyclopentadien-1-yl, 2,4-cyclohexadien-1-yl, and 2,5-cyclohexadien-1-yl or the like.
Examples of "aryl" as shown above include, for example, an aryl having 6 to 20 carbon atoms such as phenyl, indenyl, naphthyl (1-naphthyl, 2-naphthyl, etc.), anthryl, phenanthryl, acenaphthylenyl, fluorenyl (9-fluorenyl, 1-fluorenyl, etc.), or the like.
The term "heterocyclic group" as shown above refers to a heterocyclic group containing at least one heteroatom of oxygen, sulfur, nitrogen as an atom constructing the ring, and include monocyclic heterocyclic groups or fused polycyclic heterocyclic groups.
Specific examples of monocyclic heterocyclic groups include isoxazolyl, isothiazolyl, imidazolyl, 1,2,3-oxadiazolyl, 1,3,4-oxadiazolyl, oxazolyl, furazanyl, 1,2,3-thiadiazolyl, 1,2,4-thiadiazolyl, 1,3,4-thiadiazolyl, thiazolyl, thienyl, tetrazolyl, triazinyl, 1,2,3-triazolyl, 1,2,4-triazolyl, pyranyl, pyrazinyl, pyrazolyl, pyridyl, pyridazinyl, pyrimidinyl, pyrrolyl, 2H-pyrrolyl, furazanyl, furyl or the like.
Specific examples of fused polycyclic heterocyclic groups include acridinyl, 5-azabenzo[a]anthracenyl, isoindolyl, isoquinolyl, isochromanyl, isobenzofranyl, imidazo[2,1-b]thiazolyl, 4H-imidazo[4,5-d]thiazolyl, imidazo[1,2-b][1,2,4]triazinyl, imidazo[1,2-a]pyridyl, imidazo[1,5-a]pyridyl, imidazo[1,2-b]pyridadinyl, imidazo[1,2-a]pyrimidinyl, imidazolidinyl, imidazolinyl, 1H-indazolyl, indolizinyl, indolyl, 4H[1,3]-oxathioro[5,4-b]pyrrolyl, 1H-2-oxapyrenyl, carbazolyl, α-carbolinyl, β-carbolinyl, γ-carbolinyl, quinazolinyl, quinoxalinyl, quinolyl, chromanyl, 4H-1,3-dioxolo[4,5-b]imidazolyl, cyclopenta[b]pyranyl, 2,3-dithia-1,5-diazaindanyl, 4H-1,3-dithianaphthalenyl, 1,4-dithianaphthalenyl, cinnonyl, thianthrenyl, thieno[2,3-b]franyl, 2,7,9-triazaphenanthrenyl, 1,2,4-triazolo[4,3-a]pyridadinyl, 1,2,4-triazolo[4,3-a]pyridyl, 2,4,6-trithia-3a,7a-diazaindanylnaphthyridinyl, piperazinyl, piperidyl, pyrazolidinyl, 7H-pyrazino[2,3-c]carbazolyl, pyrazino[2,3-d]pyridazinyl, 1H-pyrazolo[4,3-d]oxazolyl, pyrazolo[1,5-a]pyridyl, pyrido[1',2':1,2]imidazo[4,5-b]quinoxalinyl, 5H-pyrido[2,3-d]-o-oxazinyl, 4H-pyrido[2,3-c]carbazolyl, pyrrolidinyl, pyrrolinyl, lH-pyrrolo[1,2-b][2]benzazepinyl, pyrrolo[1,2-b]pyridazinyl, phenazinyl, phenathridinyl, phenathrolinyl, phenoxazinyl, phenoxthiynyl, phenothiazinyl, phthalazinyl, pteridinyl, purinyl, 2H-furo[3,2-b]pyranyl, furo[3,4-c]cinnolinyl, 1,2-benzoisoxazolyl, benzo[h]isoquinolyl, 1,2-benzoisothiazolyl, benzimidazolyl, benzoxazolyl, 4H-3,1-benzoxazinyl, 3-benzoxepinyl, benzothiazolyl, benzo[b]thienyl, 1H-benzotriazolyl, benzo[b]franyl, morpholinyl, or the like.
Preferred groups of "a cyclic group which may be optionally substituted" represented by $Ar^1$ and $Ar^2$ include an aryl which may be optionally substituted, and specifically an aryl which may be optionally substituted with a halogen

atom, a lower alkyl, a lower alkenyl, a lower alkoxy, a halogenated lower alkyl, a halogenated lower alkenyl, a halogenated lower alkoxy, hydroxy, an acyl, or an amino, or the like. The number of the substituents on an aryl or a heterocyclic group of $Ar^1$ and $Ar^2$ is not limited to one, and the species is not also limited to one.

Typical specific examples of an aryl which may be optionally substituted with one species of the substituent are described below.

The term "halogen atom" refers to fluorine, chlorine, bromine, or the like.

Thus, examples of an aryl substituted with the halogen atom include 2-halogenatedphenyl (for example, 2-fluorophenyl, 2-chlorophenyl, 2-bromophenyl, etc.), 3-halogenatedphenyl (for example, 3-fluorophenyl, 3-chlorophenyl, 3-bromophenyl, etc.), 4-halogenatedphenyl (for example, 4-fluorophenyl, 4-chlorophenyl, 4-bromophenyl, etc.), 2-halogenatednaphthalen-1-yl (for example, 2-fluoronaphthalen-1-yl, 2-chloronaphthalen-1-yl, 2-bromonaphthalen-1-yl, etc.), 3-halogenatednaphthalen-1-yl (for example, 3-fluoronaphthalen-1-yl, 3-chloronaphthalen-1-yl, 3-bromonaphthalen-1-yl, etc.), 4-halogenatednaphthalen-1-yl (for example, 4-fluoronaphthalen-1-yl, 4-chloronaphthalen-1-yl, 4-bromonaphthalen-1-yl, etc.), 5-halogenatednaphthalen-1-yl (for example, 5-fluoronaphthalen-1-yl, 5-chloronaphthalen-1-yl, 5-bromonaphthalen-1-yl, etc.), 6-halogenatednaphthalen-1-yl (for example, 6-fluoronaphthalen-1-yl, 6-chloronaphthalen-1-yl, 6-bromonaphthalen-1-yl, etc.), 7-halogenatednaphthalen-1-yl (for example, 7-fluoronaphthalen-1-yl, 7-chloronaphthalen-1-yl, 7-bromonaphthalen-1-yl, etc.), 8-halogenatednaphthalen-1-yl (for example, 8-fluoronaphthalen-1-yl, 8-chloronaphthalen-1-yl, 8-bromonaphthalen-1-yl, etc.), 1-halogenatednaphthalen-2-yl (for example, 1-fluoronaphthalen-2-yl, 1 -chloronaphthalen-2-yl, 1-bromonaphthalen-2-yl, etc.), 3-halogenatednaphthalen-2-yl (for example, 3-fluoronaphthalen-2-yl, 3-chloronaphthalen-2-yl, 3-bromonaphthalen2-yl, etc.), 4-halogenatednaphthalen-2-yl (for example, 4-fluoronaphthalen-2-yl, 4-chloronaphthalen-2-yl, 4-bromonaphthalen-2-yl, etc.), 5-halogenatednaphthalen-2-yl (for example, 5-fluoronaphthalen-2-yl, 5-chloronaphthalen-2-yl, 5-bromonaphthalen-2-yl, etc.), 6-halogenatednaphthalen-2-yl (for example, 6-fluoronaphthalen-2-yl, 6-chloronaphthalen-2-yl, 6-bromonaphthalen-2-yl, etc.), 7-halogenatednaphthalen-2-yl (for example, 7-fluoronaphthalen-2-yl, 7-chloronaphthalen-2-yl, 7-bromonaphthalen-2-yl, etc.), 8-halogenatednaphthalen-2-yl (for example, 8-fluoronaphthalen-2-yl, 8-chloronaphthalen-2-yl, 8-bromonaphthalen-2-yl, etc.), 2-halogenated inden-1-yl (for example, 2-fluoroinden-1-yl, 2-chloroinden-1-yl, 2-bromoinden-1-yl, etc.), 3-halogenated inden-1-yl (for example, 3-fluoroinden-1-yl, 3-chloroinden-1-yl, 3-bromoinden-1-yl, etc.), 4-halogenated inden-1-yl (for example, 4-fluoroinden-1-yl, 4-chloroinden-1-yl, 4-bromoinden-1-yl, etc.), 5-halogenated inden-1-yl (for example, 5-fluoroinden-1-yl, 5-chloroinden-1-yl, 5-bromoinden-1-yl, etc.), 6-halogenated inden-1-yl (for example, 6-fluoroinden-1-yl, 6-chloroinden-1-yl, 6-bromoinden-1-yl, etc.), 7-halogenated inden-1-yl (for example, 7-fluoroinden-1-yl, 7-chloroinden-1-yl, 7-bromoinden-1-yl, etc.), 1-halogenated inden-2-yl (for example, 1-fluoroinden-2-yl, 1-chloroinden-2-yl, 1-bromoinden-2-yl, etc.), 3-halogenated inden-2-yl (for example, 3-fluoroinden-2-yl, 3-chloroinden-2-yl, 3-bromoinden-2-yl, etc.), 4-halogenated inden-2-yl (for example, 4-fluoroinden-2-yl, 4-chloroinden-2-yl, 4-bromoinden-2-yl, etc.), 5-halogenated inden-2-yl (for example, 5-fluoroinden-2-yl, 5-chloroinden-2-yl, 5-bromoinden-2-yl, etc.), 6-halogenated inden-2-yl (for example, 6-fluoroinden-2-yl, 6-chloroinden-2-yl, 6-bromoinden-2-yl, etc.), 7-halogenated inden-2-yl (for example, 7-fluoroinden-2-yl, 7-chloroinden-2-yl, 7-bromoinden-2-yl, etc.), 7-halogenated inden-4-yl (for example, 7-fluoroinden-4-yl, 7-chloroinden-4-yl, 7-bromoinden-4-yl, etc.), 5-halogenated inden-3-yl (for example, 5-fluoroinden-3-yl, 5-chloroinden-3-yl, 5-bromoinden-3-yl, etc.), 3-halogenated inden-5-yl (for example, 3-fluoroinden-5-yl, 3-chloroinden-5-yl, 3-bromoinden-5-yl, etc.), 2-halogenated inden-6-yl (for example, 2-fluoroinden-6-yl, 2-chloroinden-6-yl, 2-bromoinden-6-yl, etc.), 4-halogenated inden-7-yl (for example, 4-fluoroinden-7-yl, 4-chloroinden-7-yl, 4-bromoinden-7-yl, etc.), 1-halogenated inden-4-yl (for example, 1-fluoroinden-4-yl, 1-chloroinden-4-yl, 1-bromoinden-4-yl, etc.), 2,3-dihalogenatedphenyl (for example, 2,3-difluorophenyl, 2,3-dichlorophenyl, 2,3-dibromophenyl, etc.), 2,3-dihalogenatednaphthalen-1-yl (for example, 2,3-difluoronaphthalen-1-yl, 2,3-dichloronaphthalen-1-yl, 2,3-dibromonaphthalen-1-yl, etc.), 2,4-dihalogenated phenyl (for example, 2,4-difluorophenyl, 2,4-dichlorophenyl, 2,4-dibromophenyl, etc.), 2,6-dihalogenatedphenyl (for example, 2,6-difluorophenyl, 2,6-dichlorophenyl, 2,6-dibromophenyl, etc.), 4,5-dihalogenatednaphthalen-1-yl (for example, 4,5-difluoronaphthalen-1 -yl, 4,5-dichloronaphthalen-1-yl, 4,5-dibromonaphthalen-1-yl, etc.), 4,6-dihalogenatednaphthalen-1-yl (for example, 4,6-difluoronaphthalen-1-yl, 4,6-dichloronaphthalen-1-yl, 4,6-dibromonaphthalen-1-yl, etc.), 4,8-dihalogenatednaphthalen-1-yl (for example, 4,8-difluoronaphthalen-1-yl, 4,8-dichloronaphthalen-1-yl, 4,8-dibromonaphthalen-1-yl, etc.) As shown above, the position to be substituted is not limited so that the position may be 2-, 3-, or 4-position in the case of phenyl, whereas 2-, 3-, 4-, 5-, 6-, 7-, or 8-position in the case of naphthalen-1-yl.

Examples of "lower alkyl" include a straight or branched chain alkyl group having 1 to 10 carbon atoms, such as methyl, ethyl, propyl, isopropyl, butyl, isobutyl, sec-butyl, tert-butyl, pentyl, isopentyl, neopentyl, tert-pentyl, 1-ethylpropyl, and hexyl.

It should be noted that the term "lower" as herein used means that the number of carbon atoms of an aliphatic hydrocarbon group such as an alkyl or an alkenyl is 1-10, preferably 1-8, more preferably 1-6 unless otherwise indicated.

Thus, examples of an aryl substituted with the lower alkyl include 2-methylphenyl, 3-methylphenyl, 4-methylphenyl, 2-ethylphenyl, 3-ethylphenyl, 4-ethylphenyl, 2-methylnaphthalen-1-yl, 3-methylnaphthalen-1-yl, 4-methylnaphthalen-1-yl, 5-methylnaphthalen-1-yl, 6-methylnaphthalen-1 -yl, 7-methylnaphthalen-1-yl, 8-methylnaphthalen-1-yl, 2,4-dimethylphenyl, 2,4,6-trimethylphenyl, 4,5-dimethylnaphthalen-1-yl, 4,6-dimethylnaphthalen-1-yl, 4,8-dimethylnaphthalen-1-yl, 2-methylinden-1-yl, 3-methylinden-1-yl, 4-methylinden-1-yl, 5-methylinden-1-yl, 6-methylinden- -yl, 7-methylinden-1-yl, 1-methylinden-2-yl, 3-methylinden-2-yl, 4-methylinden-2-yl, 5-methylinden-2-yl, 6-methylinden-2-yl, 7-methylinden-2-yl, 1-methylinden-3-yl, 2-methylinden-3-yl, 4-methylinden-3-yl, 5-methylinden-3-yl, 6-methylinden-3-yl, 7-methylinden-3-yl, 1-methylinden-4-yl, 2-methylinden-4-yl, 3-methylinden-4-yl, 5-methylinden-4-yl, 6-methylinden-4-yl, 7-methylinden-4-yl, 1-methylinden-5-yl, 2-methylinden-5-yl, 3-methylinden-5-yl, 4-methylinden-5-yl, 6-methylinden-5-yl, 7-methylinden-5-yl, 1-methylinden-6-yl, 2-methylinden-6-yl, 3-methylinden-6-yl, 4-methylinden-6-yl, 5-methylinden-6-yl, 7-methylinden-6-yl, 1-methylinden-7-yl, 2-methylinden-7-yl, 3-methylinden-7-yl, 4-methylinden-7-yl, 5-methylinden-7-yl, 6-methylinden-7-yl. The position to be substituted is not limited so that the position may be 2-, 3-, or 4-position in the case of phenyl, whereas 2-, 3-, 4-, 5-, 6-, 7-, or 8-position in the case of naphthalen- 1-yl.

Examples of "lower alkenyl" include a straight or branched chain alkenyl group having 1 to 10 carbon atoms, such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, or the like.

Thus, examples of an aryl substituted with the lower alkenyl include, but are not limited to, 2-vinylphenyl, 3-vinylphenyl, 4-vinylphenyl, 2-allylphenyl, 3-allylphenyl, 4-allylphenyl, 2-vinylnaphthalen-1-yl, 3-vinylnaphthalen-1-yl, 4-vinylnaphthalen-1-yl, 5-vinylnaphthalen-1-yl, 6-vinylnaphthalen-1-yl, 7-vinylnaphthalen-1-yl, 8-vinylnaphthalen-1-yl, 2-vinylinden-1-yl, 3-vinylinden-1-yl, 4-vinylinden-1-yl, 5-vinylinden-1-yl, 6-vinylinden-1-yl, 7-vinylinden-1-yl, 1-vinylinden-2-yl, 3-vinylinden-2-yl, 4-vinylinden-2-yl, 5-vinylinden-2-yl, 6-vinylinden-2-yl, 7-vinylinden-2-yl, 1-vinylinden-3-yl, 2-vinylinden-3-yl, 4-vinylinden-3-yl, 5-vinylinden-3-yl, 6-vinylinden-3-yl, 7-vinylinden-3-yl, 1-vinylinden-4-yl, 2-vinylinden-4-yl, 3-vinylinden-4-yl, 5-vinylinden-4-yl, 6-vinylinden-4-yl, 7-vinylinden-4-yl, 1-vinylinden-5-yl, 2-vinylinden-5-yl, 3-vinylinden-5-yl, 4-vinylinden-5-yl, 6-vinylinden-5-yl, 7-vinylinden-5-yl, 1-vinylinden-6-yl, 2-vinylinden-6-yl, 3-vinylinden-6-yl, 4-vinylinden-6-yl, 5-vinylinden-6-yl, 7-vinylinden-6-yl, 1-vinylinden-7-yl, 2-vinylinden-7-yl, 3-vinylinden-7-yl, 4-vinylinden-7-yl, 5-vinylinden-7-yl, 6-vinylinden-7-yl, or the like.

Examples of "lower alkoxy" include an alkoxy group having 1 to 10 carbon atoms, such as methoxy, ethoxy, propoxy, isopropoxy, butoxy, isobutoxy, neobutoxy, t-butoxy, pentoxy, isopentoxy, or the like.

Thus, examples of an aryl substituted with the lower alkoxy include 2-methoxyphenyl, 3-methoxyphenyl, 4-methoxyphenyl, 2-ethoxyphenyl, 3-ethoxyphenyl, 4-ethoxyphenyl, 2-methoxynaphthalen-1-yl, 3-methoxynaphthalen-1-yl, 4-methoxynaphthalen-1-yl, 5-methoxynaphthalen-1-yl, 6-methoxynaphthalen-1-yl, 7-methoxynaphthalen-1-yl, 8-methoxynaphthalen-1-yl, 2,4-dimethoxyphenyl, 2,4,6-trimethoxyphenyl, 4,5-dimethoxynaphthalen-1-yl, 4,6-dimethoxynaphthalen-1-yl, 4,8-dimethoxynaphthalen-1-yl, 1-methoxyinden-2-yl, 1-methoxyinden-3-yl, 1-methoxyinden-4-yl, 1-methoxyinden-5-yl, 1-methoxyinden-6-yl, 1-methoxyinden-7-yl, 1,3-dimethoxyinden-2-yl, 1,2-dimethoxyinden-3-yl, 1,3-dimethoxyinden-4-yl, 2,3-dimethoxyinden-5-yl, 1,4-dimethoxyinden-6-yl, 1,4-dimethoxyinden-7-yl, or the like.

Examples of "halogenated lower alkyl" include a lower alkyl as shown above substituted with a halogen atom, such as fluoromethyl, chloromethyl, bromomethyl, 2-fluoroethyl, 2-chloroethyl, 2-bromoethyl, 1-fluoroethyl, 1-chloroethyl, 1-bromoethyl, trifluoromethyl, trichloromethyl, tribromomethyl, difluoromethyl, dichloromethyl, dibromomethyl, 1,2-difluoroethyl, 1,2-dichloroethyl, 1,2-dibromoethyl, 1,1-difluoroethyl, 1,1-dichloroethyl, 1,1-dibromoethyl, 2,2-difluoroethyl, 2,2-dichloroethyl, 2,2-dibromoethyl, or the like.

Thus, examples of an aryl substituted with the halogenated lower alkyl include an aryl substituted with a halomethyl (for example, a phenyl substituted with fluoromethyl, naphthalen-1-yl substituted with fluoromethyl, a phenyl substituted with chloromethyl, naphthalen-1-yl substituted with chloromethyl, a phenyl substituted with bromomethyl, naphthalen-1-yl substituted with bromomethyl, etc.), an aryl substituted with a 2-haloethyl (for example, a phenyl substituted with 2-fluoroethyl, naphthalen-1-yl substituted with 2-fluoroethyl, a phenyl substituted with 2-chloroethyl, naphthalen-1-yl substituted with 2-chloroethyl, a phenyl substituted with 2-bromoethyl, naphthalen-1-yl substituted with 2-bromoethyl, etc.), an aryl substituted with a 1-halo-ethyl (for example, a phenyl substituted with 1-fluoroethyl, naphthalen-1-yl substituted with 1-fluoroethyl, a phenyl substituted with 1-chloroethyl, naphthalen-1-yl substituted with 1-chloroethyl, a phenyl substituted with 1-bromoethyl, naphthalen-1-yl substituted with 1-bromoethyl, etc.), and an aryl substituted with a trihalomethyl (for example, a phenyl substituted with trifluoromethyl, naphthalen-1-yl substituted with trifluoromethyl, a phenyl substituted with trichloromethyl, naphthalen-1-yl substituted with trichloromethyl, a phenyl substituted with tribromomethyl, naphthalen-1-yl substituted with tribromonethyl, etc.). The position to be substituted is not limited so that the position may be 2-, 3-, or 4-position in the case of phenyl, whereas 2-, 3-, 4-, 5-, 6-, 7-, or 8-position in the case of naphthalen-1-yl. In the case of indenyl, examples include, but are not limited to, 1-fluoromethylinden-2-yl, 1-chloromethylinden-2-yl, 1-bromomethylinden-2-yl, 1-(2-fluoroethyl)inden-2-yl, 1-(2-chloroethyl)inden-2-yl, 1-(2-bromoethyl)inden-2-yl, 1-(1-fluoroethyl)inden-2-yl, 1-(1-chloroethyl)inden-2-yl, 1-(1-bromoethyl)inden-2-yl, 1-trifluoromethylinden-2-yl, 1-trichloromethylinden-2-yl, 1-tribromomethylinden-2-yl, 1-fluoromethylinden-3-yl, 1-chloromethylinden-3-yl, 1-bromomethylinden-3-yl, 1-(2-fluoroethyl)inden-3-yl, 1-(2-chloroethyl)inden-3-yl, 1-(2-bromoethyl)inden-3-yl, 1-(1-fluoroethyl)inden-3-yl, 1-(1-chloroethyl)inden-3-yl, 1-(1-bromoethyl)inden-3-yl, 1-trifluoromethylinden-3-yl, 1-trichloromethylinden-3-yl, 1-tribromomethylinden-3-yl, 1-fluoromethylinden-4-yl, 1-chloromethylinden-4-yl,

1-bromomethylinden-4-yl, 1-(2-fluoroethyl)inden-4-yl, 1-(2-chloroethyl)inden-4-yl, 1-(2-bromoethyl)inden-4-yl, 1-(1-fluoroethyl)inden-4-yl, 1-(1-chloroethyl)inden-4-yl, 1-(1-bromoethyl)inden-4-yl, 1-trifluoromethylinden-4-yl, 1-trichloromethylinden-4-yl, 1-tribromomethylinden-4-yl, 1-fluoromethylinden-5-yl, 1-chloromethylinden-5-yl, 1-bromomethylinden-5-yl, 1-(2-fluoroethyl)inden-5-yl, 1-(2-chloroethyl)inden-5-yl, 1-(2-bromoethyl)inden-5-yl, 1-(1-fluoroethyl)inden-5-yl, 1-(1-chloroethyl)inden-5-yl, 1-(1-bromoethyl)inden-5-yl, 1-trifluoromethylinden-5-yl, 1-trichloromethylinden-5-yl, 1-tribromomethylinden-5-yl, 1-fluoromethylinden-6-yl, 1-chloromethylinden-6-yl, 1-bromomethylinden-6-yl, 1-(2-fluoroethyl)inden-6-yl, 1-(2-chloroethyl)inden-6-yl, 1-(2-bromoethyl)inden-6-yl, 1-(1-fluoroethyl)inden-6-yl, 1-(1-chloroethyl)inden-6-yl, 1-(1-bromoethyl)inden-6-yl, 1-trifluoromethylinden-6-yl, 1-trichloromethylinden-6-yl, 1-tribromomethylinden-6-yl, 1-fluoromethylinden-7-yl, 1-chloromethylinden-7-yl, 1-bromomethylinden-7-yl, 1-(2-fluoroethyl)inden-7-yl, 1-(2-chloroethyl)inden-7-yl, 1-(2-bromoethyl)inden-7-yl, 1-(1-fluoroethyl)inden-7-yl, 1-(1-chloroethyl)inden-7-yl, 1-(1-bromoethyl)inden-7-yl, 1-trifluoromethylinden-7-yl, 1-trichloromethylinden-7-yl, 1-tribromomethylinden-7-yl, or the like.

Examples of "halogenated lower alkenyl" include a lower alkenyl as shown above substituted with a halogen atom such as 3-fluoro-1-propenyl, 3-chloro-1-propenyl, 3-bromo-1-propenyl, 4-fluoro-2-butenyl, 4-chloro-2-butenyl, 4-bromo-2-butenyl, 1-fluoro-2-butenyl, 1-chloro-2-butenyl, 1-bromo-2-butenyl, or the like.

Thus, examples of an aryl substituted with the halogenated lower alkenyl include an aryl substituted with a 3-halo-1-propenyl (for example, a phenyl substituted with 3-fluoro-1-propenyl, naphthalen-1-yl substituted with 3-fluoro-1-propenyl, a phenyl substituted with 3-chloro-1-propenyl, naphthalen-1-yl substituted with 3-chloro-1-propenyl, a phenyl substituted with 3-bromo-1-propenyl, naphthalen-1-yl substituted with 3-bromo-1-propenyl, etc.), an aryl substituted with a 4-halo-2-butenyl (for example, a phenyl substituted with 4-fluoro-2-butenyl, naphthalen-1-yl substituted with 4-fluoro-2-butenyl, a phenyl substituted with 4-chloro-2-butenyl, naphthalen-1-yl substituted with 4-chloro-2-butenyl, a phenyl substituted with 4-bromo-2-butenyl, naphthalen-1-yl substituted with 4-bromo-2-butenyl, etc.), an aryl substituted with a 1-halo-2-butenyl (for example, a phenyl substituted with 1-fluoro-2-butenyl, naphthalen-1-yl substituted with 1-fluoro-2-butenyl, a phenyl substituted with 1-chloro-2-butenyl, naphthalen-1-yl substituted with 1-chloro-2-butenyl, a phenyl substituted with 1-bromo-2-butenyl, naphthalen-1-yl substituted with 1-bromo-2-butenyl, etc.), or the like. The position to be substituted is not limited so that the position may be 2-, 3-, or 4-position in the case of phenyl, whereas 2-, 3-, 4-, 5-, 6-, 7-, or 8-position in the case of naphthalen-1-yl. In the case of indenyl, examples include, but are not limited to, 1-(3-fluoro-1-propenyl)inden-2-yl, 1-(3-chloro-1-propenyl)inden-2-yl, 1-(3-bromo-1-propenyl)inden-2-yl, 1-(4-fluoro-2-butenyl)inden-2-yl, 1-(4-chloro-2-butenyl)inden-2-yl, 1-(4-bromo-2-butenyl)inden-2-yl, 1-(1-fluoro-2-butenyl)inden-2-yl, 1-(1-chloro-2-butenyl)inden-2-yl, 1-(1-bromo-2-butenyl)inden-2-yl, 1-(3-fluoro-1-propenyl)inden-3-yl, 1-(3-chloro-1-propenyl)inden-3-yl, 1-(3-bromo-1-propenyl)inden-3-yl, 1-(4-fluoro-2-butenyl)inden-3-yl, 1-(4-chloro-2-butenyl)inden-3-yl, 1-(4-bromo-2-butenyl)inden-3-yl, 1-(1-fluoro-2-butenyl)inden-3-yl, 1-(1-chloro-2-butenyl)inden-3-yl, 1-(1-bromo-2-butenyl)inden-3-yl, 1-(3-fluoro-1-propenyl)inden-4-yl, 1-(3-chloro-1-propenyl)inden-4-yl, 1-(3-bromo-1-propenyl)inden-4-yl, 1-(4-fluoro-2-butenyl)inden-4-yl, 1-(4-chloro-2-butenyl)inden-4-yl, 1-(4-bromo-2-butenyl)inden-4-yl, 1-(1-fluoro-2-butenyl)inden-4-yl, 1-(1-chloro-2-butenyl)inden-4-yl, 1-(1-bromo-2-butenyl)inden-4-yl, 1-(3-fluoro-1-propenyl)inden-5-yl, 1-(3-chloro- 1-propenyl)inden-5-yl, 1-(3-bromo-1-propenyl)inden-5-yl, 1-(4-fluoro-2-butenyl)inden-5-yl, 1-(4-chloro-2-butenyl)inden-5-yl, 1-(4-bromo-2-butenyl)inden-5-yl, 1-(1-fluoro-2-butenyl)inden-5-yl, 1-(1-chloro-2-butenyl)inden-5-yl, 1-(1-bromo-2-butenyl)inden-5-yl, 1-(3-fluoro-1-propenyl)inden-6-yl, 1-(3-chloro-1-propenyl)inden-6-yl, 1-(3-bromo-1-propenyl)inden-6-yl, 1-(4-fluoro-2-butenyl)inden-6-yl, 1-(4-chloro-2-butenyl)inden-6-yl, 1-(4-bromo-2-butenyl)inden-6-yl, 1-(1-fluoro-2-butenyl)inden-6-yl, 1-(1-chloro-2-butenyl)inden-6-yl, 1-(1-bromo-2-butenyl)inden-6-yl, 1-(3-fluoro-1-propenyl)inden-7-yl, 1-(3-chloro-1-propenyl)inden-7-yl, 1-(3-bromo-1-propenyl)inden-7-yl, 1-(4-fluoro-2-butenyl)inden-7-yl, 1-(4-chloro-2-butenyl)inden-7-yl, 1-(4-bromo-2-butenyl)inden-7-yl, 1-(1-fluoro-2-butenyl)inden-7-yl, 1-(1-chloro-2-butenyl)inden-7-yl, 1-(1-bromo-2-butenyl)inden-7-yl, or the like.

Examples of "a halogenated lower alkoxy" include a lower alkoxy as shown above substituted with a halogen atom such as fluoromethoxy, chloromethoxy, bromomethoxy, 1-fluoroethoxy, 1-chloroethoxy, 1-bromoethoxy, 2-fluoroethoxy, 2-chloroethoxy, 2-bromoethoxy, or the like.

Thus, examples of an aryl substituted with the halogenated lower alkoxy include an aryl substituted with a halo-methoxy (for example, a phenyl substituted with fluoromethoxy, naphthalen-1-yl substituted with fluoromethoxy, a phenyl substituted with chloromethoxy, naphthalen-1-yl substituted with chloromethoxy, a phenyl substituted with bromomethoxy, naphthalen-1-yl substituted with bromomethoxy, etc.), an aryl substituted with a halo-ethoxy (for example, a phenyl substituted with 1-fluoroethoxy, naphthalen-1-yl substituted with 1-fluoroethoxy, a phenyl substituted with 1-chloroethoxy, naphthalen-1-yl substituted with 1-chloroethoxy, a phenyl substituted with 1-bromoethoxy, naphthalen-1-yl substituted with 1-bromoethoxy, a phenyl substituted with 2-fluoroethoxy, naphthalen-1-yl substituted with 2-fluoroethoxy, a phenyl substituted with 2-chloroethoxy, naphthalen-1-yl substituted with 2-chloroethoxy, a phenyl substituted with 2-bromoethoxy, naphthalen-1-yl substituted with 2-bromoethoxy, etc.). The position to be substituted is not limited so that the position may be 2-, 3-, or 4-position in the case of phenyl, whereas 2-, 3-, 4-, 5-, 6-, 7-, or 8-position in the case of naphthalen-1-yl. In the case of indenyl, examples include, but are not limited to, 1-fluoromethoxyinden-2-yl, 1-chloromethoxyinden-2-yl, 1-bromomethoxyinden-2-yl, 1-(1-fluoroethoxy)inden-2-yl, 1-(1-chloroethoxy)inden-2-yl, 1-(1-bromoethoxy)inden-2-yl, 1-(2-fluoroethoxy)inden-2-yl, 1-(2-chloroethoxy)inden-2-yl, 1-(2-bromoethoxy)inden-2-yl, 1-fluoromethoxyinden-3-yl, 1-chloromethoxyinden-3-yl, 1-bromomethoxyinden-3-yl, 1-(1-fluoroethoxy)inden-3-yl, 1-(1-chloroethoxy)inden-3-yl, 1-(1-bromoethoxy)inden-3-yl, 1-(2-fluoroethoxy)inden-3-yl, 1-(2-chloroethoxy)inden-3-yl, 1-(2-bromoethoxy)inden-3-yl, 1-fluoromethoxyinden-4-yl, 1-chloromethoxyinden-4-yl, 1-bromomethoxyinden-4-yl, 1-(1-fluoroethoxy)inden-4-yl, 1-(1-chloroethoxy)inden-4-yl, 1-(1-bromoethoxy)inden-4-yl, 1-(2-fluorcethoxy)inden-4-yl, 1-(2-chloroethoxy)inden-4-yl, 1-(2-bromoethoxy)inden-4-yl, 1-fluoromethoxyinden-5-yl, 1-chloromethoxyinden-5-yl, 1-bromomethoxyinden-5-yl, 1-(1-fluoroethoxy)inden-5-yl, 1-(1-chloroethoxy)inden-5-yl, 1-(1-bromoethoxy)inden-5-yl, 1-(2-fluoroethoxy)inden-5-yl, 1-(2-chloroethoxy)inden-5-yl, 1-(2-bromoethoxy)inden-5-yl, 1-fluoromethoxyinden-6-yl, 1-chloromethoxyinden-6-yl, 1-bromomethoxyinden-6-yl, 1-(1-fluoroethoxy)inden-6-yl, 1-(1-chloroethoxy)inden-6-yl, 1-(1-bromoethoxy)inden-6-yl, 1-(2-fluoroethoxy)inden-6-yl, 1-(2-chloroethoxy)inden-6-yl, 1-(2-bromoethoxy)inden-6-yl, 1-fluoromethoxyinden-7-yl, 1-chloromethoxyinden-7-yl, 1-bromomethoxyinden-7-yl, 1-(1-fluoroethoxy)inden-7-yl, 1-(1-chloroethoxy)inden-7-yl, 1-(1-bromoethoxy)inden-7-yl, 1-(2-fluoroethoxy)inden-7-yl, 1-(2-chloroethoxy)inden-7-yl, 1-(2-bromoethoxy)inden-7-yl.

Examples of an aryl substituted with hydroxy as shown above include 2-hydroxyphenyl, 2-hydroxynaphthalen-1-yl, 3-hydroxyphenyl, 3-hydroxynaphthalen-1-yl, 4-hydroxyphenyl, 4-hydroxynaphthalen-1-yl, 5-hydroxynaphthalen-1-yl, 6-hydroxynaphthalen-1-yl, 7-hydroxynaphthalen-1-yl, 8-hydroxynaphthalen-1-yl, 1-hydroxyinden-1-yl, 1-hydroxyinden-2-yl, 1-hydroxyinden-3-yl, 1-hydroxyinden-4-yl, 1-hydroxyinden-5-yl, 1-hydroxyinden-6-yl, 1-hydroxyinden-7-yl, 1,4-dihydroxyinden-1-yl, 1,5-dihydroxyinden-2-yl, 2,5-dihydroxyinden-3-yl, 1,3-dihydroxyinden-4-yl, 1,3-dihydroxyinden-5-yl, 2,4-dihydroxyinden-6-yl, 1,4-dihydroxyinden-7-yl, or the like.

The term "an acyl" as shown above has the same meaning as "an acyl" as a substituent in "an aliphatic hydrocarbon group which may be optionally substituted" as described hereinafter.

Examples of an aryl substituted with an acyl include, but are not limited to, 2-acetylphenyl, 2-acetylnaphthalen-1-yl, 3-acetylphenyl, 3-acetylnaphthalen-1-yl, 4-acetylphenyl, 4-acetylnaphthalen-1-yl, 5-acetylnaphthalen-1-yl, 6-acetylnaphthalen-1-yl, 7-acetylnaphthalen-1-yl, 8-acetylnaphthalen-1-yl, 1-acetylinden-1-yl, 1-acetylinden-2-yl, 1-acetylinden-3-yl, 1-acetylinden-4-yl, 1-acetylinden-5-yl, 1-acetylinden-6-yl, 1-acetylinden-7-yl, 1-acetylinden-2-yl, 3-acetylinden-2-yl, 4-acetylinden-2-yl, 5-acetylinden-2-yl, 6-acetylinden-2-yl, 7-acetylinden-2-yl, 1-acetylinden-3-yl, 2-acetylinden-3-yl, 4-acetylinden-3-yl, 5-acetylinden-3-yl, 6-acetylinden-3-yl, 7-acetylinden-3-yl, 1-acetylinden-4-yl, 2-acetylinden-4-yl, 3-acetylinden-4-yl, 5-acetylinden-4-yl, 6-acetylinden-4-yl, 7-acetylinden-4-yl, or the like.

Examples of an aryl substituted with an amino include 2-aminophenyl, 2-aminonaphthalen-1-yl, 3-aminophenyl, 3-aminonaphthalen-1-yl, 4-aminophenyl, 4-aminonaphthalen-1-yl, 5-aminonaphthalen-1-yl, 6-aminonaphthalen-1-yl, 7-aminonaphthalen-1-yl, 8-aminonaphthalen-1-yl, or the like.

A in $G^1$ is preferably an oxygen atom.

"halogen atom" represented by $R^1$-$R^6$ in $G^1$ has the same meaning as shown above.

Aliphatic hydrocarbon group in "an aliphatic hydrocarbon group which may be optionally substituted" represented by $R^1$-$R^6$ in $G^1$ means a straight or branched chain aliphatic hydrocarbon group (an alkyl, an alkenyl, an alkynyl, etc.).

Examples of an alkyl in the "aliphatic hydrocarbon group" include a straight or branched chain alkyl having 1 to 20 carbon atoms, such as methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, heptyl, octyl, decyl, tetrahydrogeranyl, dodecyl, tridecyl, tetradecyl, hexadecyl, octadecyl, nonadecyl, eicosanyl, or the like. Preferred groups are an alkyl having 1 to 10 carbon atoms.

Examples of an alkenyl in the "aliphatic hydrocarbon group" include a straight or branched chain alkenyl having 2 to 20 carbon atoms, such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl, 1-heptenyl, 1-octenyl, geranyl, 1-decenyl, 1-tetradecenyl, 1-octadecenyl, 9-octadecenyl, 1-eicosenyl, 3,7,11,15-tetramethyl-1-hexadecenyl, or the like. Preferred groups are an alkenyl having 2 to 8 carbon atoms.

Examples of an alkynyl in the "aliphatic hydrocarbon group" include a alkynyl having 2 to 20 carbon atoms which may contain a double bond, such as ethynyl, 1-propynyl, 2-propynyl, 2-penten-4-ynyl, or the like. Preferred groups are an alkynyl having 2 to 8 carbon atoms.

The term "(aliphatic hydrocarbon group which may be optionally substituted)-O-" represented by $R^1$-$R^6$ in $G^1$ means a group wherein an aliphatic hydrocarbon group as defined above is bound to an oxygen atom, and include an alkyloxy, an alkenyloxy, an alkynyloxy, or the like.

Preferred examples of substituents of "an aliphatic hydrocarbon group which may be optionally substituted" specifically include a halogen atom, hydroxy, a lower alkoxy, lower alkenyloxy, lower alkylcarbonyloxy, a carboxyl, a substituted or unsubstituted carbamoyl, a cyano, a substituted or unsubstituted amino, an amidino, an azido, a nitro, a nitroso, mercapto, a (lower alkyl)thio, a sulfo, a substituted or unsubstituted, saturated or unsaturated alicyclic hydrocarbon group, a substituted or unsubstituted, monocyclic or fused polycyclic aryl, a substituted or unsubstituted

heterocyclic group, an acyl, or the like. The number of substituents of "an aliphatic hydrocarbon group which may be optionally substituted" is 1 to 5, and preferably 1 to 3. The position of the substituents is not limited.

"Halogen" as a substituent on "an aliphatic hydrocarbon group which may be optionally substituted" has the same meaning as shown above. "Lower alkoxy" as a substituent on "an aliphatic hydrocarbon group which may be optionally substituted" has the same meaning as shown above.

Examples of "lower alkenyloxy" as a substituent on "an aliphatic hydrocarbon group which may be optionally substituted" include a alkenyloxy having 2 to 7 carbon atoms, such as vinyloxy, allyloxy, 1-propenyloxy, 2-methyl-1-propenyloxy, 1-butenyloxy, 2-butenyloxy, 3-butenyloxy, 2-ethyl-1-butenyloxy, 3-methyl-2-butenyloxy, 1-pentenyloxy, 2-pentenyloxy, 3-pentenyloxy, 4-pentenyloxy, 4-methyl-3-pentenyloxy, or the like.

Examples of "lower alkylcarbonyloxy" as a substituent on "an aliphatic hydrocarbon group which may be optionally substituted" include an alkylcarbonyloxy having 2 to 7 carbon atoms, such as methylcarbonyloxy, ethylcarbonyloxy, propylcarbonyloxy, isopropylcarbonyloxy, butylcarbonyloxy, isobutylcarbonyloxy, t-butylcarbonyloxy, pentylcarbonyloxy, isopentylcarbonyloxy, neopentylcarbonyloxy, t-pentylcarbonyloxy, hexylcarbonyloxy, or the like.

Examples of "substituted carbamoyl" as a substituent on "an aliphatic hydrocarbon group which may be optionally substituted" include N-mono-(a lower alkyl)carbamoyl, N,N-di-(a lower alkyl)carbamoyl, N-hydroxycarbamoyl, N-(a lower alkoxy)carbamoyl, N-hydroxy-N-(a lower alkyl)carbamoyl, N-(a lower alkoxy)-N-(a lower alkyl)carbamoyl, N-phenylcarbamoyl, N-(substituted phenyl)carbamoyl, or the like.

Examples of the "N-mono-(a lower alkyl)carbamoyl" include N-methylcarbamoyl, N-ethylcarbamoyl, N-propylcarbamoyl, N-isopropylcarbamoyl, N-pentylcarbamoyl, N-isopentylcarbamoyl, N-neopentylcarbamoyl, N-t-pentylcarbamoyl, N-1-ethylpropylcarbamoyl, N-hexylcarbamoyl, or the like, since the lower alkyl has the same meaning as shown above.

Examples of the "N,N-di-(a lower alkyl)carbamoyl" include N,N-dimethylcarbamoyl, N-ethyl-N-methylcarbamoyl, N,N-diethylcarbamoyl, N-methyl-N-propylcarbamoyl, N-butyl-N-methylcarbamoyl, N-butyl-N-ethylcarbamoyl, N-butyl-N-propylcarbamoyl, N-butyl-N-isopropylcarbamoyl, N,N-dibutylcarbamoyl, N-ethyl-N-propylcarbamoyl, N,N-dipropylcarbamoyl, N-isopropyl-N-n-propylcarbamoyl, N-isopropyl-N-methylcarbamoyl, or the like, since the lower alkyl has the same meaning as shown above.

Examples of the "N-hydroxy-N-(a lower alkyl)carbamoyl" include a N-hydroxy-N-(a lower alkyl)carbamoyl having 2 to 7 carbon atoms, such as N-hydroxy-N-methylcarbamoyl, N-hydroxy-N-ethylcarbamoyl, N-hydroxy-N-propylcarbamoyl, N-hydroxy-N-butylcarbamoyl, N-hydroxy-N-isopropylcarbamoyl, N-hydroxy-N-isobutylcarbamoyl, N-hydroxy-N-sec-butylcarbamoyl, N-hydroxy-N-t-butylcarbamoyl, N-hydroxy-N-pentylcarbamoyl, N-hydroxy-N-isopentylcarbamoyl, N-hydroxy-N-neopentylcarbamoyl or the like, since the lower alkyl has the same meaning as shown above.

Examples of "N-(a lower alkoxy)-N-(a lower alkyl)carbamoyl" include a N-(a lower alkoxy)-N-(a lower alkyl) carbamoyl having 3 to 13 carbon atoms as the whole number of carbon atoms, such as N-methoxy-N-methylcarbamoyl, N-methoxy-N-ethylcarbamoyl, N-methoxy-N-propylcarbamoyl, N-methoxy-N-butylcarbamoyl, N-methoxy-N-isopropylcarbamoyl, N-methoxy-N-isobutylcarbamoyl, N-methoxy-N-sec-butylcarbamoyl, N-methoxy-N-t-butylcarbamoyl, N-methoxy-N-pentylcarbamoyl, N-methoxy-N-isopentylcarbamoyl, N-methoxy-N-neopentylcarbamoyl or the like, since the lower alkyl has the same meaning as shown above.

Examples of the substituents of the "N-(a substituted phenyl)carbamoyl" include a lower alkyl, a lower alkoxy, hydroxy, or the like, each of which has the same meaning as shown above. Preferred examples of the "N-(a substituted phenyl)carbamoyl" include (4-methylphenyl)carbamoyl, (4-ethylphenyl)carbamoyl, (4-hydroxyphenyl)carbamoyl, (4-methoxyphenyl)carbamoyl, (2,3-dihydroxyphenyl)carbamoyl, (2,3-methoxyphenyl)carbamoyl, [2,4-dihydroxyphenyl)carbamoyl, (2,4-methoxyphenyl)carbamoyl, (2,6-dihydroxyphenyl)carbamoyl, (2,6-methoxyphenyl)carbamoyl, (2,4,6-trihydroxyphenyl)carbamoyl, (2,4,6-trimethoxyphenyl)carbamoyl, (2,4-dimethoxy-6-hydroxyphenyl)carbamoyl, (2,6-dimethoxy-4-hydroxyphenyl)carbamoyl, (4,6-dihydroxy-2-methoxyphenyl)carbamoyl, (2,6-dihydroxy-4-methoxyphenyl)carbamoyl, (2,3,4-trimethoxyphenyl)carbamoyl, (2,3-dimethoxy-4-hydroxyphenyl)carbamoyl, (2,4-dimethoxy-3-hydroxyphenyl)carbamoyl, (2,3-dihydroxy-4-methoxyphenyl)carbamoyl, (3,4-dimethoxy-2-hydroxyphenyl)carbamoyl, (2,4-dihydroxy-3-methoxyphenyl)carbamoyl, (2,4-dimethoxy-6-methylphenyl)carbamoyl, (2,6-dimethoxy-4-methylphenyl)carbamoyl, or the like.

Examples of "substituted amino" as a substituent in the definitions of $R^1$-$R^6$, and "an aliphatic hydrocarbon group which may be optionally substituted" include mono-(a lower alkyl)amino, a di-(a lower alkyl)amino, (a lower alkyl) carbonyl amino, or the like.

Examples of the "mono-(a lower alkyl)amino" include a mono-(a lower alkyl)amino in which the number of carbon atoms of the alkyl is 1 to 6, such as methylamino, ethylamino, propylamino, isopropylamino, butylamino, isobutylamino, sec-butylamino, t-butylamino, pentylamino, isopentylamino, hexylamino, or the like, since the lower alkyl has the same meaning as shown above.

Examples of the "di-(a lower alkyl)amino" include a di-(a lower alkyl)amino in which the whole number of the carbon is 2 to 20, such as dimethylamino, ethylmethylamino, diethylamino, methylpropylamiho, ethylpropylamino, isopropylmethylamino, isopropylethylamino, butylmethylamino, butylethylamino, isobutylmethylamino, isobutylethylamino, or the like, since the lower alkyl has the same meaning as shown above.

Examples of the "(a lower alkyl)carbonyl amino" include an alkylcarbonyl amino having 2 to 7 carbon atoms, such as methylcarbonylamino, ethylcarbonylamino, propylcarbonylamino, isopropylcarbonylamino, butylcarbonylamino, isobutylcarbonylamino, sec-butylcarbonylamino, t-butylcarbonylamino, pentylcarbonylamino, isopentylcarbonylamino, or the like, since the lower alkyl has the same meaning as shown above.

Examples of "(a lower alkyl)thio" as a substituent on "an aliphatic hydrocarbon group which may be optionally substituted" include a (lower alkyl)thio having 1 to 6 carbon atoms, such as methylthio, ethylthio, propylthio, isopropylthio, butylthio, isobutylthio, neobutylthio, t-butylthio, pentylthio, hexylthio, or the like, since the lower alkyl has the same meaning as shown above.

"A substituted or unsubstituted, saturated or unsaturated alicyclic hydrocarbon group" as a substituent on "an aliphatic hydrocarbon group which may be optionally substituted" is exemplified by a cycloalkyl, a cycloalkenyl, a cycloalkadienyl, or the like, and has a similar meaning to those in "a cyclic group which may be optionally substituted" represented by $Ar^1$ and $Ar^2$.

"A substituted or unsubstituted, monocyclic or fused polycyclic aryl" as a substituent on "an aliphatic hydrocarbon group which may be optionally substituted" has a similar meaning to those in "a cyclic group which may be optionally substituted" represented by $Ar^1$ and $Ar^2$.

A heterocyclic group of "a substituted or unsubstituted heterocyclic group" as a substituent on "an aliphatic hydrocarbon group which may be optionally substituted" has a similar meaning to those in "a cyclic group which may be optionally substituted" represented by $Ar^1$ and $Ar^2$.

Preferred examples of the substituents of "a substituted, saturated or unsaturated alicyclic hydrocarbon group", "a substituted, monocyclic or fused polycyclic aryl", and "a substituted heterocyclic group" as shown above include halogen, hydroxy, a lower alkyl, a substituted lower alkyl (wherein the substituent is a halogen, hydroxy, a lower alkoxy or (a lower alkyl)carbonyl), a lower alkenyl, a lower alkynyl, a lower alkoxy, a lower alkenyloxy, a lower alkylcarbonyloxy, a carboxyl, a lower alkylcarbonyl, (a lower alkoxy)carbonyl, a carbamoyl, a lower alkylcarbamoyl, N-di(a lower alkyl)carbamoyl, N-hydroxycarbamoyl, N-hydroxyN-(a lower alkyl)carbamoyl, N-(a phenyl)carbamoyl, N-(a substituted phenyl)carbamoyl, a cyano, an amino, mono-(a lower alkyl)amino, di-(a lower alkyl)amino, (a lower alkyl)carbonylamino, an amidino, an azido, a nitro, a nitroso, mercapto, (a lower alkyl)thio, a sulfo, a saturated or unsaturated, alicyclic hydrocarbon group, a monocyclic or fused polycyclic aryl, a heterocyclic group, or the like. The number of the substituent(s) is 1 to 3, and preferably 1. The position of the substituent(s) is not limited.

Preferred examples of "(a lower alkyl)carbonyl" as shown above include an alkanoyl having 2 to 6 carbon atoms, such as acetyl, propionyl, butylyl, isobutylyl, valeryl, isovaleryl, pivaloyl, hexanoyl, or the like, since the lower alkyl has the same meaning as shown above.

Preferred examples of "(a lower alkoxy)carbonyl" include an alkoxy carbonyl having 2 to 7 carbon atoms, such as methoxycarbonyl, ethoxycarbonyl, n-propoxycarbonyl, n-butoxycarbonyl, since the lower alkoxy has the same meaning as shown above.

The substituents other than those described above have a similar meaning to those mentioned in the substituents of "an aliphatic hydrocarbon group which may be optionally substituted".

"An acyl" as a substituent in the definitions of $R^1$-$R^6$ and in "an aliphatic hydrocarbon group which may be optionally substituted" means an acyl derived from a carboxylic acid which may be optionally substituted, an oxycarboxylic acid which may be optionally substituted, a sulfonic acid which may be optionally substituted, a sulfinic acid which may be optionally substituted, or the like. Specific examples include a group of the formula:

$$R^7CO\text{-}, R^8OCO\text{-}, R^9SO_2\text{-}, \text{or } R^{10}SO\text{-}$$

in which $R^7$, $R^8$, $R^9$ and $R^{10}$ are each a hydrocarbon group which may be optionally substituted or a heterocyclic group which may be optionally substituted.

Examples of "a hydrocarbon group" in "a hydrocarbon group or heterocyclic group which may be optionally substituted" represented by $R^7$, $R^8$, $R^9$ and $R^{10}$ include a straight or branched chain aliphatic hydrocarbon group (an alkyl, an alkenyl, an alkynyl, etc.) or the like as a non-cyclic group, and include a saturated or unsaturated, alicyclic hydrocarbon group (a cycloalkyl, a cycloalkenyl, a cycloalkadienyl, etc.), a monocyclic or fused polycyclic aryl, or the like as a cyclic group. The meanings and specific examples of them are as shown above.

Examples of the substituents of "an amino group which may be optionally substituted" represented by $R^1$-$R^6$ in $G^1$ include hydroxy, a lower alkyl, a substituted lower alkyl (wherein the substituent is a halogen, hydroxy, a lower alkoxy or (a lower alkyl)carbonyl), a lower alkenyl, a lower alkoxy, a lower alkenyloxy, a lower alkylcarbonyloxy, a carboxyl, a lower alkylcarbonyl, (a lower alkoxy)carbonyl, a carbamoyl, a lower alkylcarbamoyl, N-di(a lower alkyl) carbamoyl, N-hydroxycarbamoyl, N-hydroxy-N-(a lower alkyl)carbamoyl, N-(a phenyl)carbamoyl, N-(a substituted phenyl)carbamoyl, a saturated or unsaturated, alicyclic hydrocarbon group, an monocyclic or fused polycyclic aryl, a heterocyclic group, or the like. The meanings of them are as shown above.

Examples of the substituents of "a sulfo group which may be optionally substituted" represented by $R^1$-$R^6$ in $G^1$ include hydroxy, a lower alkyl, a substituted lower alkyl (wherein the substituent is a halogen, hydroxy, a lower alkoxy or (a lower alkyl)carbonyl), a lower alkenyl, a lower alkynyl, a lower alkoxy, a lower alkenyloxy, a lower alkylcarbonyloxy, a carboxyl, a lower alkylcarbonyl, lower alkoxy carbonyl, a carbamoyl, a lower alkylcarbamoyl, N-di(a lower alkyl)carbamoyl, N-hydroxycarbamoyl, N-hydroxy-N-(a lower alkyl)carbamoyl, N-(a phenyl)carbamoyl, N-(a substituted phenyl)carbamoyl, a cyano, an amino, mono lower alkyl amino, di lower alkyl amino, lower alkylcarbonyl amino, a nitro, a saturated or unsaturated, alicyclic hydrocarbon group, an monocyclic or fused polycyclic aryl, a heterocyclic group, or the like. The meanings of them are as shown above.

The meaning of an aryl of "an aryl which may be optionally substituted" represented by $R^1$-$R^6$ in $G^1$ is as shown above.

Examples of the substituents of "an aryl which may be optionally substituted" represented by $R^1$-$R^6$ in $G^1$ include a similar substituent to those in "a substituted, saturated or unsaturated alicyclic hydrocarbon group", "a substituted, monocyclic or fused polycyclic aryl", "a substituted heterocyclic group" as shown above. The meanings of them are as shown above.

Examples of the substituents of "a methylene which may be optionally substituted" that is formed by the combination of $R^1$ and $R^2$, $R^3$ and $R^4$ or $R^5$ and $R^6$ in $G^1$ include a halogen, hydroxy, a lower alkoxy, a lower alkenyloxy, (a lower alkyl)carbonyloxy, a carboxyl, a substituted or unsubstituted carbamoyl, a cyano, a substituted or unsubstituted amino, an amidino, an azido, a nitro, a nitroso, mercapto, (a lower alkyl)thio, a sulfo, an acyl, or the like. The meanings of them are as shown above.

Examples of the substituents of "an imino which may be optionally substituted" that is formed by the combination of $R^1$ and $R^2$, $R^3$ and $R^4$ or $R^5$ and $R^6$ in $G^1$ include halogen, hydroxy, a lower alkoxy, a lower alkenyloxy, (a lower alkyl)carbonyloxy, a carboxyl, a substituted or unsubstituted carbamoyl, mercapto, (a lower alkyl)thio, a sulfo, an acyl, or the like. The meanings of them are as shown above.

Preferably, $R^1$-$R^6$ in $G^1$ are the same or different, and are a hydrogen atom, or a lower alkyl.

$G^1$ is Preferably -O- or -O-$CR^1R^2$-.

"An azacyclic group which may be optionally substituted (hereinafter the group is conveniently abbreviated as "$G^2$")" of the formula:

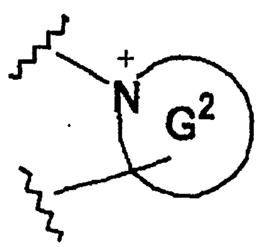

may be either a monocyclic or fused cyclic group. Examples of the cyclic groups are listed below. In the following formulae, even though the endocyclic nitrogen atom bound to the boron is not associated with a symbol +, the nitrogen atom is acquiring a plus ion charge on substitution by hydrogen or an aliphatic hydrocarbon group which may be optionally substituted (preferably, a lower alkyl (for example, methyl, etc.)).

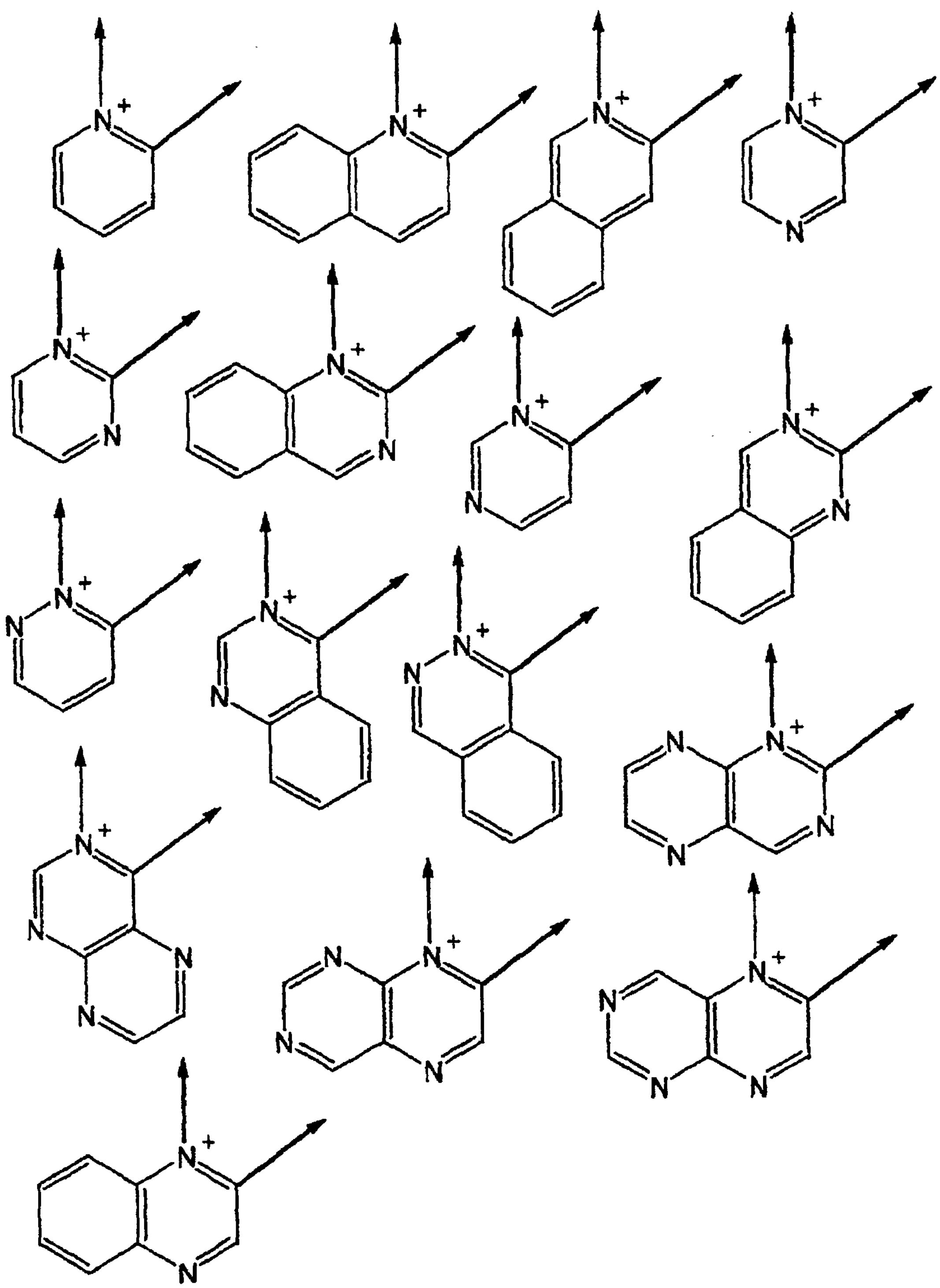

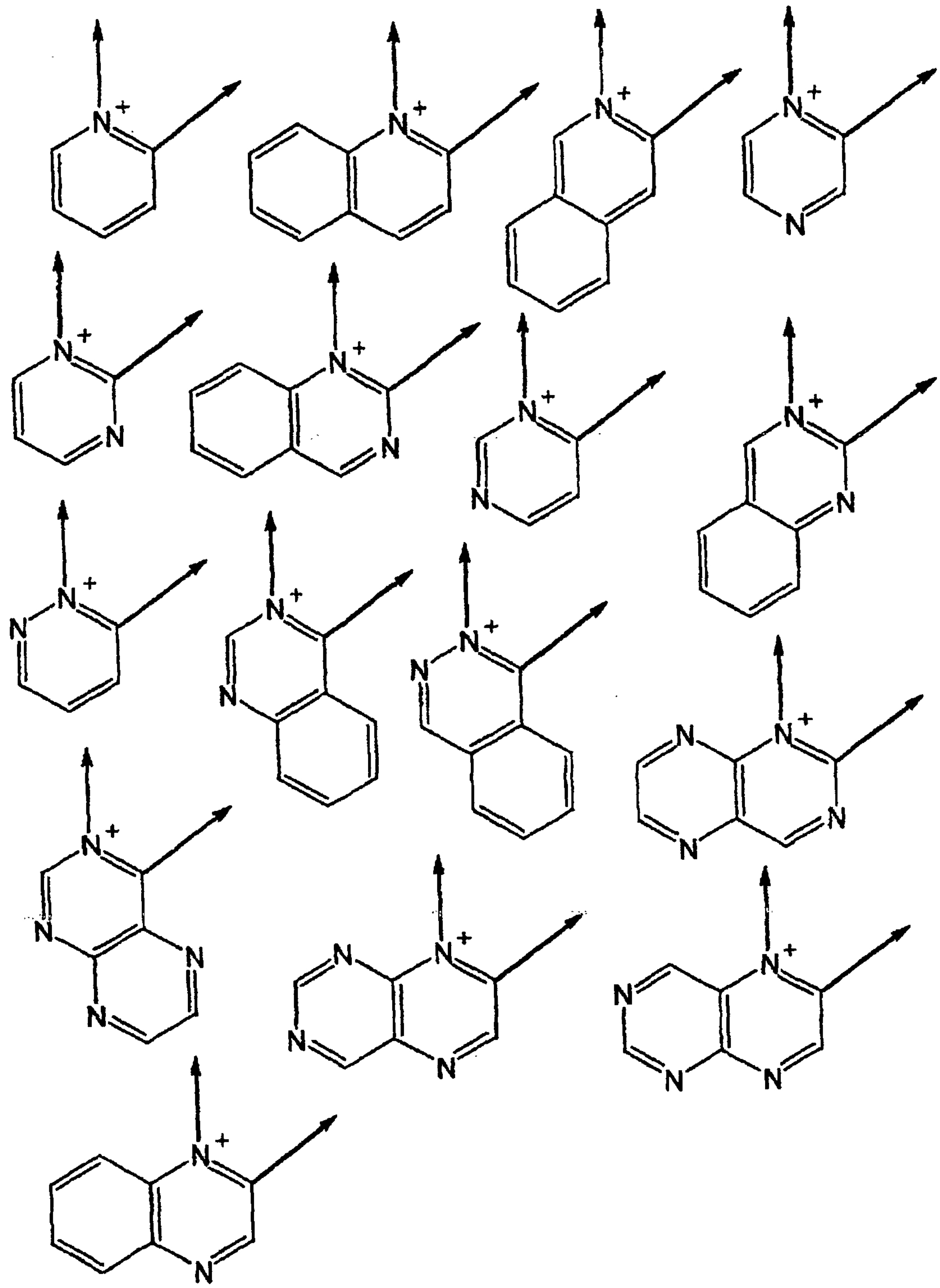

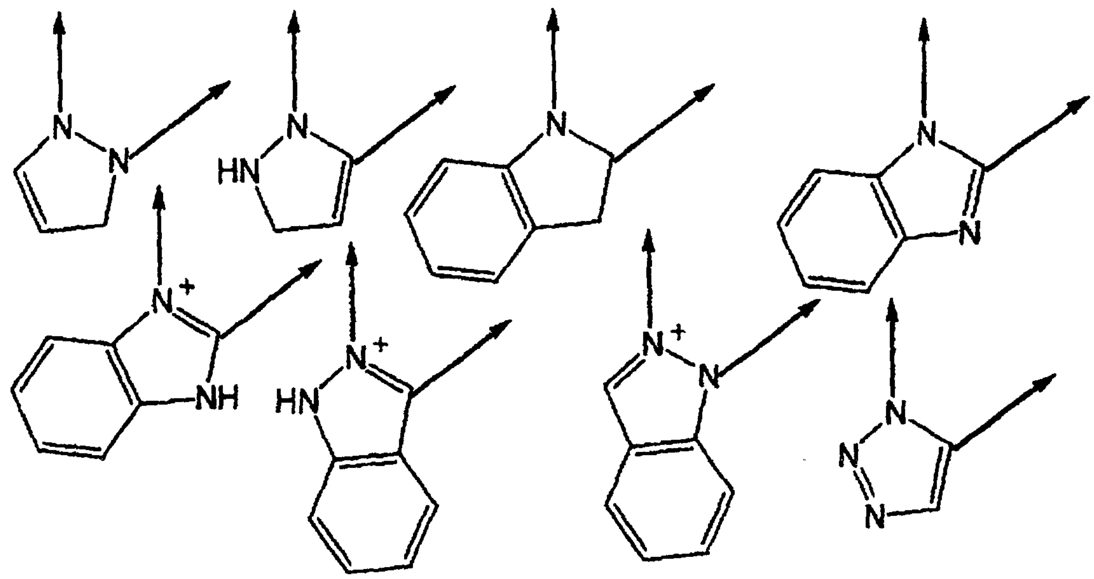

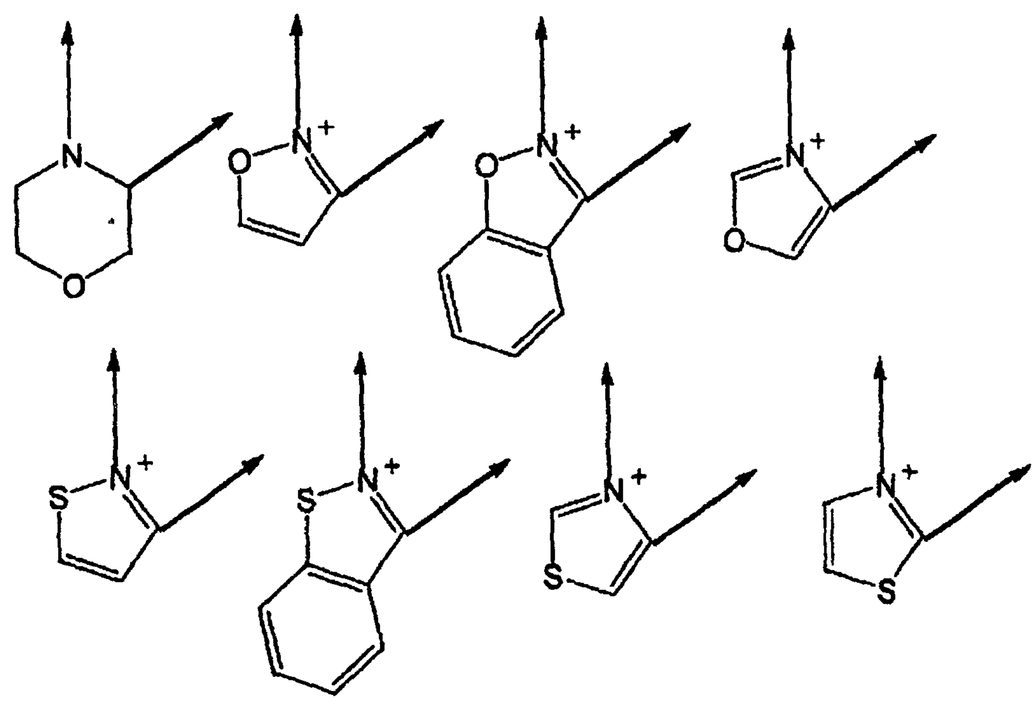

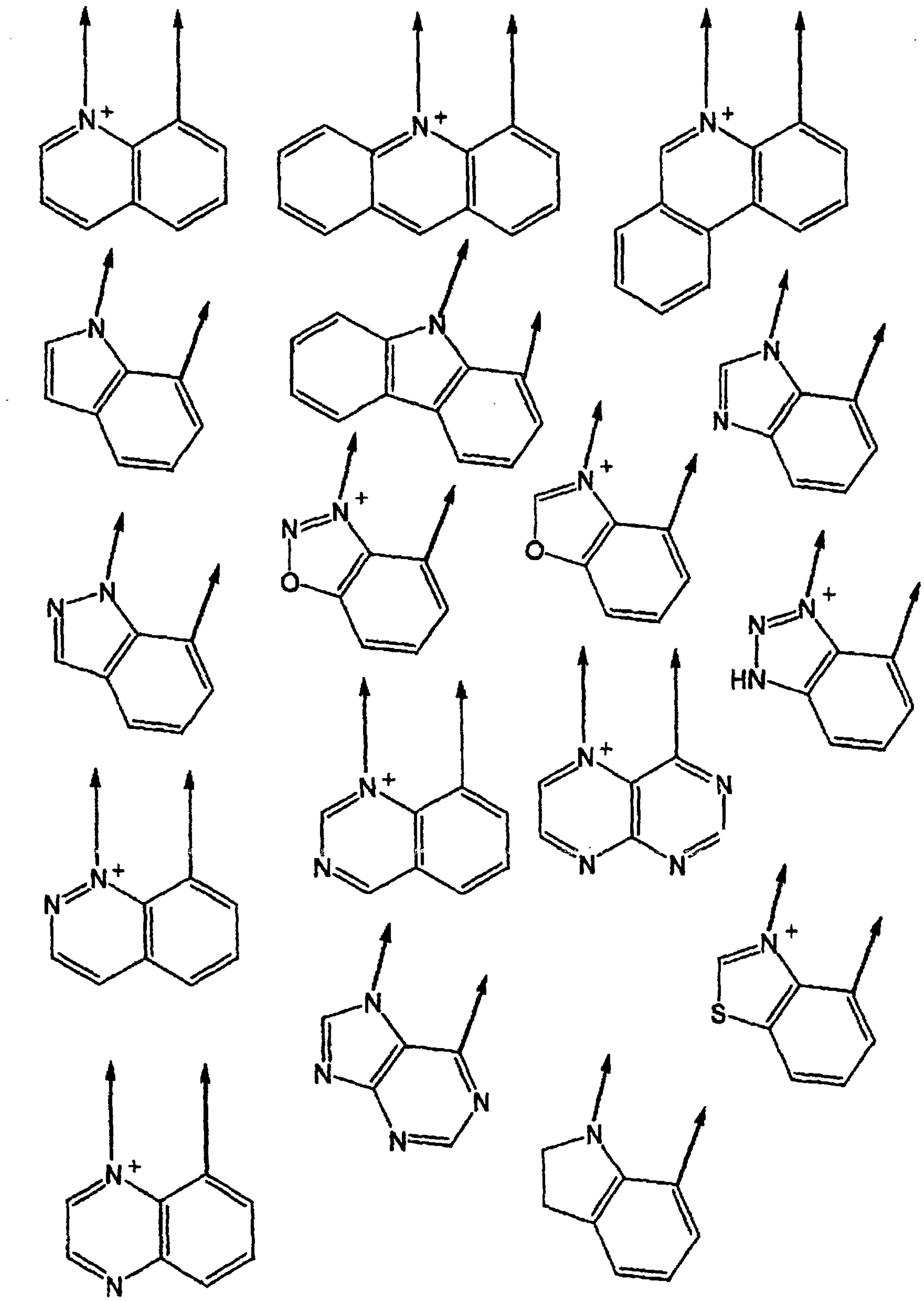

More preferred examples of the cyclic groups as described above include the following ones.

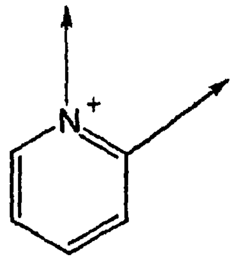 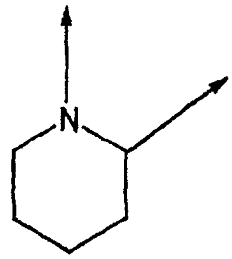 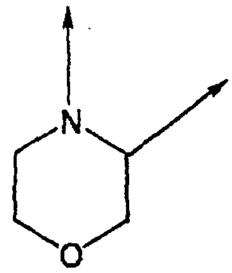

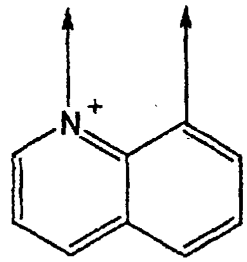 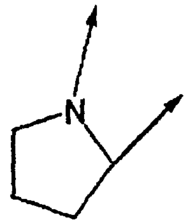 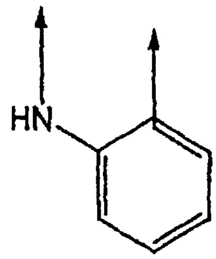

The phrase "provided that the smallest ring among rings constructed with $B^-$, $G^1$ and $G^2$ is a 5- or 6-membered ring" in the definitions of formula (I) means that the smallest membered ring among those which contains $B^-$, $G^1$ that is a chain group, and the nitrogen atom bound to the boron in the group: $G^2$ in the definition of formula (I), is a 5- or 6-membered ring.

A pharmaceutical composition useful for animals except for humans, which comprises a compound of formula (I) wherein $Ar^1$ and $Ar^2$ are the same, and are an aryl which may be optionally substituted, or a salt thereof or a hydrate of them, is preferred, and a pharmaceutical composition useful for animals except for humans, which comprises a compound of formula (I) wherein both $Ar^1$ and $Ar^2$ are an aryl which may be optionally substituted, $G^1$ is a group of the formula: -O-, $-O-CR^1R^2-$ or $-O-CR^3R^4-CR^5R^6-$ wherein $R^1$-$R^6$ are the same or different, and are each a hydrogen atom, a halogen atom, hydroxy, a lower alkyl which may be optionally substituted, a lower alkenyl which may be optionally substituted, a lower alkyloxy which may be optionally substituted, or a lower alkenyloxy which may be optionally substituted; or a salt thereof or a hydrate of them is more preferred.

In the formula (II), "an aryl which may be optionally substituted" represented by $Ar^{a1}$ has the same meaning as shown above excect that naphthalen-2-yl is substituted at the 1-position by naphthalen-1-yl, in this context.

Examples of a fused aryl which may be optionally substituted (provided that when the fused aryl is naphthalen-2-yl, then naphthalen-2-yl is not substituted at the 1-position by naphthalen-1-yl) represented by $Ar^{a2}$, include indenyl (1-indenyl, 2-indenyl, 3-indenyl, 4-indenyl, 5-indenyl, 6-indenyl, 7-indenyl), naphthyl (1-naphthyl, 2-naphthyl, etc.), anthryl, phenanthryl, acenaphthylenyl, fluorenyl (9-fluorenyl, 1-fluorenyl, etc.), or the like.

Examples of the substituents on the fused aryl which may be optionally substituted include halogen, hydroxy, a lower alkyl, a substituted lower alkyl (wherein the substituent is a halogen, hydroxy, a lower alkoxy, or (a lower alkyl) carbonyl), a lower alkenyl, a lower alkynyl, a lower alkoxy, a lower alkenyloxy, (a lower alkyl)carbonyloxy, a carboxyl, (a lower alkyl)carbonyl, (a lower alkoxy)carbonyl, a carbamoyl, (a lower alkyl)carbamoyl, N-di-(a lower alkyl)carbamoyl, N-hydroxycarbamoyl, N-hydroxy-N-(a lower alkyl)carbamoyl, N-(a phenyl)carbamoyl, N-(a substituted phenyl)carbamoyl, a cyano, an amino, a mono-(a lower alkyl)amino, a di-(a lower alkyl)amino, (a lower alkyl)carbonylamino, an amidino, an azido, a nitro, a nitroso, a mercapto, (a lower alkyl)thio, a sulfo, a saturated or unsaturated alicyclic hydrocarbon group, a monocyclic or fused polycyclic aryl, a heterocyclic group, or the like. The number of the substituent is 1 to 3, and preferably 1. The position of the substituent is not limited.

Examples of a preferable substituent among them include a halogen atom, methyl, tri-halomethyl, hydroxy, methoxy, an amino group, or a sulfo group. More preferable substituents include fluorine, chlorine, bromine, methyl, trifluoromethyl, trichloromethyl, hydroxy, methoxy, or a sulfo group.

The definitions of $R^{a1}$ and $R^{a2}$ are as shown above.

Among the compounds of formula (II), preferred compounds are shown by formula (IIa):

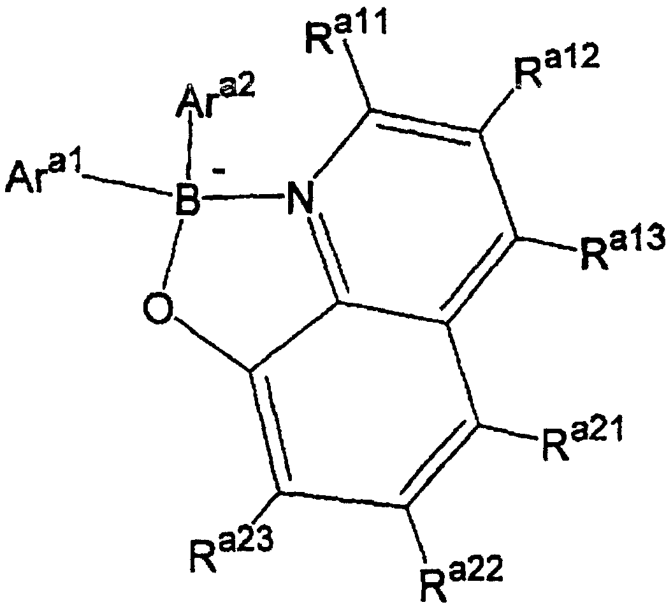

(IIa)

in which

both $Ar^{a1}$ and $Ar^{a2}$ are an aryl which may be optionally substituted;
each of $R^{a11}$-$R^{a13}$ and $R^{a21}$-$R^{a23}$ are the same or different, and are independently a hydrogen atom, a halogen atom, a lower alkyl, a lower alkenyl, a lower haloalkyl, hydroxy, an amino group which may be optionally substituted, or a sulfo group which may be optionally substituted. More preferably, each of $R^{a11}$-$R^{a13}$ and $R^{a21}$-$R^{a23}$ are the same or different, and are each a hydrogen atom, a halogen atom, methyl, trihalomethyl, hydroxy, methoxy, an amino group, or a sulfo group. Even more preferably, $R^{a11}$, $R^{a21}$, and $R^{a23}$ are the same or different, and are fluorine, chlorine, bromine, methyl, trifluoromethyl, trichloromethyl, hydroxy, methoxy, or a sulfo group.

In formula (III), the definitions of $Ar^{b1}$, $Ar^{b2}$, $R^{b1}$, $R^{b2}$, and $R^{b3}$ are as shown above.

Among the compounds of formula (III), preferred compounds are shown by formula (IIIa):

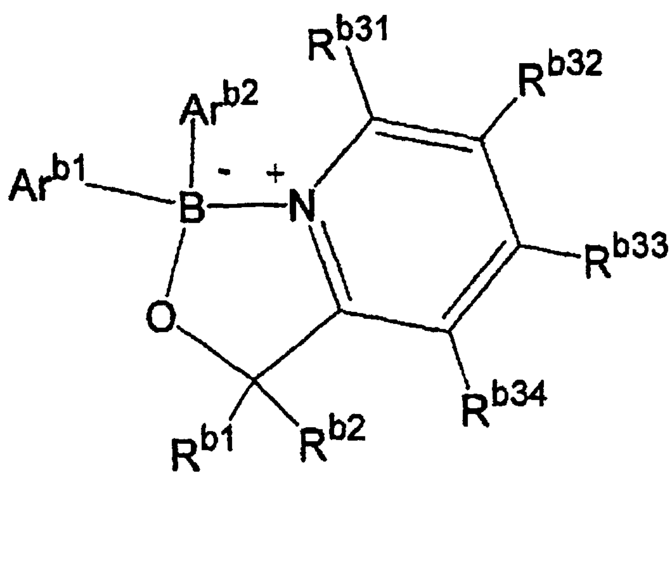

(IIIa)

in which

both $Ar^{b1}$ and $Ar^{b2}$ are an aryl which may be optionally substituted;
$R^{b1}$ and $R^{b2}$ are independently a hydrogen atom, a halogen atom, a lower alkyl, a lower alkenyl, a halogenated

lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{b1}$ and $R^{b2}$ are combined together to form oxo, a methylene which may be optionally substituted, or an imino which may be optionally substituted;

$R^{b31}$-$R^{b34}$ are the same or different, and are each a hydrogen atom, a halogen atom, a lower alkyl, a lower alkenyl, a lower haloalkyl, hydroxy, an amino group which may be optionally substituted or a sulfo group which may be optionally substituted. More preferably, $R^{b31}$-$R^{b34}$ are the same or different, and are each a hydrogen atom, a halogen atom, methyl, trihalomethyl, hydroxy, methoxy, an ammo group, or a sulfo group. Even more preferably, $R^{b32}$, $R^{b33}$, and $R^{b34}$ are the same or different, and are fluorine, chlorine, bromine, methyl, trifluoromethyl, trichloromethyl, hydroxy, methoxy, or a sulfo group.

The definitions of $Ar^{c1}$, $Ar^{c2}$, $R^{c1}$, $R^{c2}$, and $R^{c3}$ in formula (IV) are as shown above.
Among the compounds of formula (IV), preferred compounds are those wherein $Ar^{c1}$ and $Ar^{c2}$ are an aryl which may be optionally substituted, $R^{c3}$ are independently a hydrogen atom, a halogen atom, a lower alkyl, a lower alkenyl, a lowerhalo alkyl, hydroxy, an amino group which may be optionally substituted, or a sulfo group which may be optionally substituted. More preferred compounds are those wherein $R^{c3}$ are independently fluorine, chlorine, bromine, methyl, trifluoromethyl, trichloromethyl, hydroxy, methoxy or a sulfo group.
The definitions of $Ar^{d1}$, $Ar^{d2}$, $R^{d1}$, $R^{d2}$, $R^{d3}$, $R^{d4}$, and $R^{d5}$ in formula (V) are as shown above.
Among the compounds of formula (V), preferred compounds are shown by formula (Va):

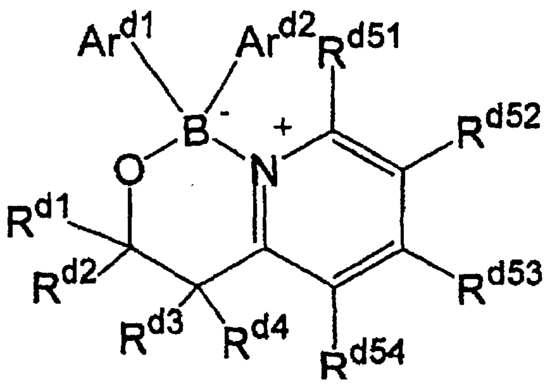

(Va)

in which

$Ar^{d1}$ and $Ar^{d2}$ are aryl which may be optionally substituted;

$R^{d1}$, $R^{d2}$, $R^{d3}$ and $R^{d4}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{d1}$ and $R^{d2}$ and/or $R^{d3}$ and $R^{d4}$ are combined together to form an oxo, a methylene which may be optionally substituted, or an imino which may be optionally substituted;

$R^{d51}$-$R^{d54}$ are the same or different, and are each a hydrogen atom, a halogen atom, a lower alkyl, a lower alkenyl, a lower haloalkyl, hydroxy, an amino group which may be optionally substituted, or a sulfo group which may be optionally substituted. More preferred compounds are those wherein $R^{d51}$-$R^{d54}$ are the same or different, and are a hydrogen atom, a halogen atom, methyl, trihalomethyl, hydroxy, methoxy, an amino group, or a sulfo group. Even more preferred compounds are those wherein $R^{d52}$, $R^{d53}$, and $R^{d54}$ are the same or different, and are fluorine, chlorine, bromine, methyl, trifluoromethyl, trichloromethyl, hydroxy, methoxy or a sulfo group.

The present invention also encompasses pharmaceutically acceptable salt and hydrates thereof.
Preferred salts of the objective compounds of the invention are pharmaceutically acceptable salts, and examples of such salts include a salt formed with an inorganic base, a salt formed with an organic base, a salt formed with an inorganic acid, a salt formed with an organic acid, and a basic or acidic amino acid salt. Examples of the salt

formed with an inorganic base include those formed with an alkali metal such as a sodium salt and a potassium salt, those formed with an alkali earth metal such as a calcium salt, a magnesium salt and a barium salt, as well as an aluminium salt, an ammonium salt, and the like. Examples of the salt formed with an organic base include those formed with trimethylamine, triethylamine, pyridine, picoline, ethanolamine, diethanolamine, triethanolamine, dicyclohexylamine, N,N'-dibenzylethylenediamine, and the like. Examples of the salt formed with an inorganic acid include those formed with hydrochloric acid, hydrofluoric acid, hydrobromic acid, nitric acid, sulfuric acid, phosphoric acid, perchloric acid, hydroiodic acid, and the like. Examples of the salt formed with an organic acid include those formed with formic acid, acetic acid, trifluoroacetic acid, fumaric acid, oxalic acid, tartaric acid, maleic acid, citric acid, succinic acid, malic acid, mandelic acid, ascorbic acid, lactic acid, gluconic acid, methanesulfonic acid, p-toluenesulfonic acid, benzenesulfonic acid, and the like. Examples of the salt formed with a basic amino acid include those formed with arginine, lysine, ornithine, and the like. Examples of the salt formed with an acidic amino acid include those formed with aspartic acid, glutaminic acid, and the like.

Preferred "hydrates" of the objective compounds of the present invention and the salts thereof are pharmaceutically acceptable hydrates, and specifically, a monohydrate, a dihydrate, a hexahydrate and the like are exemplified.

In another aspect, the present invention relates to a pharmaceutical composition useful for animals except for humans, a pharmaceutical composition for use as an antiprotozoal agent and a pharmaceutical composition for use as an anticoccidial agent, each of which comprises the compound of formula (I), (II), (III), (IV) or (v) as shown above or a salt thereof, or a hydrate of them.

As used herein, "a pharmaceutical composition useful for animals except for humans" means a medicament used in diagnosis, treatment or prevention of any disease in animals except for humans, and uses of the pharmaceutical composition useful for animals of the invention include those as all animal drugs such as an anticoccidial agent, a growth promoting agent and the like. Examples of the animals to be subjected to the pharmaceutical composition useful for animals of the invention include poultry such as fowl, goose, pheasant, turkey, and ducks, and livestock such as cattle, rabbit, sheep, goat, and pig.

The antiprotozoal agent means a medicament for prevention or treatment of the infections caused by protozoa including toxoplasma, malaria and Coccidia which is as follows.

The anticoccidial agent means a medicament for prevention or treatment of the infection mostly caused by *Eimeria tenella, Eimeria acervulina* and *Eimeria necatrix* and the like.

BEST MODE FOR CARRYING OUT THE INVENTION

Compounds (I) of the invention, i.e., the compounds represented by formula (I) (the compounds represented by the other formulae may be hereinafter referred to as in a similar manner) can be prepared, for example, via the synthetic pathway as shown below, making reference to known literature (e.g., Youji Huaxue (1989), 9(3), 226-9, Pharmazie (1985), 40(11), 767-71, Pharmazie (1985), 40(6), 387-93, Pharmazie (1985), 40(5), 307-11, J. Chem. Soc. Perkin Train. 2 (1992) 527-532, Journal of Organometallic Chemistry, 297(1985) 13-19).

Some kinds of substituents ($R^1$, $R^2$, $R^3$ and $R^4$) in the compound (for example, hydroxy group, carbonyl group, amino group, and the like) may be reactive during a reaction in the following synthetic pathway, and then any protecting group may be appropriately used to protect such substituents and removed after the reaction so that the preparation is conducted.

Reaction Scheme

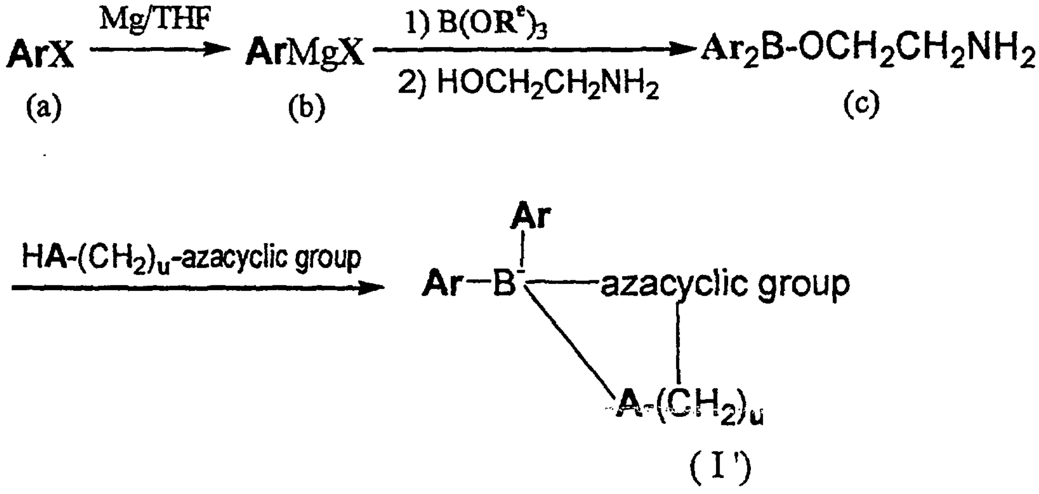


(a) (b) (c) (I')

wherein, u is an integer of 0, 1 or 2, $R^e$ is an alkyl group having 1 to 8 carbon atoms, and the other symbols are as defined above.

Step 1

First at all, compounds of formula: ArMgX (b) are prepared by reacting compound (a) with magnesium in an ether solvent such as tetrahydrofuran (THF). In this reaction, compound (a) can be used in one equivalent or more, preferably 1.1 to 1.3 equivalents relative to magnesium. Examples of useful ether solvents include tetrahydrofuran, diethyl ether, dioxane, and the like. If necessary, a mixture of an ether solvent with an aromatic hydrocarbon (for example, toluene, benzene, xylene, etc.) or a saturated hydrocarbon (for example, cyclohexane, hexane, etc.) may be used. Reaction temperature is -20 to 150 °C, and preferably 0 to 50 °C. The reaction may be conducted for 5 to 10 hours although the reaction time will vary depending on the compound to be reacted. Usually, the reaction product can be obtained in a clear solution or in a form of slurry.

Step 2

Compounds of formula (c) are prepared by reacting compound (b) as obtained above in a solution or in a form of slurry with borate compound ($B(OR^e)_3$), and then with ethanolamine ($HOCH_2CH_2NH_2$) in an appropriate solvent.

In the reaction, the borate ester can be used in 0.5 equivalents or less, preferably 0.5 to C.4 equivalents relative to compound (b) obtained in Step 1. In the subsequent reaction, ethanolamine can be used in 0.5 equivalents or more, preferably 0.5 to 0.7 equivalents relative to compound (b). If necessary in these reactions, ethers, aromatic hydrocarbons, saturated hydrocarbons as described in Step 1 may be used as a solvent. The reaction between compound (b) and a borate ester may be conducted at a temperature of -90 to 100 °C, preferably -30 to 40 °C, and may be kept for 0.5 to 10 hours, although the reaction time will vary depending on the compound to be reacted. The subsequent reaction with ethanolamine may be conducted at a temperature of 0 to 100 °C, preferably 30 to 80 °C, and usually may be kept for 0.5 to 5 hours. Compound (c) may be used in the next step as a crude product or after purification by any conventional method such as column chromatography, recrystallization and the like.

Step 3

The compound of the invention represented by the formula (I') can be prepared by reacting compound (c) with a compound of formula: HA-$(CH_2)_u$-azacyclic group in an appropriate solvent. The compound of formula HA-$(CH_2)_u$-azacyclic group can be used in 1 to 2 equivalents, preferably 1 to 1.5 equivalents relative to compound (c). Examples of the solvent which can be used include ethers (e.g., diethyl ether, tetrahydrofuran, dioxane, etc.), hydrocarbons (e.g., benzene, toluene, xylene, etc.), alcohols (e.g., methanol, ethanol, n-propanol, etc.), water, a mixture thereof, and the like. The reaction temperature may be 0 to 150 °C, preferably 20 to 120 °C, and the reaction time generally may be 0.5 to 10 hours. The desired compound (I') thus obtained, if necessary, may be purified by column chromatography, recrystallization or the like, for example.

EXAMPLES

The present invention is further described by the following Reference Examples and Examples, which are not intended to limit the scope of the present invention.

In the following Reference Examples and Examples, Ph, Naph, $Me_3Si$-Ph and t-Bu represent phenyl group, naphthyl group, trimethylsilylphenyl group and t-butyl group, respectively.

Reference Example 1

Synthesis of diphenylborate ethanolamine ester ($Ph_2B$-$OCH_2CH_2NH_2$)

A solution of phenyl magnesium bromide in tetrahydrofuran was prepared according to a conventional method using 83.2 g (0.53 mol) of bromobenzene, 12.9 g (0.53 mol) of magnesium and 250 ml of tetrahydrofuran. Subsequently, 58.5 g (0.26 mol) of tributyl borate ester and 200 ml of ethyl ether were contained in a flask, and the flask was cooled at -60 °C. To the mixture was added dropwise a solution of phenyl magnesium bromide (Grignard reagent) in tetrahydrofuran while maintaining the temperature at -60 °C. After the addition was completed, the reaction mixture was stirred for 10 hours at room temperature and hydrolyzed by addition of 150 ml of a 10% aqueous hydrochloric acid, followed by isolating the organic layer. To the organic layer were added 31.1 g (0.51 mol) of ethanolamine and 50 ml of ethanol, and the mixture was stirred for two hours at room temperature. The precipitated product was collected by filtration, recrystallized from a hydrous alcohol, and dried to afford 31.9 g (yield 55%) of diphenylborate ethanolamine ester as white crystals.

Examples 1-4 as shown below describe the preparations of compounds represented by the formula:

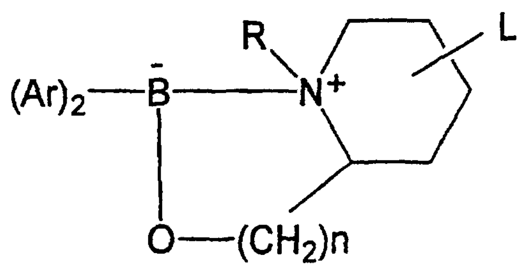

Example 1

Synthesis of compound 1 of the formula wherein n is 1, Ar is phenyl, and R and L are hydrogen

A mixture of 2.6 g (0.012 mol) of diphenylborate ethanolamine ester, 30 ml of ethyl ether and 30 ml of 10% aqueous hydrochloric acid that was contained in a funnel was mixed with shaking for about 15 minutes, and the layers were then separated. The organic layer was washed once with water and transferred to an eggplant type flask. Subsequently, 2.1 g (0.018 mol) of 2-hydroxymethylpiperidine and 7 ml of ethanol were added thereto, and the mixture was stirred for two hours at room temperature. The precipitated product was collected by filtration, recrystallized from a hydrous alcohol, and dried to afford 2.8 g (yield 83%) of compound 1 as white crystals.
Mp: 169-171°C

Example 2

Synthesis of compound 2 of the formula wherein n is 1, Ar is 2-methylphenyl, and R and L are hydrogen

Di-(2-methyl)-phenylboronic acid ethanolamine ester (3.04 g, 0.012 mol) and 2-hydroxymethylpiperidine (2.10 g, 0.018 mol) were reacted and treated in a similar manner to the synthesis of compound 1 to afford 2.18 g (yield 78%) of compound 2 as white crystals.
Mp: 126-128 °C

Example 3

Synthesis of compound 9

Di-(3-chrolo)-phenylboronic acid ethanolamine ester (3.54 g, 0.012 mol) and 2-hydroxymethylpiperidine (2.10 g, 0.018 mol) were reacted and treated in a similar manner to the synthesis of compound 1 to afford 3.28 g (yield 78%) of compound 9 as white crystals.
Mp: 155-159 °C

Example 4

Synthesis of compound 31 of the formula wherein n is 1, Ar is α-naphthyl, and R and L are hydrogen

Di-α-naphthylboronic acid ethanolamine ester (3.90 g, 0.012 mol) and 2-hydroxymethylpiperidine (2.10 g, 0.018 mol) were reacted and treated in a similar manner to the synthesis of compound 1 to afford 3.01 g (yield 79%) of compound 31 as white crystals.
Mp: 176-178 °C

The following compounds listed in Tables 1-6 can be prepared with reference to Examples 1-4 as described above.

Table 1

| Compound No. | n | Ar | R | L | m.p. (°C) |
|---|---|---|---|---|---|
| 1 | 1 | Ph | H | H | 169-171 |
| 2 | 1 | 2-$CH_3$-Ph | H | H | 126-128 |
| 3 | 1 | 3-$CH_3$-Ph | H | H | 203-205 |
| 4 | 1 | 4-$CH_3$-Ph | H | H | 134-136 |
| 5 | 1 | 2-F-Ph | H | H | |
| 6 | 1 | 3-F-Ph | H | H | |
| 7 | 1 | 4-F-Ph | H | H | 134-136 |
| 8 | 1 | 2-Cl-Ph | H | H | |
| 9 | 1 | 3-Cl-Ph | H | H | 155-159 |
| 10 | 1 | 4-Cl-Ph | H | H | |
| 11 | 1 | 2-Br-Ph | H | H | |
| 12 | 1 | 3-Br-Ph | H | H | |
| 13 | 1 | 4-Br-Ph | H | H | |
| 14 | 1 | 2-$CF_3$-Ph | H | H | |
| 15 | 1 | 3-$CF_3$-Ph | H | H | 95-97 |
| 16 | 1 | 4-$CF_3$-Ph | H | H | |
| 17 | 1 | 2-$OCH_3$-Ph | H | H | |
| 18 | 1 | 3-$OCH_3$-Ph | H | H | 148-151 |
| 19 | 1 | 4-$OCH_3$-Ph | H | H | |
| 20 | 1 | 4-tBu-Ph | H | H | |
| 21 | 1 | 2-OPh-Ph | H | H | |
| 22 | 1 | 3-OPh-Ph | H | H | |
| 23 | 1 | 4-OPh-Ph | H | H | |
| 24 | 1 | 2,4-$Cl_2$-Ph | H | H | |
| 25 | 1 | 3,4-$Cl_2$-Ph | H | H | |
| 26 | 1 | 2-Cl,4-$CF_3$-Ph | H | H | |
| 27 | 1 | 2-CF3,3-Cl-Ph | H | H | |
| 28 | 1 | 3-CF3,4-Cl-Ph | H | H | |
| 29 | 1 | 3-Cl,4-$CF_3$-Ph | H | H | |
| 30 | 1 | 3-CF3,4-$CF_3$-Ph | H | H | |
| 31 | 1 | α-Naph | H | H | 176-178 |

Table 1 (continued)

| Compound No. | n | Ar | R | L | m.p. (°C) |
|---|---|---|---|---|---|
| 32 | 1 | β-Naph | H | H | |
| 33 | 1 | 4-$Me_3$Si-Ph | H | H | |

Table 2

| Compound No. | n | Ar | R | L | m.p. (°C) |
|---|---|---|---|---|---|
| 34 | 2 | Ph | H | H | |
| 35 | 2 | 2-$CH_3$-Ph | H | H | |
| 36 | 2 | 3-$CH_3$-Ph | H | H | |
| 37 | 2 | 4-$CH_3$-Ph | H | H | |
| 38 | 2 | 2-F-Ph | H | H | |
| 39 | 2 | 3-F-Ph | H | H | |
| 40 | 2 | 4-F-Ph | H | H | |
| 41 | 2 | 2-Cl-Ph | H | H | |
| 42 | 2 | 3-Cl-Ph | H | H | |
| 43 | 2 | 4-Cl-Ph | H | H | |
| 44 | 2 | 2-Br-Ph | H | H | |
| 45 | 2 | 3-Br-Ph | H | H | |
| 46 | 2 | 4-Br-Ph | H | H | |
| 47 | 2 | 2-$CF_3$-Ph | H | H | |
| 48 | 2 | 3-$CF_3$-Ph | H | H | |
| 49 | 2 | 4-$CF_3$-Ph | H | H | |
| 50 | 2 | 2-$OCH_3$-Ph | H | H | |
| 51 | 2 | 3-$OCH_3$-Ph | H | H | |
| 52 | 2 | 4-$OCH_3$-Ph | H | H | |
| 53 | 2 | 4-tBu-Ph | H | H | |
| 54 | 2 | 2-OPh-Ph | H | H | |
| 55 | 2 | 3-OPh-Ph | H | H | |
| 56 | 2 | 4-OPh-Ph | H | H | |
| 57 | 2 | 2,4-$Cl_2$-Ph | H | H | |
| 58 | 2 | 3,4-$Cl_2$-Ph | H | H | |
| 59 | 2 | 2-Cl,4-$CF_3$-Ph | H | H | |
| 60 | 2 | 2-CF3,3-Cl-Ph | H | H | |
| 61 | 2 | 3-CF3,4-Cl-Ph | H | H | |
| 62 | 2 | 3-Cl,4-$CF_3$-Ph | H | H | |
| 63 | 2 | 3-CF3,4-$CF_3$-Ph | H | H | |
| 64 | 2 | α-Naph | H | H | |
| 65 | 2 | β-Naph | H | H | |
| 66 | 2 | 4-$Me_3$Si-Ph | H | H | |

Table 3

| Compound No. | n | Ar | R | L | m.p. (°C) |
|---|---|---|---|---|---|
| 67 | 1 | Ph | $CH_3$ | H | |
| 68 | 1 | 2-$CH_3$-Ph | $CH_3$ | H | |
| 69 | 1 | 3-$CH_3$-Ph | $CH_3$ | H | |
| 70 | 1 | 4-$CH_3$-Ph | $CH_3$ | H | |
| 71 | 1 | 2-F-Ph | $CH_3$ | H | |
| 72 | 1 | 3-F-Ph | $CH_3$ | H | |

Table 3 (continued)

| Compound No. | n | Ar | R | L | m.p. (°C) |
|---|---|---|---|---|---|
| 73 | 1 | 4-F-Ph | $CH_3$ | H | |
| 74 | 1 | 2-Cl-Ph | $CH_3$ | H | |
| 75 | 1 | 3-Cl-Ph | $CH_3$ | H | |
| 76 | 1 | 4-Cl-Ph | $CH_3$ | H | |
| 77 | 1 | 2-Br-Ph | $CH_3$ | H | |
| 78 | 1 | 3-Br-Ph | $CH_3$ | H | |
| 79 | 1 | 4-Br-Ph | $CH_3$ | H | |
| 80 | 1 | 2-$CF_3$-Ph | $CH_3$ | H | |
| 81 | 1 | 3-$CF_3$-Ph | $CH_3$ | H | |
| 82 | 1 | 4-$CF_3$-Ph | $CH_3$ | H | |
| 83 | 1 | 2-$OCH_3$-Ph | $CH_3$ | H | |
| 84 | 1 | 3-$OCH_3$-Ph | $CH_3$ | H | |
| 85 | 1 | 4-$OCH_3$-Ph | $CH_3$ | H | |
| 86 | 1 | 4-tBu-Ph | $CH_3$ | H | |
| 87 | 1 | 2-OPh-Ph | $CH_3$ | H | |
| 88 | 1 | 3-OPh-Ph | $CH_3$ | H | |
| 89 | 1 | 4-OPh-Ph | $CH_3$ | H | |
| 90 | 1 | 2,4-$Cl_2$-Ph | $CH_3$ | H | |
| 91 | 1 | 3,4-$Cl_2$-Ph | $CH_3$ | H | |
| 92 | 1 | 2-Cl,4-$CF_3$-Ph | $CH_3$ | H | |
| 93 | 1 | 2-CF3,3-Cl-Ph | $CH_3$ | H | |
| 94 | 1 | 3-CF3,4-Cl-Ph | $CH_3$ | H | |
| 95 | 1 | 3-Cl,4-$CF_3$-Ph | $CH_3$ | H | |
| 96 | 1 | 3-CF3,4-$CF_3$-Ph | $CH_3$ | H | |
| 97 | 1 | $\alpha$-Naph | $CH_3$ | H | |
| 98 | 1 | $\beta$-Naph | $CH_3$ | H | |
| 99 | 1 | 4-$Me_3$Si-Ph | $CH_3$ | H | |

Table 4

| Compound No. | n | Ar | R | L | m.p. (°C) |
|---|---|---|---|---|---|
| 100 | 2 | Ph | $CH_3$ | H | |
| 101 | 2 | 2-$CH_3$-Ph | $CH_3$ | H | |
| 102 | 2 | 3-$CH_3$-Ph | $CH_3$ | H | |
| 103 | 2 | 4-$CH_3$-Ph | $CH_3$ | H | |
| 104 | 2 | 2-F-Ph | $CH_3$ | H | |
| 105 | 2 | 3-F-Ph | $CH_3$ | H | |
| 106 | 2 | 4-F-Ph | $CH_3$ | H | |
| 107 | 2 | 2-Cl-Ph | $CH_3$ | H | |
| 108 | 2 | 3-Cl-Ph | $CH_3$ | H | |
| 109 | 2 | 4-Cl-Ph | $CH_3$ | H | |
| 110 | 2 | 2-Br-Ph | $CH_3$ | H | |
| 111 | 2 | 3-Br-Ph | $CH_3$ | H | |
| 112 | 2 | 4-Br-Ph | $CH_3$ | H | |
| 113 | 2 | 2-$CF_3$-Ph | $CH_3$ | H | |
| 114 | 2 | 3-$CF_3$-Ph | $CH_3$ | H | |
| 115 | 2 | 4-$CF_3$-Ph | $CH_3$ | H | |
| 116 | 2 | 2-$OCH_3$-Ph | $CH_3$ | H | |
| 117 | 2 | 3-$OCH_3$-Ph | $CH_3$ | H | |

Table 4 (continued)

| Compound No. | n | Ar | R | L | m.p. (°C) |
|---|---|---|---|---|---|
| 118 | 2 | 4-$OCH_3$-Ph | $CH_3$ | H | |
| 119 | 2 | 4-tBu-Ph | $CH_3$ | H | |
| 120 | 2 | 2-OPh-Ph | $CH_3$ | H | |
| 121 | 2 | 3-OPh-Ph | $CH_3$ | H | |
| 122 | 2 | 4-OPh-Ph | $CH_3$ | H | |
| 123 | 2 | 2,4-$Cl_2$-Ph | $CH_3$ | H | |
| 124 | 2 | 3,4-$Cl_2$-Ph | $CH_3$ | H | |
| 125 | 2 | 2-Cl,4-$CF_3$-Ph | $CH_3$ | H | |
| 126 | 2 | 2-CF3,3-Cl-Ph | $CH_3$ | H | |
| 127 | 2 | 3-CF3,4-Cl-Ph | $CH_3$ | H | |
| 128 | 2 | 3-Cl,4-$CF_3$-Ph | $CH_3$ | H | |
| 129 | 2 | 3-CF3,4-$CF_3$-Ph | $CH_3$ | H | |
| 130 | 2 | α-Naph | $CH_3$ | H | |
| 131 | 2 | β-Naph | $CH_3$ | H | |
| 132 | 2 | 4-$Me_3$Si-Ph | $CH_3$ | H | |

Table 5

| Compound No. | n | Ar | R | L | m.p. (°C) |
|---|---|---|---|---|---|
| 133 | 1 | Ph | H | 6-$CH_3$ | |
| 134 | 1 | 2-$CH_3$-Ph | H | 6-$CH_3$ | |
| 135 | 1 | 3-$CH_3$-Ph | H | 6-$CH_3$ | |
| 136 | 1 | 4-$CH_3$-Ph | H | 6-$CH_3$ | |
| 137 | 1 | 2-F-Ph | H | 6-$CH_3$ | |
| 138 | 1 | 3-F-Ph | H | 6-$CH_3$ | |
| 139 | 1 | 4-F-Ph | H | 6-$CH_3$ | |
| 140 | 1 | 2-Cl-Ph | H | 6-$CH_3$ | |
| 141 | 1 | 3-Cl-Ph | H | 6-$CH_3$ | |
| 142 | 1 | 4-Cl-Ph | H | 6-$CH_3$ | |
| 143 | 1 | 2-Br-Ph | H | 6-$CH_3$ | |
| 144 | 1 | 3-Br-Ph | H | 6-$CH_3$ | |
| 145 | 1 | 4-Br-Ph | H | 6-$CH_3$ | |
| 146 | 1 | 2-$CF_3$-Ph | H | 6-$CH_3$ | |
| 147 | 1 | 3-$CF_3$-Ph | H | 6-$CH_3$ | |
| 148 | 1 | 4-$CF_3$-Ph | H | 6-$CH_3$ | |
| 149 | 1 | 2-$OCH_3$-Ph | H | 6-$CH_3$ | |
| 150 | 1 | 3-$OCH_3$-Ph | H | 6-$CH_3$ | |
| 151 | 1 | 4-$OCH_3$-Ph | H | 6-$CH_3$ | |
| 152 | 1 | 4-tBu-Ph | H | 6-$CH_3$ | |
| 153 | 1 | 2-OPh-Ph | H | 6-$CH_3$ | |
| 154 | 1 | 3-OPh-Ph | H | 6-$CH_3$ | |
| 155 | 1 | 4-OPh-Ph | H | 6-$CH_3$ | |
| 156 | 1 | 2,4-$Cl_2$-Ph | H | 6-$CH_3$ | |
| 157 | 1 | 3,4-$Cl_2$-Ph | H | 6-$CH_3$ | |
| 158 | 1 | 2-Cl,4-$CF_3$-Ph | H | 6-$CH_3$ | |
| 159 | 1 | 2-CF3,3-Cl-Ph | H | 6-$CH_3$ | |
| 160 | 1 | 3-CF3,4-Cl-Ph | H | 6-$CH_3$ | |
| 161 | 1 | 3-Cl,4-$CF_3$-Ph | H | 6-$CH_3$ | |
| 162 | 1 | 3-CF3,4-$CF_3$-Ph | H | 6-$CH_3$ | |

Table 5 (continued)

| Compound No. | n | Ar | R | L | m.p. (°C) |
|---|---|---|---|---|---|
| 163 | 1 | α-Naph | H | 6-$CH_3$ | |
| 164 | 1 | β-Naph | H | 6-$CH_3$ | |
| 165 | 1 | 4-$Me_3$Si-Ph | H | 6-$CH_3$ | |

Table 6

| Compound No. | n | Ar | R | L | m.p. (°C) |
|---|---|---|---|---|---|
| 166 | 1 | Ph | H | 3,5-$Me_2$ | |
| 167 | 1 | 2-$CH_3$-Ph | H | 3,5-$Me_2$ | |
| 168 | 1 | 3-$CH_3$-Ph | H | 3,5-$Me_2$ | |
| 169 | 1 | 4-$CH_3$-Ph | H | 3,5-$Me_2$ | |
| 170 | 1 | 2-F-Ph | H | 3,5-$Me_2$ | |
| 171 | 1 | 3-F-Ph | H | 3,5-$Me_2$ | |
| 172 | 1 | 4-F-Ph | H | 3,5-$Me_2$ | |
| 173 | 1 | 2-Cl-Ph | H | 3,5-$Me_2$ | |
| 174 | 1 | 3-Cl-Ph | H | 3,5-$Me_2$ | |
| 175 | 1 | 4-Cl-Ph | H | 3,5-$Me_2$ | |
| 176 | 1 | 2-Br-Ph | H | 3,5-$Me_2$ | |
| 177 | 1 | 3-Br-Ph | H | 3,5-$Me_2$ | |
| 178 | 1 | 4-Br-Ph | H | 3,5-$Me_2$ | |
| 179 | 1 | 2-$CF_3$-Ph | H | 3,5-$Me_2$ | |
| 180 | 1 | 3-$CF_3$-Ph | H | 3,5-$Me_2$ | |
| 181 | 1 | 4-$CF_3$-Ph | H | 3,5-$Me_2$ | |
| 182 | 1 | 2-$OCH_3$-Ph | H | 3,5-$Me_2$ | |
| 183 | 1 | 3-$OCH_3$-Ph | H | 3,5-$Me_2$ | |
| 184 | 1 | 4-$OCH_3$-Ph | H | 3,5-$Me_2$ | |
| 185 | 1 | 4-tBu-Ph | H | 3,5-$Me_2$ | |
| 186 | 1 | 2-OPh-Ph | H | 3,5-$Me_2$ | |
| 187 | 1 | 3-OPh-Ph | H | 3,5-$Me_2$ | |
| 188 | 1 | 4-OPh-Ph | H | 3,5-$Me_2$ | |
| 189 | 1 | 2,4-$Cl_2$-Ph | H | 3,5-$Me_2$ | |
| 190 | 1 | 3,4-$Cl_2$-Ph | H | 3,5-$Me_2$ | |
| 191 | 1 | 2-Cl,4-$CF_3$-Ph | H | 3,5-$Me_2$ | |
| 192 | 1 | 2-CF3,3-Cl-Ph | H | 3,5-$Me_2$ | |
| 193 | 1 | 3-CF3,4-Cl-Ph | H | 3,5-$Me_2$ | |
| 194 | 1 | 3-Cl,4-$CF_3$-Ph | H | 3,5-$Me_2$ | |
| 195 | 1 | 3-CF3,4-$CF_3$-Ph | H | 3,5-$Me_2$ | |
| 196 | 1 | α-Naph | H | 3,5-$Me_2$ | |
| 197 | 1 | β-Naph | H | 3,5-$Me_2$ | |
| 198 | 1 | 4-$Me_3$Si-Ph | H | 3,5-$Me_2$ | |

Examples 5 and 6 as shown below describe the preparation of compounds represented by the formula:

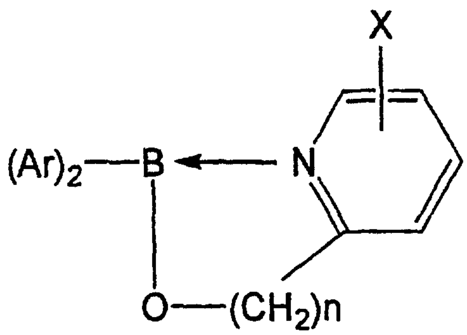

Example 5

Synthesis of compound 199 of the formula wherein n is 1, Ar is phenyl, and X is hydrogen

A mixture of 2.6 g (0.012 mol) of diphenylborate ethanolamine ester, 30 ml of ethyl ether and 30 ml of a 10% aqueous hydrochloric acid that was contained in a funnel was mixed with shaking for about 15 minutes, and the layers were then separated. The organic layer was washed once with water and transferred to an eggplant type flask.

To the mixture were added 1.94 g (0.018 mol) of 2-hydroxymethyl pyridine and 7 ml of ethanol, and the mixture was stirred for two hours at the room temperature. The precipitated product was collected by filtration, recrystallized from a hydrous alcohol and dried to afford 2.5 g (yield 76%) of compound 199 as white crystals.
Mp: 150-151°C

Example 6

Synthesis of compound 246 of the formula wherein n is 2, Ar is 3-trifluoromethyl, and X is hydrogen

Di-(3-trifluoromethyl)-phenylboronic acid ethanolamine ester (2.77 g, 0.012 mol) and 2-hydroxyethylpyridine (2.21 g, 0.018 mol) were reacted and treated in a similar manner to the synthesis of compound 1 to afford 3.96 g (yield 78%) of compound 246 as white crystals.
Mp: 140-142 °C

The following compounds listed in Tables 7-20 can be prepared with reference to Examples 5 and 6 as described above.

Table 7

| Compound No. | n | Ar | X | m.p. (°C) |
|---|---|---|---|---|
| 199 | 1 | Ph | H | 150-151 |
| 200 | 1 | 2-$CH_3$-Ph | H | 188-192 |
| 201 | 1 | 3-$CH_3$-Ph | H | 117-119 |
| 202 | 1 | 4-$CH_3$-Ph | H | 147-149 |
| 203 | 1 | 2-F-Ph | H | |
| 204 | 1 | 3-F-Ph | H | |
| 205 | 1 | 4-F-Ph | H | 137-139 |
| 206 | 1 | 2-Cl-Ph | H | |
| 207 | 1 | 3-Cl-Ph | H | |
| 208 | 1 | 4-Cl-Ph | H | 133-134 |
| 209 | 1 | 2-Br-Ph | H | |
| 210 | 1 | 3-Br-Ph | H | |
| 211 | 1 | 4-Br-Ph | H | |
| 212 | 1 | 2-$CF_3$-Ph | H | |
| 213 | 1 | 3-$CF_3$-Ph | H | 92-94 |
| 214 | 1 | 4-$CF_3$-Ph | H | |

Table 7 (continued)

| Compound No. | n | Ar | X | m.p. (°C) |
|---|---|---|---|---|
| 215 | 1 | 2-$OCH_3$-Ph | H | 163-165 |
| 216 | 1 | 3-$OCH_3$-Ph | H | |
| 217 | 1 | 4-$OCH_3$-Ph | H | 129-131 |
| 218 | 1 | 2-OPh-Ph | H | |
| 219 | 1 | 3-OPh-Ph | H | |
| 220 | 1 | 4-OPh-Ph | H | |
| 221 | 1 | 4-tBu-Ph | H | |
| 222 | 1 | 2,4-$Cl_2$-Ph | H | |
| 223 | 1 | 3,4-$Cl_2$-Ph | H | |
| 224 | 1 | 2-Cl,4-$CF_3$-Ph | H | |
| 225 | 1 | 2-CF3,3-Cl-Ph | H | |
| 226 | 1 | 3-CF3,4-Cl-Ph | H | |
| 227 | 1 | 3-Cl,4-$CF_3$-Ph | H | |
| 228 | 1 | 3-CF3,4-$CF_3$-Ph | H | |
| 229 | 1 | $\alpha$-Naph | H | 233-236 |
| 230 | 1 | $\beta$-Naph | H | |
| 231 | 1 | 4-$Me_3$Si-Ph | H | |

Table 8

| Compound No. | n | Ar | X | m.p. (°C) |
|---|---|---|---|---|
| 232 | 2 | Ph | H | 176-178 |
| 233 | 2 | 2-$CH_3$-Ph | H | 208-211 |
| 234 | 2 | 3-$CH_3$-Ph | H | |
| 235 | 2 | 4-$CH_3$-Ph | H | |
| 236 | 2 | 2-F-Ph | H | |
| 237 | 2 | 3-F-Ph | H | |
| 238 | 2 | 4-F-Ph | H | 118-120 |
| 239 | 2 | 2-Cl-Ph | H | |
| 240 | 2 | 3-Cl-Ph | H | |
| 241 | 2 | 4-Cl-Ph | H | 140-142 |
| 242 | 2 | 2-Br-Ph | H | |
| 243 | 2 | 3-Br-Ph | H | |
| 244 | 2 | 4-Br-Ph | H | |
| 245 | 2 | 2-$CF_3$-Ph | H | |
| 246 | 2 | 3-$CF_3$-Ph | H | 140-142 |
| 247 | 2 | 4-$CF_3$-Ph | H | |
| 248 | 2 | 2-$OCH_3$-Ph | H | |
| 249 | 2 | 3-$OCH_3$-Ph | H | |
| 250 | 2 | 4-$OCH_3$-Ph | H | 131-133 |
| 251 | 2 | 4-tBu-Ph | H | |
| 252 | 2 | 2-OPh-Ph | H | |
| 253 | 2 | 3-OPh-Ph | H | |
| 254 | 2 | 4-OPh-Ph | H | |
| 255 | 2 | 2,4-$Cl_2$-Ph | H | |
| 256 | 2 | 3,4-$Cl_2$-Ph | H | |
| 257 | 2 | 2-C1,4-$CF_3$-Ph | H | |
| 258 | 2 | 2-CF3,3-Cl-Ph | H | |
| 259 | 2 | 3-CF3,4-Cl-Ph | H | |

Table 8 (continued)

| Compound No. | n | Ar | X | m.p. (°C) |
|---|---|---|---|---|
| 260 | 2 | 3-Cl,4-$CF_3$-Ph | H | |
| 261 | 2 | 3-CF3,4-$CF_3$-Ph | H | |
| 262 | 2 | α-Naph | H | 215-218 |
| 263 | 2 | β-Naph | H | |
| 264 | 2 | 4-$Me_3$Si-Ph | H | |

Table 9

| Compound No. | n | Ar | X | m.p. (°C) |
|---|---|---|---|---|
| 265 | 1 | Ph | 2-Cl | |
| 266 | 1 | 2-$CH_3$-Ph | 2-Cl | |
| 267 | 1 | 3-$CH_3$-Ph | 2-Cl | |
| 268 | 1 | 4-$CH_3$-Ph | 2-Cl | |
| 269 | 1 | 2-F-Ph | 2-Cl | |
| 270 | 1 | 3-F-Ph | 2-Cl | |
| 271 | 1 | 4-F-Ph | 2-Cl | |
| 272 | 1 | 2-Cl-Ph | 2-Cl | |
| 273 | 1 | 3-Cl-Ph | 2-Cl | |
| 274 | 1 | 4-Cl-Ph | 2-Cl | |
| 275 | 1 | 2-Br-Ph | 2-Cl | |
| 276 | 1 | 3-Br-Ph | 2-Cl | |
| 277 | 1 | 4-Br-Ph | 2-Cl | |
| 278 | 1 | 2-$CF_3$-PH | 2-Cl | |
| 279 | 1 | 3-$CF_3$-Ph | 2-Cl | |
| 280 | 1 | 4-$CF_3$-Ph | 2-Cl | |
| 281 | 1 | 2-$OCH_3$-PH | 2-Cl | |
| 282 | 1 | 3-$OCH_3$-Ph | 2-Cl | |
| 283 | 1 | 4-$OCH_3$-Ph | 2-Cl | |
| 284 | 1 | 4-tBu-Ph | 2-Cl | |
| 285 | 1 | 2-OPh-Ph | 2-Cl | |
| 286 | 1 | 3-OPh-Ph | 2-Cl | |
| 287 | 1 | 4-OPh-Ph | 2-Cl | |
| 288 | 1 | 2,4-$Cl_2$-Ph | 2-Cl | |
| 289 | 1 | 3,4-$Cl_2$-Ph | 2-Cl | |
| 290 | 1 | 2-Cl,4-$CF_3$-Ph | 2-Cl | |
| 291 | 1 | 2-CF3,3-Cl-Ph | 2-Cl | |
| 292 | 1 | 3-CF3,4-Cl-Ph | 2-Cl | |
| 293 | 1 | 3-Cl,4-$CF_3$-Ph | 2-Cl | |
| 294 | 1 | 3-CF3,4-$CF_3$-Ph | 2-Cl | |
| 295 | 1 | α-Naph | 2-Cl | |
| 296 | 1 | β-Naph | 2-Cl | |
| 297 | 1 | 4-$Me_3$Si-Ph | 2-Cl | |

Table 10

| Compound No. | n | Ar | X | m.p. (°C) |
|---|---|---|---|---|
| 298 | 2 | Ph | 2-Cl | |
| 299 | 2 | 2-$CH_3$-Ph | 2-Cl | |
| 300 | 2 | 3-$CH_3$-Ph | 2-Cl | |

Table 10 (continued)

| Compound No. | n | Ar | X | m.p. (°C) |
|---|---|---|---|---|
| 301 | 2 | 4-$CH_3$-Ph | 2-Cl | |
| 302 | 2 | 2-F-Ph | 2-Cl | |
| 303 | 2 | 3-F-Ph | 2-Cl | |
| 304 | 2 | 4-F-Ph | 2-Cl | |
| 305 | 2 | 2-Cl-Ph | 2-Cl | |
| 306 | 2 | 3-Cl-Ph | 2-Cl | |
| 307 | 2 | 4-Cl-Ph | 2-Cl | |
| 308 | 2 | 2-Br-Ph | 2-Cl | |
| 309 | 2 | 3-Br-Ph | 2-Cl | |
| 310 | 2 | 4-Br-Ph | 2-Cl | |
| 311 | 2 | 2-$CF_3$-Ph | 2-Cl | |
| 312 | 2 | 3-$CF_3$-Ph | 2-Cl | |
| 313 | 2 | 4-$CF_3$-Ph | 2-Cl | |
| 314 | 2 | 2-$OCH_3$-Ph | 2-Cl | |
| 315 | 2 | 3-$OCH_3$-Ph | 2-Cl | |
| 316 | 2 | 4-$OCH_3$-Ph | 2-Cl | |
| 317 | 2 | 4-tBu-Ph | 2-Cl | |
| 318 | 2 | 2-OPh-Ph | 2-Cl | |
| 319 | 2 | 3-OPh-Ph | 2-Cl | |
| 320 | 2 | 4-OPh-Ph | 2-Cl | |
| 321 | 2 | 2,4-$Cl_2$-Ph | 2-Cl | |
| 322 | 2 | 3,4-$Cl_2$-Ph | 2-Cl | |
| 323 | 2 | 2-Cl,4-$CF_3$-Ph | 2-Cl | |
| 324 | 2 | 2-CF3,3-Cl-Ph | 2-Cl | |
| 325 | 2 | 3-CF3,4-Cl-Ph | 2-Cl | |
| 326 | 2 | 3-Cl,4-$CF_3$-Ph | 2-Cl | |
| 327 | 2 | 3-CF3,4-$CF_3$-Ph | 2-Cl | |
| 328 | 2 | α-Naph | 2-Cl | |
| 329 | 2 | β-Naph | 2-Cl | |
| 330 | 2 | 4-$Me_3Si$-Ph | 2-Cl | |

Table 11

| Compound No. | n | Ar | X | m.p. (°C) |
|---|---|---|---|---|
| 331 | 1 | Ph | 3-Cl | |
| 332 | 1 | 2-$CH_3$-Ph | 3-Cl | |
| 333 | 1 | 3-$CH_3$-Ph | 3-Cl | |
| 334 | 1 | 4-$CH_3$-Ph | 3-Cl | |
| 335 | 1 | 2-F-Ph | 3-Cl | |
| 336 | 1 | 3-F-Ph | 3-Cl | |
| 337 | 1 | 4-F-Ph | 3-Cl | |
| 338 | 1 | 2-Cl-Ph | 3-Cl | |
| 339 | 1 | 3-Cl-Ph | 3-Cl | |
| 340 | 1 | 4-Cl-Ph | 3-Cl | |
| 341 | 1 | 2-Br-Ph | 3-Cl | |
| 342 | 1 | 3-Br-Ph | 3-Cl | |
| 343 | 1 | 4-Br-Ph | 3-Cl | |
| 344 | 1 | 2-$CF_3$-Ph | 3-Cl | |
| 345 | 1 | 3-$CF_3$-Ph | 3-Cl | |

Table 11 (continued)

| Compound No. | n | Ar | X | m.p. (°C) |
|---|---|---|---|---|
| 346 | 1 | 4-$CF_3$-Ph | 3-Cl | |
| 347 | 1 | 2-$OCH_3$-Ph | 3-Cl | |
| 348 | 1 | 3-$OCH_3$-Ph | 3-Cl | |
| 349 | 1 | 4-$OCH_3$-Ph | 3-Cl | |
| 350 | 1 | 2-OPh-Ph | 3-Cl | |
| 351 | 1 | 4-tBu-Ph | 3-Cl | |
| 352 | 1 | 3-OPh-Ph | 3-Cl | |
| 353 | 1 | 4-OPh-Ph | 3-Cl | |
| 354 | 1 | 2,4-$Cl_2$-Ph | 3-Cl | |
| 355 | 1 | 3,4-$Cl_2$-Ph | 3-Cl | |
| 356 | 1 | 2-Cl,4-$CF_3$-Ph | 3-Cl | |
| 357 | 1 | 2-CF3,3-Cl-Ph | 3-Cl | |
| 358 | 1 | 3-CF3,4-Cl-Ph | 3-Cl | |
| 359 | 1 | 3-Cl,4-$CF_3$-Ph | 3-Cl | |
| 360 | 1 | 3-CF3,4-$CF_3$-Ph | 3-Cl | |
| 361 | 1 | α-Naph | 3-Cl | |
| 362 | 1 | β-Naph | 3-Cl | |
| 363 | 1 | 4-$Me_3$Si-Ph | 3-Cl | |

Table 12

| Compound No. | n | Ar | X | m.p. (°C) |
|---|---|---|---|---|
| 364 | 2 | Ph | 3-Cl | |
| 365 | 2 | 2-$CH_3$-Ph | 3-Cl | |
| 366 | 2 | 3-$CH_3$-Ph | 3-Cl | |
| 367 | 2 | 4-$CH_3$-Ph | 3-Cl | |
| 368 | 2 | 2-F-Ph | 3-Cl | |
| 369 | 2 | 3-F-Ph | 3-Cl | |
| 370 | 2 | 4-F-Ph | 3-Cl | |
| 371 | 2 | 2-Cl-Ph | 3-Cl | |
| 372 | 2 | 3-Cl-Ph | 3-Cl | |
| 373 | 2 | 4-Cl-Ph | 3-Cl | |
| 374 | 2 | 2-Br-Ph | 3-Cl | |
| 375 | 2 | 3-Br-Ph | 3-Cl | |
| 376 | 2 | 4-Br-Ph | 3-Cl | |
| 377 | 2 | 2-$CF_3$-Ph | 3-Cl | |
| 378 | 2 | 3-$CF_3$-Ph | 3-Cl | |
| 379 | 2 2 | 4-$CF_3$-Ph | 3-Cl | |
| 380 | 2 | 2-$OCH_3$-Ph | 3-Cl | |
| 381 | 2 | 3-$OCH_3$-Ph | 3-Cl | |
| 382 | 2 | 4-$OCH_3$-Ph | 3-Cl | |
| 383 | 2 | 4-tBu-Ph | 3-Cl | |
| 384 | 2 | 2-OPh-Ph | 3-Cl | |
| 385 | 2 | 3-OPh-Ph | 3-Cl | |
| 386 | 2 | 4-OPh-Ph | 3-Cl | |
| 387 | 2 | 2,4-$Cl_2$-Ph | 3-Cl | |
| 388 | 2 | 3,4-$Cl_2$-Ph | 3-Cl | |
| 389 | 2 | 2-Cl,4-$CF_3$-Ph | 3-Cl | |
| 390 | 2 | 2-CF3,3-Cl-Ph | 3-Cl | |

Table 12 (continued)

| Compound No. | n | Ar | X | m.p. (°C) |
|---|---|---|---|---|
| 391 | 2 | 3-CF3,4-Cl-Ph | 3-Cl | |
| 392 | 2 | 3-Cl,4-$CF_3$-Ph | 3-Cl | |
| 393 | 2 | 3-CF3,4-$CF_3$-Ph | 3-Cl | |
| 394 | 2 | α-Naph | 3-Cl | |
| 395 | 2 | β-Naph | 3-Cl | |
| 396 | 2 | 4-$Me_3Si$-Ph | 3-Cl | |

Table 13

| Compound No. | n | Ar | X | m.p. (°C) |
|---|---|---|---|---|
| 397 | 1 | Ph | 4-Cl | |
| 398 | 1 | 2-$CH_3$-Ph | 4-Cl | |
| 399 | 1 | 3-$CH_3$-Ph | 4-Cl | |
| 400 | 1 | 4-$CH_3$-Ph | 4-Cl | |
| 401 | 1 | 2-F-Ph | 4-Cl | |
| 402 | 1 | 3-F-Ph | 4-Cl | |
| 403 | 1 | 4-F-Ph | 4-Cl | |
| 404 | 1 | 2-Cl-Ph | 4-Cl | |
| 405 | 1 | 3-Cl-Ph | 4-Cl | |
| 406 | 1 | 4-Cl-Ph | 4-Cl | |
| 407 | 1 | 2-Br-Ph | 4-Cl | |
| 408 | 1 | 3-Br-Ph | 4-Cl | |
| 409 | 1 | 4-Br-Ph | 4-Cl | |
| 410 | 1 | 2-$CF_3$-Ph | 4-Cl | |
| 411 | 1 | 3-$CF_3$-Ph | 4-Cl | |
| 412 | 1 | 4-$CF_3$-Ph | 4-Cl | |
| 413 | 1 | 2-$OCH_3$-Ph | 4-Cl | |
| 414 | 1 | 3-$OCH_3$-Ph | 4-Cl | |
| 415 | 1 | 4-$OCH_3$-Ph | 4-Cl | |
| 416 | 1 | 4-tBu-Ph | 4-Cl | |
| 417 | 1 | 2-OPh-Ph | 4-Cl | |
| 418 | 1 | 3-OPh-Ph | 4-Cl | |
| 419 | 1 | 4-OPh-Ph | 4-Cl | |
| 420 | 1 | 2,4-$Cl_2$-Ph | 4-Cl | |
| 421 | 1 | 3,4-$Cl_2$-Ph | 4-Cl | |
| 422 | 1 | 2-Cl,4-$CF_3$-Ph | 4-Cl | |
| 423 | 1 | 2-CF3,3-Cl-Ph | 4-Cl | |
| 424 | 1 | 3-CF3,4-Cl-Ph | 4-Cl | |
| 425 | 1 | 3-Cl,4-$CF_3$-Ph | 4-Cl | |
| 426 | 1 | 3-CF3,4-$CF_3$-Ph | 4-Cl | |
| 427 | 1 | α-Naph | 4-Cl | |
| 428 | 1 | β-Naph | 4-Cl | |
| 429 | 1 | 4-$Me_3Si$-Ph | 4-Cl | |

Table 14

| Compound No. | n | Ar | X | m.p. (°C) |
|---|---|---|---|---|
| 430 | 2 | Ph | 4-Cl | |
| 431 | 2 | 2-$CH_3$-Ph | 4-Cl | |

Table 14 (continued)

| Compound No. | n | Ar | X | m.p. (°C) |
|---|---|---|---|---|
| 432 | 2 | 3-$CH_3$-Ph | 4-Cl | |
| 433 | 2 | 4-$CH_3$-Ph | 4-Cl | |
| 434 | 2 | 2-F-Ph | 4-Cl | |
| 435 | 2 | 3-F-Ph | 4-Cl | |
| 436 | 2 | 4-F-Ph | 4-Cl | |
| 437 | 2 | 2-Cl-Ph | 4-Cl | |
| 438 | 2 | 3-Cl-Ph | 4-Cl | |
| 439 | 2 | 4-Cl-Ph | 4-Cl | |
| 440 | 2 | 2-Br-Ph | 4-Cl | |
| 441 | 2 | 3-Br-Ph | 4-Cl | |
| 442 | 2 | 4-Br-Ph | 4-Cl | |
| 443 | 2 | 2-$CF_3$-Ph | 4-Cl | |
| 444 | 2 | 3-$CF_3$-Ph | 4-Cl | |
| 445 | 2 | 4-$CF_3$-Ph | 4-Cl | |
| 446 | 2 | 2-$OCH_3$-Ph | 4-Cl | |
| 447 | 2 | 3-$OCH_3$-Ph | 4-Cl | |
| 448 | 2 | 4-$OCH_3$-Ph | 4-Cl | |
| 449 | 2 | 4-tBu-Ph | 4-Cl | |
| 450 | 2 | 2-OPh-Ph | 4-Cl | |
| 451 | 2 | 3-OPh-Ph | 4-Cl | |
| 452 | 2 | 4-OPh-Ph | 4-Cl | |
| 453 | 2 | 2,4-$Cl_2$-Ph | 4-Cl | |
| 454 | 2 | 3,4-$Cl_2$-Ph | 4-Cl | |
| 455 | 2 | 2-Cl,4-$CF_3$-Ph | 4-Cl | |
| 456 | 2 | 2-CF3,3-Cl-Ph | 4-Cl | |
| 457 | 2 | 3-CF3,4-Cl-Ph | 4-Cl | |
| 458 | 2 | 3-Cl,4-$CF_3$-Ph | 4-Cl | |
| 459 | 2 | 3-CF3,4-$CF_3$-Ph | 4-Cl | |
| 460 | 2 | $\alpha$-Naph | 4-Cl | |
| 461 | 2 | $\beta$-Naph | 4-Cl | |
| 462 | 2 | 4-$Me_3$Si-Ph | 4-Cl | |

Table 15

| Compound No. | n | AT | X | m.p. (°C) |
|---|---|---|---|---|
| 463 | 1 | Ph | 4-$CF_3$ | |
| 464 | 1 | 2-$CH_3$-Ph | 4-$CF_3$ | |
| 465 | 1 | 3-$CH_3$-Ph | 4-$CF_3$ | |
| 466 | 1 | 4-$CH_3$-Ph | 4-$CF_3$ | |
| 467 | 1 | 2-F-Ph | 4-$CF_3$ | |
| 468 | 1 | 3-F-Ph | 4-$CF_3$ | |
| 469 | 1 | 4-F-Ph | 4-$CF_3$ | |
| 470 | 1 | 2-Cl-Ph | 4-$CF_3$ | |
| 471 | 1 | 3-Cl-Ph | 4-$CF_3$ | |
| 472 | 1 | 4-Cl-Ph | 4-$CF_3$ | |
| 473 | 1 | 2-Br-Ph | 4-$CF_3$ | |
| 474 | 1 | 3-Br-Ph | 4-$CF_3$ | |
| 475 | 1 | 4-Br-Ph | 4-$CF_3$ | |
| 476 | 1 | 2-$CF_3$-Ph | 4-$CF_3$ | |

Table 15 (continued)

| Compound No. | n | AT | X | m.p. (°C) |
|---|---|---|---|---|
| 477 | 1 | 3-$CF_3$-Ph | 4-$CF_3$ | |
| 478 | 1 | 4-$CF_3$-Ph | 4-$CF_3$ | |
| 479 | 1 | 2-$OCH_3$-Ph | 4-$CF_3$ | |
| 480 | 1 | 3-$OCH_3$-PH | 4-$CF_3$ | |
| 481 | 1 | 4-$OCH_3$-Ph | 4-$CF_3$ | |
| 482 | 1 | 4-tBu-Ph | 4-$CF_3$ | |
| 483 | 1 | 2-OPh-Ph | 4-$CF_3$ | |
| 484 | 1 | 3-OPh-Ph | 4-$CF_3$ | |
| 485 | 1 | 4-OPh-Ph | 4-$CF_3$ | |
| 486 | 1 | 2,4-$Cl_2$-Ph | 4-$CF_3$ | |
| 487 | 1 | 3,4-$Cl_2$-Ph | 4-$CF_3$ | |
| 488 | 1 | 2-Cl,4-$CF_3$-Ph | 4-$CF_3$ | |
| 489 | 1 | 2-CF3,3-Cl-Ph | 4-$CF_3$ | |
| 490 | 1 | 3-CF3,4-Cl-Ph | 4-$CF_3$ | |
| 491 | 1 | 3-Cl,4-$CF_3$-Ph | 4-$CF_3$ | |
| 492 | 1 | 3-CF3,4-$CF_3$-Ph | 4-$CF_3$ | |
| 493 | 1 | α-Naph | 4-$CF_3$ | |
| 494 | 1 | β-Naph | 4-$CF_3$ | |
| 495 | 1 | 4-$Me_3$Si-Ph | 4-$CF_3$ | |

Table 16

| Compound No. | n | Ar | X | m.p. (°C) |
|---|---|---|---|---|
| 496 | 2 | Ph | 4-$CF_3$ | |
| 497 | 2 | 2-$CH_3$-Ph | 4-$CF_3$ | |
| 498 | 2 | 3-$CH_3$-Ph | 4-$CF_3$ | |
| 499 | 2 | 4-$CH_3$-Ph | 4-$CF_3$ | |
| 500 | 2 | 2-F-Ph | 4-$CF_3$ | |
| 501 | 2 | 3-F-Ph | 4-$CF_3$ | |
| 502 | 2 | 4-F-Ph | 4-$CF_3$ | |
| 503 | 2 | 2-Cl-Ph | 4-$CF_3$ | |
| 504 | 2 | 3-Cl-Ph | 4-$CF_3$ | |
| 505 | 2 | 4-Cl-Ph | 4-$CF_3$ | |
| 506 | 2 | 2-Br-Ph | 4-$CF_3$ | |
| 507 | 2 | 3-Br-Ph | 4-$CF_3$ | |
| 508 | 2 | 4-Br-Ph | 4-$CF_3$ | |
| 509 | 2 | 2-$CF_3$-PH | 4-$CF_3$ | |
| 510 | 2 | 3-$CF_3$-Ph | 4-$CF_3$ | |
| 511 | 2 | 4-$CF_3$-Ph | 4-$CF_3$ | |
| 512 | 2 | 2-$OCH_3$-Ph | 4-CF3 | |
| 513 | 2 | 3-$OCH_3$-Ph | 4-$CF_3$ | |
| 514 | 2 | 4-$OCH_3$-Ph | 4-$CF_3$ | |
| 515 | 2 | 4-tBu-Ph | 4-$CF_3$ | |
| 516 | 2 | 2-OPh-Ph | 4-$CF_3$ | |
| 517 | 2 | 3-OPh-Ph | 4-$CF_3$ | |
| 518 | 2 | 4-OPh-Ph | 4-$CF_3$ | |
| 519 | 2 | 2,4-$Cl_2$-Ph | 4-$CF_3$ | |
| 520 | 2 | 3,4-$Cl_2$-Ph | 4-$CF_3$ | |
| 521 | 2 | 2-Cl,4-$CF_3$-Ph | 4-$CF_3$ | |

Table 16 (continued)

| Compound No. | n | Ar | X | m.p. (°C) |
|---|---|---|---|---|
| 522 | 2 | 2-CF3,3-Cl-Ph | 4-$CF_3$ | |
| 523 | 2 | 3-CF3,4-Cl-Ph | 4-$CF_3$ | |
| 524 | 2 | 3-Cl,4-$CF_3$-Ph | 4-$CF_3$ | |
| 525 | 2 | 3-CF3,4-$CF_3$-Ph | 4-$CF_3$ | |
| 526 | 2 | α-Naph | 4-$CF_3$ | |
| 527 | 2 | β-Naph | 4-$CF_3$ | |
| 528 | 2 | 4-$Me_3$Si-Ph | 4-$CF_3$ | |

Table 17

| Compound No. | n | Ar | X | m.p. (°C) |
|---|---|---|---|---|
| 529 | 1 | Ph | 5-$CF_3$ | |
| 530 | 1 | 2-$CH_3$-Ph | 5-$CF_3$ | |
| 531 | 1 | 3-$CH_3$-Ph | 5-$CF_3$ | |
| 532 | 1 | 4-$CH_3$-Ph | 5-$CF_3$ | |
| 533 | 1 | 2-F-Ph | 5-$CF_3$ | |
| 534 | 1 | 3-F-Ph | 5-$CF_3$ | |
| 535 | 1 | 4-F-Ph | 5-$CF_3$ | |
| 536 | 1 | 2-Cl-Ph | 5-$CF_3$ | |
| 537 | 1 | 3-Cl-Ph | 5-$CF_3$ | |
| 538 | 1 | 4-Cl-Ph | 5-$CF_3$ | |
| 539 | 1 | 2-Br-Ph | 5-$CF_3$ | |
| 540 | 1 | 3-Br-Ph | 5-$CF_3$ | |
| 541 | 1 | 4-Br-Ph | 5-$CF_3$ | |
| 542 | 1 | 2-$CF_3$-Ph | 5-$CF_3$ | |
| 543 | 1 | 3-$CF_3$-Ph | 5-$CF_3$ | |
| 544 | 1 | 4-$CF_3$-Ph | 5-$CF_3$ | |
| 545 | 1 | 2-$OCH_3$-Ph | 5-$CF_3$ | |
| 546 | 1 | 3-$OCH_3$-Ph | 5-$CF_3$ | |
| 547 | 1 | 4-$OCH_3$-Ph | 5-$CF_3$ | |
| 548 | 1 | 4-tBu-Ph | 5-$CF_3$ | |
| 549 | 1 | 2-OPh-Ph | 5-$CF_3$ | |
| 550 | 1 | 3-OPh-Ph | 5-$CF_3$ | |
| 551 | 1 | 4-OPh-Ph | 5-$CF_3$ | |
| 552 | 1 | 2,4-$Cl_2$-Ph | 5-$CF_3$ | |
| 553 | 1 | 3,4-$Cl_2$-Ph | 5-$CF_3$ | |
| 554 | 1 | 2-Cl,4-$CF_3$-Ph | 5-$CF_3$ | |
| 555 | 1 | 2-CF3,3-Cl-Ph | 5-$CF_3$ | |
| 556 | 1 | 3-CF3,4-Cl-Ph | 5-$CF_3$ | |
| 557 | 1 | 3-Cl,4-$CF_3$-Ph | 5-$CF_3$ | |
| 558 | 1 | 3-CF3,4-$CF_3$-Ph | 5-$CF_3$ | |
| 559 | 1 | α-Naph | 5-$CF_3$ | |
| 560 | 1 | β-Naph | 5-$CF_3$ | |
| 561 | 1 | 4-$Me_3$Si-Ph | 5-$CF_3$ | |

Table 18

| Compound No. | n | Ar | X | m.p. (°C) |
|---|---|---|---|---|
| 562 | 2 | Ph | 5-$CF_3$ | |

Table 18 (continued)

| Compound No. | n | Ar | X | m.p. (°C) |
|---|---|---|---|---|
| 563 | 2 | 2-$CH_3$-Ph | 5-$CF_3$ | |
| 564 | 2 | 3-$CH_3$-Ph | 5-$CF_3$ | |
| 565 | 2 | 4-$CH_3$-Ph | 5-$CF_3$ | |
| 566 | 2 | 2-F-Ph | 5-$CF_3$ | |
| 567 | 2 | 3-F-Ph | 5-$CF_3$ | |
| 568 | 2 | 4-F-Ph | 5-$CF_3$ | |
| 569 | 2 | 2-Cl-Ph | 5-$CF_3$ | |
| 570 | 2 | 3-Cl-Ph | 5-$CF_3$ | |
| 571 | 2 | 4-Cl-Ph | 5-$CF_3$ | |
| 572 | 2 | 2-Br-Ph | 5-$CF_3$ | |
| 573 | 2 | 3-Br-Ph | 5-$CF_3$ | |
| 574 | 2 | 4-Br-Ph | 5-$CF_3$ | |
| 575 | 2 | 2-$CF_3$-Ph | 5-$CF_3$ | |
| 576 | 2 | 3-$CF_3$-Ph | 5-$CF_3$ | |
| 577 | 2 | 4-$CF_3$-Ph | 5-$CF_3$ | |
| 578 | 2 | 2-$OCH_3$-Ph | 5-$CF_3$ | |
| 579 | 2 | 3-$OCH_3$-Ph | 5-$CF_3$ | |
| 580 | 2 | 4-$OCH_3$-Ph | 5-$CF_3$ | |
| 581 | 2 | 4-tBu-Ph | 5-$CF_3$ | |
| 582 | 2 | 2-OPh-Ph | 5-$CF_3$ | |
| 583 | 2 | 3-OPh-Ph | 5-$CF_3$ | |
| 584 | 2 | 4-OPh-Ph | 5-$CF_3$ | |
| 585 | 2 | 2,4-$Cl_2$-Ph | 5-$CF_3$ | |
| 586 | 2 | 3,4-$Cl_2$-Ph | 5-$CF_3$ | |
| 587 | 2 | 2-Cl,4-$CF_3$-Ph | 5-$CF_3$ | |
| 588 | 2 | 2-CF3,3-Cl-Ph | 5-$CF_3$ | |
| 589 | 2 | 3-CF3,4-Cl-Ph | 5-$CF_3$ | |
| 590 | 2 | 3-Cl,4-$CF_3$-Ph | 5-$CF_3$ | |
| 591 | 2 | 3-CF3,4-$CF_3$-Ph | 5-$CF_3$ | |
| 592 | 2 | $\alpha$-Naph | 5-$CF_3$ | |
| 593 | 2 | $\beta$-Naph | 5-$CF_3$ | |
| 594 | 2 | 4-$Me_3Si$-Ph | 5-$CF_3$ | |

Table 19

| Compound No. | n | Ar | X | m.p. (°C) |
|---|---|---|---|---|
| 628 | 1 | Ph | 2,4-$Cl_2$ | |
| 629 | 1 | 2-$CH_3$-Ph | 2,4-$Cl_2$ | |
| 630 | 1 | 3-$CH_3$-Ph | 2,4-$Cl_2$ | |
| 631 | 1 | 4-$CH_3$-Ph | 2,4-$Cl_2$ | |
| 632 | 1 | 2-F-Ph | 2,4-$Cl_2$ | |
| 633 | 1 | 3-F-Ph | 2,4-$Cl_2$ | |
| 634 | 1 | 4-F-Ph | 2,4-$Cl_2$ | |
| 635 | 1 | 2-Cl-Ph | 2,4-$Cl_2$ | |
| 636 | 1 | 3-Cl-Ph | 2,4-$Cl_2$ | |
| 637 | 1 | 4-Cl-Ph | 2,4-$Cl_2$ | |
| 638 | 1 | 2-Br-Ph | 2,4-$Cl_2$ | |
| 639 | 1 | 3-Br-Ph | 2,4-$Cl_2$ | |
| 640 | 1 | 4-Br-Ph | 2,4-$Cl_2$ | |

Table 19 (continued)

| Compound No. | n | Ar | X | m.p. (°C) |
|---|---|---|---|---|
| 641 | 1 | 2-$CF_3$-Ph | 2.4-$Cl_2$ | |
| 642 | 1 | 3-$CF_3$-Ph | 2,4-$Cl_2$ | |
| 643 | 1 | 4-$CF_3$-Ph | 2,4-$Cl_2$ | |
| 644 | 1 | 2-$OCH_3$-Ph | 2,4-$Cl_2$ | |
| 645 | 1 | 3-$OCH_3$-Ph | 2,4-$Cl_2$ | |
| 646 | 1 | 4-$OCH_3$-Ph | 2,4-$Cl_2$ | |
| 647 | 1 | 4-tBu-Ph | 2,4-$Cl_2$ | |
| 648 | 1 | 2-OPh-Ph | 2,4-$Cl_2$ | |
| 649 | 1 | 3-OPh-Ph | 2,4-$Cl_2$ | |
| 650 | 1 | 4-OPh-Ph | 2,4-$Cl_2$ | |
| 651 | 1 | 2,4-$Cl_2$-Ph | 2,4-$Cl_2$ | |
| 652 | 1 | 3,4-$Cl_2$-Ph | 2,4-$Cl_2$ | |
| 653 | 1 | 2-Cl,4-$CF_3$-Ph | 2,4-$Cl_2$ | |
| 654 | 1 | 2-CF3,3-Cl-Ph | 2,4-$Cl_2$ | |
| 655 | 1 | 3-CF3,4-Cl-Ph | 2,4-$Cl_2$ | |
| 656 | 1 | 3-Cl,4-$CF_3$-Ph | 2,4-$Cl_2$ | |
| 657 | 1 | 3-CF3,4-$CF_3$-Ph | 2,4-$Cl_2$ | |
| 658 | 1 | α-Naph | 2,4-$Cl_2$ | |
| 659 | 1 | β-Naph | 2,4-$Cl_2$ | |
| 660 | 1 | 4-$Me_3Si$-Ph | 2,4-$Cl_2$ | |

Table 20

| Compound No. | n | Ar | X | m.p. (°C) |
|---|---|---|---|---|
| 661 | 2 | Ph | 2,4-$Cl_2$ | |
| 662 | 2 | 2-$CH_3$-Ph | 2,4-$Cl_2$ | |
| 663 | 2 | 3-$CH_3$-Ph | 2,4-$Cl_2$ | |
| 664 | 2 | 4-$CH_3$-Ph | 2,4-$Cl_2$ | |
| 665 | 2 | 2-F-Ph | 2,4-$Cl_2$ | |
| 666 | 2 | 3-F-Ph | 2,4-$Cl_2$ | |
| 667 | 2 | 4-F-Ph | 2,4-$Cl_2$ | |
| 668 | 2 | 2-Cl-Ph | 2,4-$Cl_2$ | |
| 669 | 2 | 3-Cl-Ph | 2,4-$Cl_2$ | |
| 670 | 2 | 4-Cl-Ph | 2,4-$Cl_2$ | |
| 671 | 2 | 2-Br-Ph | 2,4-$Cl_2$ | |
| 672 | 2 | 3-Br-Ph | 2,4-$Cl_2$ | |
| 673 | 2 | 4-Br-Ph | 2,4-$Cl_2$ | |
| 674 | 2 | 2-$CF_3$-Ph | 2,4-$Cl_2$ | |
| 675 | 2 | 3-$CF_3$-Ph | 2,4-$Cl_2$ | |
| 676 | 2 | 4-$CF_3$-Ph | 2,4-$Cl_2$ | |
| 677 | 2 | 2-$OCH_3$-Ph | 2,4-$Cl_2$ | |
| 678 | 2 | 3-$OCH_3$-Ph | 2,4-$Cl_2$ | |
| 679 | 2 | 4-$OCH_3$-Ph | 2,4-$Cl_2$ | |
| 680 | 2 | 4-tBu-Ph | 2,4-$Cl_2$ | |
| 681 | 2 | 2-OPh-Ph | 2,4-$Cl_2$ | |
| 682 | 2 | 3-OPh-Ph | 2,4-$Cl_2$ | |
| 683 | 2 | 4-OPh-Ph | 2,4-$Cl_2$ | |
| 684 | 2 | 2,4-$Cl_2$-Ph | 2,4-$Cl_2$ | |
| 685 | 2 | 3,4-$Cl_2$-Ph | 2,4-$Cl_2$ | |

Table 20 (continued)

| Compound No. | n | Ar | X | m.p. (°C) |
|---|---|---|---|---|
| 686 | 2 | 2-Cl,4-$CF_3$-Ph | 2,4-$Cl_2$ | |
| 687 | 2 | 2-CF3,3-Cl-Ph | 2,4-$Cl_2$ | |
| 688 | 2 | 3-CF3,4-Cl-Ph | 2,4-$Cl_2$ | |
| 689 | 2 | 3-Cl,4-$CF_3$-Ph | 2,4-$Cl_2$ | |
| 690 | 2 | 3-CF3,4-$CF_3$-Ph | 2,4-$Cl_2$ | |
| 691 | 2 | α-Naph | 2,4-$Cl_2$ | |
| 692 | 2 | β-Naph | 2,4-$Cl_2$ | |
| 693 | 2 | 4-$Me_3$Si-Ph | 2,4-$Cl_2$ | |

Examples 7-9 as shown below describe the preparation of compounds represented by the formula:

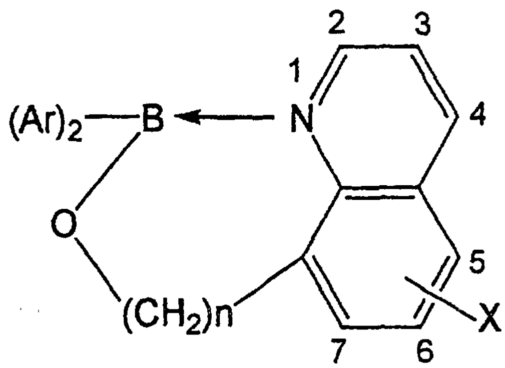

Example 7

Synthesis of compound 697 of the formula wherein n is 0, Ar is 4-methylphenyl, and X is hydrogen

Di-(4-methyl)-phenylboronic acid ethanolamine ester (3.04 g, 0.012 mol) and 8-oxyquinoline (2.61 g, 0.018 mol) were reacted and treated in a similar manner to the synthesis of compound 1 to afford 3.28 g (yield 81%) of compound 697 as white crystals.
Mp: 203-205 °C

Example 8

Synthesis of compound 708 of the formula wherein n is 0, Ar is 3-trifluoromethylphenyl, and X is hydrogen

Di-(3-trifluoromethyl)-phenylboronic acid ethanolamine ester (2.77 g, 0.012 mol) and 8-oxyquinoline (2.61 g, 0.018 mol) were reacted and treated in a similar manner to the synthesis of compound 1 to afford 4.31 g (yield 80.8%) of compound 708 as white crystals.
Mp: 130-132 °C

Example 9

Synthesis of compound 724 of the formula wherein n is 0, Ar is a-naphthyl, and X is hydrogen

Di-α-naphthylboronic acid ethanolamine ester (3.90 g, 0.012 mol) and 8-oxyquinoline (2.61 g, 0.018 mol) were reacted and treated in a similar manner to the synthesis of compound 1 to afford 4.11 g (yield 86.3%) of compound 724 as white crystals.
Mp: 225-226°C

The following compounds listed in Tables 21-32 can be prepared with reference to Examples 7-9 as described above.

Table 21

| Compound No. | n | Ar | X | m.p. (°C) |
|---|---|---|---|---|
| 694 | 0 | Ph | H | 206-207 |
| 695 | 0 | 2-$CH_3$-Ph | H | 189-191 |
| 696 | 0 | 3-$CH_3$-Ph | H | 165-167 |
| 697 | 0 | 4-$CH_3$-Ph | H | 203-205 |
| 698 | 0 | 2-F-Ph | H | |
| 699 | 0 | 3-F-Ph | H | |
| 700 | 0 | 4-F-Ph | H | 167-169 |
| 701 | 0 | 2-Cl-Ph | H | |
| 702 | 0 | 3-Cl-Ph | H | |
| 703 | 0 | 4-Cl-Ph | H | 181-182 |
| 704 | 0 | 2-Br-Ph | H | |
| 705 | 0 | 3-Br-Ph | H | |
| 706 | 0 | 4-Br-Ph | H | |
| 707 | 0 | 2-$CF_3$-Ph | H | |
| 708 | 0 | 3-$CF_3$-Ph | H | 130-132 |
| 709 | 0 | 4-$CF_3$-Ph | H | |
| 710 | 0 | 2-$OCH_3$-Ph | H | 199-200 |
| 711 | 0 | 3-$OCH_3$-Ph | H | |
| 712 | 0 | 4-$OCH_3$-Ph | H | 212-214 |
| 713 | 0 | 4-tBu-Ph | H | |
| 714 | 0 | 2-OPh-Ph | H | |
| 715 | 0 | 3-OPh-Ph | H | |
| 716 | 0 | 4-OPh-Ph | H | |
| 717 | 0 | 2,4-$Cl_2$-Ph | H | |
| 718 | 0 | 3,4-$Cl_2$-Ph | H | |
| 719 | 0 | 2-Cl,4-$CF_3$-Ph | H | |
| 720 | 0 | 2-CF3,3-Cl-Ph | H | |
| 721 | 0 | 3-CF3,4-Cl-Ph | H | |
| 722 | 0 | 3-Cl,4-$CF_3$-Ph | H | |
| 723 | 0 | 3-CF3,4-$CF_3$-Ph | H | |
| 724 | 0 | α-Naph | H | 225-226 |
| 725 | 0 | β-Naph | H | |
| 726 | 0 | 4-$Me_3$Si-Ph | H | |

Table 22

| Compound No. | n | Ar | X | m.p. (°C) |
|---|---|---|---|---|
| 727 | 1 | Ph | H | |
| 728 | 1 | 2-$CH_3$-Ph | H | |
| 729 | 1 | 3-$CH_3$-Ph | H | |
| 730 | 1 | 4-$CH_3$-Ph | H | |
| 731 | 1 | 2-F-Ph | H | |
| 732 | 1 | 3-F-Ph | H | |
| 733 | 1 | 4-F-Ph | H | |
| 734 | 1 | 2-Cl-Ph | H | |
| 735 | 1 | 3-Cl-Ph | H | |
| 736 | 1 | 4-Cl-Ph | H | |
| 737 | 1 | 2-Br-Ph | H | |

Table 22 (continued)

| Compound No. | n | Ar | X | m.p. (°C) |
|---|---|---|---|---|
| 738 | 1 | 3-Br-Ph | H | |
| 739 | 1 | 4-Br-Ph | H | |
| 740 | 1 | 2-$CF_3$-Ph | H | |
| 741 | 1 | 3-$CF_3$-Ph | H | |
| 742 | 1 | 4-$CF_3$-Ph | H | |
| 743 | 1 | 2-$OCH_3$-Ph | H | |
| 744 | 1 | 3-$OCH_3$-Ph | H | |
| 745 | 1 | 4-$OCH_3$-Ph | H | |
| 746 | 1 | 4-tBu-Ph | H | |
| 747 | 1 | 2-OPh-Ph | H | |
| 748 | 1 | 3-OPh-Ph | H | |
| 749 | 1 | 4-OPh-Ph | H | |
| 750 | 1 | 2,4-$Cl_2$-Ph | H | |
| 751 | 1 | 3,4-$Cl_2$-Ph | H | |
| 752 | 1 | 2-Cl,4-$CF_3$-Ph | H | |
| 753 | 1 | 2-CF3,3-Cl-Ph | H | |
| 754 | 1 | 3-CF3,4-Cl-Ph | H | |
| 755 | 1 | 3-Cl,4-$CF_3$-Ph | H | |
| 756 | 1 | 3-CF3,4-$CF_3$-Ph | H | |
| 757 | 1 | α-Naph | H | |
| 758 | 1 | β-Naph | H | |
| 759 | 1 | 4- $Me_3Si$-Ph | H | |

Table 23

| Compound No. | n | Ar | X | m.p. (°C) |
|---|---|---|---|---|
| 760 | 0 | Ph | 5-Cl | |
| 761 | 0 | 2-$CH_3$-Ph | 5-Cl | |
| 762 | 0 | 3-$CH_3$-Ph | 5-Cl | |
| 763 | 0 | 4-$CH_3$-Ph | 5-Cl | |
| 764 | 0 | 2-F-Ph | 5-Cl | |
| 765 | 0 | 3-F-Ph | 5-Cl | |
| 766 | 0 | 4-F-Ph | 5-Cl | |
| 767 | 0 | 2-Cl-Ph | 5-Cl | |
| 768 | 0 | 3-Cl-Ph | 5-Cl | |
| 769 | 0 | 4-Cl-Ph | 5-Cl | |
| 770 | 0 | 2-Br-Ph | 5-Cl | |
| 771 | 0 | 3-Br-Ph | 5-Cl | |
| 772 | 0 | 4-Br-Ph | 5-Cl | |
| 773 | 0 | 2-$CF_3$-Ph | 5-Cl | |
| 774 | 0 | 3-$CF_3$-Ph | 5-Cl | |
| 775 | 0 | 4-$CF_3$-Ph | 5-Cl | |
| 776 | 0 | 2-$OCH_3$-Ph | 5-Cl | |
| 777 | 0 | 3-$OCH_3$-Ph | 5-Cl | |
| 778 | 0 | 4-$OCH_3$-Ph | 5-Cl | |
| 779 | 0 | 4-tBu-Ph | 5-Cl | |
| 780 | 0 | 2-OPh-Ph | 5-Cl | |
| 781 | 0 | 3-OPh-Ph | 5-Cl | |
| 782 | 0 | 4-OPh-Ph | 5-Cl | |

Table 23 (continued)

| Compound No. | n | Ar | X | m.p. (°C) |
|---|---|---|---|---|
| 783 | 0 | 2,4-$Cl_2$-Ph | 5-Cl | |
| 784 | 0 | 3,4-$Cl_2$-Ph | 5-Cl | |
| 785 | 0 | 2-Cl,4-$CF_3$-Ph | 5-Cl | |
| 786 | 0 | 2-CF3,3-Cl-Ph | 5-Cl | |
| 787 | 0 | 3-CF3,4-Cl-Ph | 5-Cl | |
| 788 | 0 | 3-Cl,4-$CF_3$-Ph | 5-Cl | |
| 789 | 0 | 3-CF3,4-$CF_3$-Ph | 5-Cl | |
| 790 | 0 | α-Naph | 5-Cl | |
| 791 | 0 | β-Naph | 5-Cl | |
| 792 | 0 | 4- $Me_3Si$-Ph | 5-Cl | |

Table 24

| Compound No. | n | Ar | X | m.p. (°C) |
|---|---|---|---|---|
| 793 | 1 | Ph | 5-Cl | |
| 794 | 1 | 2-$CH_3$-Ph | 5-Cl | |
| 795 | 1 | 3-$CH_3$-Ph | 5-Cl | |
| 796 | 1 | 4-$CH_3$-Ph | 5-Cl | |
| 797 | 1 | 2-F-Ph | 5-Cl | |
| 798 | 1 | 3-F-Ph | 5-Cl | |
| 799 | 1 | 4-F-Ph | 5-Cl | |
| 800 | 1 | 2-Cl-Ph | 5-Cl | |
| 801 | 1 | 3-Cl-Ph | 5-Cl | |
| 802 | 1 | 4-Cl-Ph | 5-Cl | |
| 803 | 1 | 2-Br-Ph | 5-Cl | |
| 804 | 1 | 3-Br-Ph | 5-Cl | |
| 805 | 1 | 4-Br-Ph | 5-Cl | |
| 806 | 1 | 2-$CF_3$-Ph | 5-Cl | |
| 807 | 1 | 3-$CF_3$-Ph | 5-Cl | |
| 808 | 1 | 4-$CF_3$-Ph | 5-Cl | |
| 809 | 1 | 2-$OCH_3$-Ph | 5-Cl | |
| 810 | 1 | 3-$OCH_3$-Ph | 5-Cl | |
| 811 | 1 | 4-$OCH_3$-Ph | 5-Cl | |
| 812 | 1 | 4-tBu-Ph | 5-Cl | |
| 813 | 1 | 2-OPh-Ph | 5-Cl | |
| 814 | 1 | 3-OPh-Ph | 5-Cl | |
| 815 | 1 | 4-OPh-Ph | 5-Cl | |
| 816 | 1 | 2,4-$Cl_2$-Ph | 5-Cl | |
| 817 | 1 | 3,4-$Cl_2$-Ph | 5-Cl | |
| 818 | 1 | 2-Cl,4-$CF_3$-Ph | 5-Cl | |
| 819 | 1 | 2-CF3,3-Cl-Ph | 5-Cl | |
| 820 | 1 | 3-CF3,4-Cl-Ph | 5-Cl | |
| 821 | 1 | 3-C1,4-$CF_3$-Ph | 5-Cl | |
| 822 | 1 | 3-CF3,4-$CF_3$-Ph | 5-Cl | |
| 823 | 1 | α-Naph | 5-Cl | |
| 824 | 1 | β-Naph | 5-Cl | |
| 825 | 1 | 4-$Me_3Si$-Ph | 5-Cl | |

Table 25

| Compound No. | n | Ar | X | m.p. (°C) |
|---|---|---|---|---|
| 826 | 0 | Ph | 7-Cl | |
| 827 | 0 | 2-$CH_3$-Ph | 7-Cl | |
| 828 | 0 | 3-$CH_3$-Ph | 7-Cl | |
| 829 | 0 | 4-$CH_3$-Ph | 7-Cl | |
| 830 | 0 | 2-F-Ph | 7-Cl | |
| 831 | 0 | 3-F-Ph | 7-Cl | |
| 832 | 0 | 4-F-Ph | 7-Cl | |
| 833 | 0 | 2-Cl-Ph | 7-Cl | |
| 834 | 0 | 3-Cl-Ph | 7-Cl | |
| 835 | 0 | 4-Cl-Ph | 7-Cl | |
| 836 | 0 | 2-Br-Ph | 7-Cl | |
| 837 | 0 | 3-Br-Ph | 7-Cl | |
| 838 | 0 | 4-Br-Ph | 7-Cl | |
| 839 | 0 | 2-$CF_3$-Ph | 7-Cl | |
| 840 | 0 | 3-$CF_3$-Ph | 7-Cl | |
| 841 | 0 | 4-$CF_3$-Ph | 7-Cl | |
| 842 | 0 | 2-$OCH_3$-Ph | 7-Cl | |
| 843 | 0 | 3-$OCH_3$-Ph | 7-Cl | |
| 844 | 0 | 4-$OCH_3$-Ph | 7-Cl | |
| 845 | 0 | 4-tBu-Ph | 7-Cl | |
| 846 | 0 | 2-OPh-Ph | 7-Cl | |
| 847 | 0 | 3-OPh-Ph | 7-Cl | |
| 848 | 0 | 4-OPh-Ph | 7-Cl | |
| 849 | 0 | 2,4-$Cl_2$-Ph | 7-Cl | |
| 850 | 0 | 3,4-$Cl_2$-Ph | 7-Cl | |
| 851 | 0 | 2-Cl,4-$CF_3$-Ph | 7-Cl | |
| 852 | 0 | 2-CF3,3-Cl-Ph | 7-Cl | |
| 853 | 0 | 3-CF3,4-Cl-Ph | 7-Cl | |
| 854 | 0 | 3-Cl,4-$CF_3$-Ph | 7-Cl | |
| 855 | 0 | 3-CF3,4-$CF_3$-Ph | 7-Cl | |
| 856 | 0 | α-Naph | 7-Cl | |
| 857 | 0 | β-Naph | 7-Cl | |
| 858 | 0 | 4- $Me_3$Si-Ph | 7-Cl | |

Table 26

| Compound No. | n | Ar | X | m.p. (°C) |
|---|---|---|---|---|
| 859 | 1 | Ph | 7-Cl | |
| 860 | 1 | 2-$CH_3$-Ph | 7-Cl | |
| 861 | 1 | 3-$CH_3$-Ph | 7-Cl | |
| 862 | 1 | 4-$CH_3$-Ph | 7-Cl | |
| 863 | 1 | 2-F-Ph | 7-Cl | |
| 864 | 1 | 3-F-Ph | 7-Cl | |
| 865 | 1 | 4-F-Ph | 7-Cl | |
| 866 | 1 | 2-Cl-Ph | 7-Cl | |
| 867 | 1 | 3-Cl-Ph | 7-Cl | |
| 868 | 1 | 4-Cl-Ph | 7-Cl | |
| 869 | 1 | 2-Br-Ph | 7-Cl | |

Table 26 (continued)

| Compound No. | n | Ar | X | m.p. (°C) |
|---|---|---|---|---|
| 870 | 1 | 3-Br-Ph | 7-Cl | |
| 871 | 1 | 4-Br-Ph | 7-Cl | |
| 872 | 1 | 2-$CF_3$-Ph | 7-Cl | |
| 873 | 1 | 3-$CF_3$-Ph | 7-Cl | |
| 874 | 1 | 4-$CF_3$-Ph | 7-Cl | |
| 875 | 1 | 2-$OCH_3$-Ph | 7-Cl | |
| 876 | 1 | 3-$OCH_3$-Ph | 7-Cl | |
| 877 | 1 | 4-$OCH_3$-Ph | 7-Cl | |
| 878 | 1 | 4-tBu-Ph | 7-Cl | |
| 879 | 1 | 2-OPh-Ph | 7-Cl | |
| 880 | 1 | 3-OPh-Ph | 7-Cl | |
| 881 | 1 | 4-OPh-Ph | 7-Cl | |
| 882 | 1 | 2,4-$Cl_2$-Ph | 7-Cl | |
| 883 | 1 | 3,4-$Cl_2$-Ph | 7-Cl | |
| 884 | 1 | 2-Cl,4-$CF_3$-Ph | 7-Cl | |
| 885 | 1 | 2-CF3,3-Cl-Ph | 7-Cl | |
| 886 | 1 | 3-CF3,4-Cl-Ph | 7-Cl | |
| 887 | 1 | 3-Cl,4-$CF_3$-Ph | 7-Cl | |
| 888 | 1 | 3-CF3,4-$CF_3$-Ph | 7-Cl | |
| 889 | 1 | $\alpha$-Naph | 7-Cl | |
| 890 | 1 | $\beta$-Naph | 7-Cl | |
| 891 | 1 | 4- $Me_3Si$-Ph | 7-Cl | |

Table 27

| Compound No. | n | Ar | X | m.p. (°C) |
|---|---|---|---|---|
| 892 | 0 | Ph | 2-$CH_3$ | |
| 893 | 0 | 2-$CH_3$-Ph | 2-$CH_3$ | |
| 894 | 0 | 3-$CH_3$-Ph | 2-$CH_3$ | |
| 895 | 0 | 4-$CH_3$-Ph | 2-$CH_3$ | |
| 896 | 0 | 2-F-Ph | 2-$CH_3$ | |
| 897 | 0 | 3-F-Ph | 2-$CH_3$ | |
| 898 | 0 | 4-F-Ph | 2-$CH_3$ | |
| 899 | 0 | 2-Cl-Ph | 2-$CH_3$ | |
| 900 | 0 | 3-Cl-Ph | 2-$CH_3$ | |
| 901 | 0 | 4-Cl-Ph | 2-$CH_3$ | |
| 902 | 0 | 2-Br-Ph | 2-$CH_3$ | |
| 903 | 0 | 3-Br-Ph | 2-$CH_3$ | |
| 904 | 0 | 4-Br-Ph | 2-$CH_3$ | |
| 905 | 0 | 2-$CF_3$-Ph | 2-$CH_3$ | |
| 906 | 0 | 3-$CF_3$-Ph | 2-$CH_3$ | |
| 907 | 0 | 4-$CF_3$-Ph | 2-$CH_3$ | |
| 908 | 0 | 2-$OCH_3$-Ph | 2-$CH_3$ | |
| 909 | 0 | 3-$OCH_3$-Ph | 2-$CH_3$ | |
| 910 | 0 | 4-$OCH_3$-Ph | 2-$CH_3$ | |
| 911 | 0 | 4-tBu-Ph | 2-$CH_3$ | |
| 912 | 0 | 2-OPh-Ph | 2-$CH_3$ | |
| 913 | 0 | 3-OPh-Ph | 2-$CH_3$ | |
| 914 | 0 | 4-OPh-Ph | 2-$CH_3$ | |

Table 27 (continued)

| Compound No. | n | Ar | X | m.p. (°C) |
|---|---|---|---|---|
| 915 | 0 | 2,4-$Cl_2$-Ph | 2-$CH_3$ | |
| 916 | 0 | 3,4-$Cl_2$-Ph | 2-$CH_3$ | |
| 917 | 0 | 2-Cl,4-$CF_3$-Ph | 2-$CH_3$ | |
| 918 | 0 | 2-CF3,3-Cl-Ph | 2-$CH_3$ | |
| 919 | 0 | 3-CF3,4-Cl-Ph | 2-$CH_3$ | |
| 920 | 0 | 3-Cl,4-$CF_3$-Ph | 2-$CH_3$ | |
| 921 | 0 | 3-CF3,4-$CF_3$-Ph | 2-$CH_3$ | |
| 922 | 0 | α-Naph | 2-$CH_3$ | |
| 923 | 0 | β-Naph | 2-$CH_3$ | |
| 924 | 0 | 4- $Me_3Si$-Ph | 2-$CH_3$ | |

Table 28

| Compound No. | n | Ar | X | m.p. (°C) |
|---|---|---|---|---|
| 925 | 2 | Ph | 2-$CH_3$ | |
| 926 | 2 | 2-$CH_3$-Ph | 2-$CH_3$ | |
| 927 | 2 | 3-$CH_3$-Ph | 2-$CH_3$ | |
| 928 | 2 | 4-$CH_3$-Ph | 2-$CH_3$ | |
| 929 | 2 | 2-F-Ph | 2-$CH_3$ | |
| 930 | 2 | 3-F-Ph | 2-$CH_3$ | |
| 931 | 2 | 4-F-Ph | 2-$CH_3$ | |
| 932 | 2 | 2-Cl-Ph | 2-$CH_3$ | |
| 933 | 2 | 3-Cl-Ph | 2-$CH_3$ | |
| 934 | 2 | 4-Cl-Ph | 2-$CH_3$ | |
| 935 | 2 | 2-Br-Ph | 2-$CH_3$ | |
| 936 | 2 | 3-Br-Ph | 2-$CH_3$ | |
| 937 | 2 | 4-Br-Ph | 2-$CH_3$ | |
| 938 | 2 | 2-$CF_3$-Ph | 2-$CH_3$ | |
| 939 | 2 | 3-$CF_3$-PH | 2-$CH_3$ | |
| 940 | 2 | 4-$CF_3$-Ph | 2-$CH_3$ | |
| 941 | 2 | 2-$OCH_3$-Ph | 2-$CH_3$ | |
| 942 | 2 | 3-$OCH_3$-Ph | 2-$CH_3$ | |
| 943 | 2 | 4-$OCH_3$-Ph | 2-$CH_3$ | |
| 944 | 2 | 4-tBu-Ph | 2-$CH_3$ | |
| 945 | 2 | 2-OPh-Ph | 2-$CH_3$ | |
| 946 | 2 | 3-OPh-Ph | 2-$CH_3$ | |
| 947 | 2 | 4-OPh-Ph | 2-$CH_3$ | |
| 948 | 2 | 2,4-$Cl_2$-Ph | 2-$CH_3$ | |
| 949 | 2 | 3,4-$Cl_2$-Ph | 2-$CH_3$ | |
| 950 | 2 | 2-Cl,4-$CF_3$-Ph | 2-$CH_3$ | |
| 951 | 2 | 2-CF3,3-Cl-Ph | 2-$CH_3$ | |
| 952 | 2 | 3-CF3,4-Cl-Ph | 2-$CH_3$ | |
| 953 | 2 | 3-Cl,4-$CF_3$-Ph | 2-$CH_3$ | |
| 954 | 2 | 3-CF3,4-$CF_3$-Ph | 2-$CH_3$ | |
| 955 | 2 | α-Naph | 2-$CH_3$ | |
| 956 | 2 | β-Naph | 2-$CH_3$ | |
| 957 | 2 | 4- $Me_3Si$-Ph | 2-$CH_3$ | |

Table 29

| Compound No. | n | Ar | X | m.p. (°C) |
|---|---|---|---|---|
| 958 | 0 | Ph | 5-$SO_3H$ | |
| 959 | 0 | 2-$CH_3$-Ph | 5-$SO_3H$ | |
| 960 | 0 | 3-$CH_3$-Ph | 5-$SO_3H$ | |
| 961 | 0 | 4-$CH_3$-Ph | 5-$SO_3H$ | |
| 962 | 0 | 2-F-Ph | 5-$SO_3H$ | |
| 963 | 0 | 3-F-Ph | 5-$SO_3H$ | |
| 964 | 0 | 4-F-Ph | 5-$SO_3H$ | |
| 965 | 0 | 2-Cl-Ph | 5-$SO_3H$ | |
| 966 | 0 | 3-Cl-Ph | 5-$SO_3H$ | |
| 967 | 0 | 4-Cl-Ph | 5-$SO_3H$ | |
| 968 | 0 | 2-Br-Ph | 5-$SO_3H$ | |
| 969 | 0 | 3-Br-Ph | 5-$SO_3H$ | |
| 970 | 0 | 4-Br-Ph | 5-$SO_3H$ | |
| 971 | 0 | 2-$CF_3$-Ph | 5-$SO_3H$ | |
| 972 | 0 | 3-$CF_3$-Ph | 5-$SO_3H$ | |
| 973 | 0 | 4-$CF_3$-Ph | 5-$SO_3H$ | |
| 974 | 0 | 2-$OCH_3$-Ph | 5-$SO_3H$ | |
| 975 | 0 | 3-$OCH_3$-Ph | 5-$SO_3H$ | |
| 976 | 0 | 4-$OCH_3$-Ph | 5-$SO_3H$ | |
| 977 | 0 | 4-tBu-Ph | 5-$SO_3H$ | |
| 978 | 0 | 2-OPh-Ph | 5-$SO_3H$ | |
| 979 | 0 | 3-OPh-Ph | 5-$SO_3H$ | |
| 980 | 0 | 4-OPh-Ph | 5-$SO_3H$ | |
| 981 | 0 | 2,4-$Cl_2$-Ph | -5-$SO_3H$ | |
| 982 | 0 | 3,4-$Cl_2$-Ph | 5-$SO_3H$ | |
| 983 | 0 | 2-Cl,4-$CF_3$-Ph | 5-$SO_3H$ | |
| 984 | 0 | 2-CF3,3-Cl-Ph | 5-$SO_3H$ | |
| 985 | 0 | 3-CF3,4-Cl-Ph | 5-$SO_3H$ | |
| 986 | 0 | 3-Cl,4-$CF_3$-Ph | 5-$SO_3H$ | |
| 987 | 0 | 3-CF3,4-$CF_3$-Ph | 5-$SO_3H$ | |
| 988 | 0 | $\alpha$-Naph | 5-$SO_3H$ | |
| 989 | 0 | $\beta$-Naph | 5-$SO_3H$ | |
| 990 | 0 | 4-$Me_3Si$-Ph | 5-$SO_3H$ | |

Table 30

| Compound No. | n | Ar | X | *m.p.* (°C) |
|---|---|---|---|---|
| 991 | 0 | Ph | 5-$SO_3H$ | |
| 992 | 0 | 2-$CH_3$-Ph | 5-$SO_3H$ | |
| 993 | 0 | 3-$CH_3$-Ph | 5-$SO_3H$ | |
| 994 | 0 | 4-$CH_3$-Ph | 5-$SO_3H$ | |
| 995 | 0 | 2-F-Ph | 5-$SO_3H$ | |
| 996 | 0 | 3-F-Ph | 5-$SO_3H$ | |
| 997 | 0 | 4-F-Ph | 5-$SO_3H$ | |
| 998 | 0 | 2-Cl-Ph | 5-$SO_3H$ | |
| 999 | 0 | 3-Cl-Ph | 5-$SO_3H$ | |
| 1000 | 0 | 4-Cl-Ph | 5-$SO_3H$ | |
| 1001 | 0 | 2-Br-Ph | 5-$SO_3H$ | |

Table 30 (continued)

| Compound No. | n | Ar | X | *m.p.* (°C) |
|---|---|---|---|---|
| 1002 | 0 | 3-Br-Ph | 5-$SO_3H$ | |
| 1003 | 0 | 4-Br-Ph | 5-$SO_3H$ | |
| 1004 | 0 | 2-$CF_3$-Ph | 5-$SO_3H$ | |
| 1005 | 0 | 3-$CF_3$-Ph | 5-$SO_3H$ | |
| 1006 | 0 | 4-$CF_3$-Ph | 5-$SO_3H$ | |
| 1007 | 0 | 2-$OCH_3$-Ph | 5-$SO_3H$ | |
| 1008 | 0 | 3-$OCH_3$-Ph | 5-$SO_3H$ | |
| 1009 | 0 | 4-$OCH_3$-Ph | 5-$SO_3H$ | |
| 1010 | 0 | 4-tBu-Ph | 5-$SO_3H$ | |
| 1011 | 0 | 2-OPh-Ph | 5-$SO_3H$ | |
| 1012 | 0 | 3-OPh-Ph | 5-$SO_3H$ | |
| 1013 | 0 | 4-OPh-Ph | 5-$SO_3H$ | |
| 1014 | 0 | 2,4-$Cl_2$-Ph | 5-$SO_3H$ | |
| 1015 | 0 | 3,4-$Cl_2$-Ph | 5-$SO_3H$ | |
| 1016 | 0 | 2-Cl,4-$CF_3$-Ph | 5-$SO_3H$ | |
| 1017 | 0 | 2-CF3,3-Cl-Ph | 5-$SO_3H$ | |
| 1018 | 0 | 3-CF3,4-Cl-Ph | 5-$SO_3H$ | |
| 1019 | 0 | 3-Cl,4-$CF_3$-Ph | 5-$SO_3H$ | |
| 1020 | 0 | 3-CF3,4-$CF_3$-Ph | 5-$SO_3H$ | |
| 1021 | 0 | α-Naph | 5-$SO_3H$ | |
| 1022 | 0 | β-Naph | 5-$SO_3H$ | |
| 1023 | 0 | 4-$Me_3Si$-Ph | 5-$SO_3H$ | |

Table 31

| Compound No. | n | Ar | X | m.p. (°C) |
|---|---|---|---|---|
| 1024 | 0 | Ph | 5,7-$Cl_2$ | |
| 1025 | 0 | 2-$CH_3$-Ph | 5,7-$Cl_2$ | |
| 1026 | 0 | 3-$CH_3$-Ph | 5,7-$Cl_2$ | |
| 1027 | 0 | 4-$CH_3$-Ph | 5,7-$Cl_2$ | |
| 1028 | 0 | 2-F-Ph | 5,7-$Cl_2$ | |
| 1029 | 0 | 3-F-Ph | 5,7-$Cl_2$ | |
| 1030 | 0 | 4-F-Ph | 5,7-$Cl_2$ | |
| 1031 | 0 | 2-Cl-Ph | 5,7-$Cl_2$ | |
| 1032 | 0 | 3-Cl-Ph | 5,7-$Cl_2$ | |
| 1033 | 0 | 4-Cl-Ph | 5,7-$Cl_2$ | |
| 1034 | 0 | 2-Br-Ph | 5,7-$Cl_2$ | |
| 1035 | 0 | 3-Br-Ph | 5,7-$Cl_2$ | |
| 1036 | 0 | 4-Br-Ph | 5,7-$Cl_2$ | |
| 1037 | 0 | 2-$CF_3$-Ph | 5,7-$Cl_2$ | |
| 1038 | 0 | 3-$CF_3$-Ph | 5,7-$Cl_2$ | |
| 1039 | 0 | 4-$CF_3$-Ph | 5,7-$Cl_2$ | |
| 1040 | 0 | 2-$OCH_3$-Ph | 5,7-$Cl_2$ | |
| 1041 | 0 | 3-$OCH_3$-Ph | 5,7-$Cl_2$ | |
| 1042 | 0 | 4-$OCH_3$-Ph | 5,7-$Cl_2$ | |
| 1043 | 0 | 4-tBu-Ph | 5,7-$Cl_2$ | |
| 1044 | 0 | 2-OPh-Ph | 5,7-$Cl_2$ | |
| 1045 | 0 | 3-OPh-Ph | 5,7-$Cl_2$ | |
| 1046 | 0 | 4-OPh-Ph | 5,7-$Cl_2$ | |

Table 31 (continued)

| Compound No. | n | Ar | X | m.p. (°C) |
|---|---|---|---|---|
| 1047 | 0 | 2,4-$Cl_2$-Ph | 5,7-$Cl_2$ | |
| 1048 | 0 | 3,4-$Cl_2$-Ph | 5,7-$Cl_2$ | |
| 1049 | 0 | 2-Cl,4-$CF_3$-Ph | 5,7-$Cl_2$ | |
| 1050 | 0 | 2-CF3,3-Cl-Ph | 5,7-$Cl_2$ | |
| 1051 | 0 | 3-CF3,4-Cl-Ph | 5,7-$Cl_2$ | |
| 1052 | 0 | 3-Cl,4-$CF_3$-Ph | 5,7-$Cl_2$ | |
| 1053 | 0 | 3-CF3,4-$CF_3$-Ph | 5,7-$Cl_2$ | |
| 1054 | 0 | α-Naph | 5,7-$Cl_2$ | |
| 1055 | 0 | β-Naph | 5,7-$Cl_2$ | |
| 1056 | 0 | 4-$Me_3$Si-Ph | 5,7-$Cl_2$ | |

Table 32

| Compound No. | n | Ar | X | m.p. (°C) |
|---|---|---|---|---|
| 1057 | 1 | Ph | 5,7-$Cl_2$ | |
| 1058 | 1 | 2-$CH_3$-Ph | 5,7-$Cl_2$ | |
| 1059 | 1 | 3-$CH_3$-Ph | 5,7-$Cl_2$ | |
| 1060 | 1 | 4-$CH_3$-Ph | 5,7-$Cl_2$ | |
| 1061 | 1 | 2-F-Ph | 5,7-$Cl_2$ | |
| 1062 | 1 | 3-F-Ph | 5,7-$Cl_2$ | |
| 1063 | 1 | 4-F-Ph | 5,7-$Cl_2$ | |
| 1064 | 1 | 2-Cl-Ph | 5,7-$Cl_2$ | |
| 1065 | 1 | 3-Cl-Ph | 5,7-$Cl_2$ | |
| 1066 | 1 | 4-Cl-Ph | 5,7-$Cl_2$ | |
| 1067 | 1 | 2-Br-Ph | 5,7-$Cl_2$ | |
| 1068 | 1 | 3-Br-Ph | 5,7-$Cl_2$ | |
| 1069 | 1 | 4-Br-Ph | 5,7-$Cl_2$ | |
| 1070 | 1 | 2-$CF_3$-Ph | 5,7-$Cl_2$ | |
| 1071 | 1 | 3-$CF_3$-Ph | 5,7-$Cl_2$ | |
| 1072 | 1 | 4-$CF_3$-Ph | 5,7-$Cl_2$ | |
| 1073 | 1 | 2-$OCH_3$-Ph | 5,7-$Cl_2$ | |
| 1074 | 1 | 3-$OCH_3$-Ph | 5,7-$Cl_2$ | |
| 1075 | 1 | 4-$OCH_3$-Ph | 5,7-$Cl_2$ | |
| 1076 | 1 | 4-tBu-Ph | 5,7-$Cl_2$ | |
| 1077 | 1 | 2-OPh-Ph | 5,7-$Cl_2$ | |
| 1078 | 1 | 3-OPh-Ph | 5,7-$Cl_2$ | |
| 1079 | 1 | 4-OPh-Ph | 5,7-$Cl_2$ | |
| 1080 | 1 | 2,4-$Cl_2$-Ph | 5,7-$Cl_2$ | |
| 1081 | 1 | 3,4-$Cl_2$-Ph | 5,7-$Cl_2$ | |
| 1082 | 1 | 2-Cl,4-$CF_3$-Ph | 5,7-$Cl_2$ | |
| 1083 | 1 | 2-CF3,3-Cl-Ph | 5,7-$Cl_2$ | |
| 1084 | 1 | 3-CF3,4-Cl-Ph | 5,7-$Cl_2$ | |
| 1085 | 1 | 3-Cl,4-$CF_3$-Ph | 5,7-$Cl_2$ | |
| 1086 | 1 | 3-CF3,4-$CF_3$-Ph | 5,7-$Cl_2$ | |
| 1087 | 1 | α-Naph | 5,7-$Cl_2$ | |
| 1088 | 1 | β-Naph | 5,7-$Cl_2$ | |
| 1089 | 1 | 4-$Me_3$Si-Ph | 5,7-$Cl_2$ | |

The compounds of the invention can be formulated into a composition for preventing or treating coccidiosis

in poultry according to methods well known to the art. Specifically, the compounds of the invention can be formulated into the form of powder, granules, solution, suspension, premix, capsule, emulsion, tablets, or the like, alone or together with any appropriate carrier commonly used for this kind of medicament, optionally using excipients, disintegrating agents, lubricants, coatings, etc.

Usually, the carriers used in the formulations of the invention are not limited so long as they are capable of being used as an additive in feed or drinking water for poultry. Examples of such carriers include water, lactose, sucrose, talc, colloidal silica, pectin, wheat flour, rice bran, cornmeal, soy beans, oilcake, cracked starch and other commercially available feed for poultry, and the like.

As to dose of the compound of the invention, in the case of use as an additive in feed, the bulk product may be generally mixed with the feed at a concentration of at least 0.1 to 500 ppm, preferably 0.5 to 100 ppm. In the case of use as an additive in drinking water, an approximate half concentration of the above can provide sufficient effectiveness.

Also, the compounds of the invention may be used associated with known veterinary medicaments including anticoccidial agents for poultry, anthelmintics, agents for preventing infections, growth promoting agents, or the like.

Test Examples

Anticoccidial activity of the compounds of the invention was demonstrated in the following test examples. *In vitro* test of the anticoccidial agent for poultry of the invention was conducted as shown below.

Test Example 1

*In vitro* inhibitory activity of coccidial proliferation in chicken kidney (CK) cell culture

1. Cell treatments

Kidney was aseptically removed from an SPF chick, and digested with trypsin in a publicly known method. The cells were washed, and the cell number was adjusted, before the cells were incubated at 37°C for 72 hours in a $CO_2$ incubator to give a cell monolayer. The cell monolayer was inoculated with a dilution of a defined concentration of test compounds and sporozoites of the *Eimeria tenella* oocysts that had been artificially excysted, and incubated at 40° C for 48 hours. The cells were fixed with alcohol, Giemsa-stained, and then observed as shown below.

2. Observation and estimation

The primary schizonts (mature and immature) divided and proliferated in the cytoplasm of the chicken kidney cells were observed under an inverted microscope, and the inhibitory rate of the protozoan proliferation was estimated on the basis of the schizont count relative to that of the control group wherein sporozoites that were not treated were inoculated.

At the same time, cytopathogenic effect (CPE) was additionally observed, and estimated on a scale of one to four (- to +++): -: no difference compared to the control group, +: slight change, ++: moderate change, and +++: death of whole cells (the protozoan was invisible).

The results are shown in Table 33 as described below.

Test Example 2

*In vivo* estimation of prevention/treatment effect on coccidiosis in chicken chicks

The compounds of the invention were sequentially administered in the form of feed (200 ppm or 400 ppm) so as to estimate for their effectiveness (anticoccidial effect) on *Eimeria tenella.*

One group consisted of three white Leghorn chicks aged seven to ten days, each of which chicks was administered with a feed supplemented with a test compound. On the second day after the administration, spore forming oocysts of *Eimeria tenella* were infected at 50,000 oocysts per chick. The test compounds were administered over a period of consecutive nine days, during which the hemorrhagic stool excretion, the survival rate, and the relative weight gain ratio were estimated, and, on the eighth day after the infection, the cecum lesion were observed at autopsy to estimate the lesion index.

The criteria were as shown below.

Relative weight gain ratio:

is represented by the following expression:

Weight gain in test group ÷ Weight gain in control group of uninfected chicks x 100 (%).

Hemorrhagic stool excretion:

Excretion of the hemorrhagic stool excreted during the test period was estimated on a scale of one to four as following. In the table, "d" represents the number of days after the administration of oocysts.

- : no hemorrhagic stool excretion was observed;
+ : a slight hemorrhagic stool excretion was observed;
++ : a moderate hemorrhagic stool excretion was observed; and
+++ : a hemorrhagic stool excretion equal to that in the control group of the infected chicks was observed.

Cecum lesion index:

is in accordance with Merck assay.
Eight days after the infection, survived chicks were autopsied, and the cecum lesion was visually observed to determine the intensity caused during the period infection on a scale of zero to four (no lesion; 0, severe lesion; 4), thereby estimating the lesion severity at the average of five results.
The test results are shown in Table 33 with reference to those in the control group of the untreated, infected chicks, and in the control group of untreated, uninfected chicks.

Table 33

| ***in vitro* antiprotozoal activity *(E. tenella)*** | | | | ***in vivo* anticoccidial effect *(E.tenella)*** | | | | | | | |
|---|---|---|---|---|---|---|---|---|---|---|---|
| Comp. No | concentration (μg/ml) | | | dose (ppm in feed) | relative weight gain ratio (%) | hemorrhagic stool excretion | | | mortality (/) | OPG value | cecum lesion index (6d) |
| | 10 | 1.0 | 0.1 | | | 4 | 5 | 6d | | | |
| | % (CPE) | % (CPE) | % (CPE) | | | | | | | | |
| 1 | <0(-) | 0.2 (-) | <0 (-) | | | | | | | | |
| 694 | (+++) | 12.5 (-) | <0 (-) | | | | | | | | |
| 199 | 39.6 (-) | <0 (-) | <0 (-) | | | | | | | | |
| 232 | 32.2 (-) | 0.9(-) | 6.0(-) | | | | | | | | |
| 31 | 69.7 (-) | 0 (-) | <0(-) | 400 | 66.8 | ± | +++ | ++ | 0/3 | None | 4.0 |
| 724 | (+++) | 100 (-) | <0 (-) | 400 | 58.9 | ± | +++ | ++ | 0/3 | " | 4.0 |
| 229 | 77.0 (-) | <0(-) | <0(-) | 400 | 56.5 | + | +++ | ++ | 0/3 | " | 4.0 |
| 262 | 60.4 (-) | 0.9(-) | <0(-) | 400 | 53.8 | ± | +++ | +++ | 0/3 | " | 4.0 |
| 2 | <0(-) | <0(-) | <0 (-) | | | | | | | | |
| 695 | (+++) | (+++) | <0 (-) | 400 | 61.5 | + | +++ | +++ | 0/3 | " | 3.7 |
| 200 | <0 (-) | <0(-) | <0 (-) | | | | | | | | |

Table 33 (continued)

| ***in vitro* antiprotozoal activity *(E. tenella)*** | | | | ***in vivo* anticoccidial effect *(E.tenella)*** | | | | | | | |
|---|---|---|---|---|---|---|---|---|---|---|---|
| Comp. No | concentration (μg/ml) | | | dose (ppm in feed) | relative weight gain ratio (%) | hemorrhagic stool excretion | | | mortality (/) | OPG value | cecum lesion index (6d) |
| | 10 | 1.0 | 0.1 | | | 4 | 5 | 6d | | | |
| | % (CPE) | % (CPE) | % (CPE) | | | | | | | | |
| 233 | <0 (-) | <0(-) | <0 (-) | | | | | | | | |
| 3 | 19.3 (-) | <0(-) | <0 (-) | | | | | | | | |
| 696 | (+++) | 90.7 (+) | 30.5 (-) | 400 | 61.5 | + | +++ | ++ | 1/3 | " | 4.0 |
| 201 | 71.3 (-) | <0(-) | <0(-) | 400 | 71.8 | + | +++ | ++ | 1/3 | " | 4.0 |
| 4 | <0(-) | <0(-) | <0(-) | | | | | | | | |
| 697 | (+++) | 65.1 (±) | <0(-) | 400 | 76.9 | ± | +++ | +++ | 1/3 | " | 3.7 |
| 202 | 44.2 (-) | <0(-) | <0 (-) | | | | | | | | |
| 710 | (+++) | <0(-) | <0(-) | | | | | | | | |
| 215 | 4.5(-) | <0(-) | 1.8(-) | | | | | | | | |
| 19 | <0(-) | <0(-) | <0(-) | | | | | | | | |
| 712 | (+++) | 28.8 (-) | <0(-) | | | | | | | | |
| 217 | 14.5 (-) | <0(-) | <0(-) | | | | | | | | |
| 250 | <0 (-) | 2.4(-) | <0 (-) | | | | | | | | |
| 703 | (+++) | <0(-) | <0 (-) | | | | | | | | |
| 208 | 17.2 (-) | <0(-) | 1.8 (-) | | | | | | | | |
| 241 | 14.0 (-) | <0(-) | <0 (-) | | | | | | | | |
| 10 | 100 (+) | 12.1 (-) | <0 (-) | 400 | 82.5 | - | - | - | 0/3 | " | 0.0(H) |
| 15 | 99.6 (+) | <0 (-) | <0 (-) | 200 | 25.7 | + | +++ | ++ | 0/3 | " | 4.0 |
| 708 | (+++) | 94.7 (++) | 16.6 (-) | 400 | 42.4 | + | ++ | +++ | 0/3 | " | 4.0 |
| 213 | 100 (+) | 13.0 (-) | <0 (-) | 400 | 57.4 | ± | ++ | ++ | 0/3 | " | 3.7 |
| 246 | 100 (+) | 14.8 (-) | <0 (-) | 400 | 82.5 | - | + | ++ | 0/3 | " | 3.3(S) |
| 7 | <0 (-) | <0 (-) | <0 (-) | | | | | | | | |
| 700 | (+++) | 99.8 (++) | 30.6 (-) | | | | | | | | |
| 205 | 33.2 (±) | <0 (-) | <0 (-) | | | | | | | | |

Table 33 (continued)

| ***in vitro* antiprotozoal activity *(E. tenella)*** | | | | ***in vivo* anticoccidial effect *(E.tenella)*** | | | | | | | |
|---|---|---|---|---|---|---|---|---|---|---|---|
| Comp. No | concentration (μg/ml) | | | dose (ppm in feed) | relative weight gain ratio (%) | hemorrhagic stool excretion | | | mortality (/) | OPG value | cecum lesion index (6d) |
| | 10 | 1.0 | 0.1 | | | 4 | 5 | 6d | | | |
| | % (CPE) | % (CPE) | % (CPE) | | | | | | | | |
| 238 | 27.1 (±) | <0 (-) | <0 (-) | | | | | | | | |

Results:

Compounds 10 and 246, etc. explicitly provided improvements in the clinical conditions, the relative weight gain ratio, and the cecum lesion index. Especially, compound 10 caused no hemorrhagic stool excretion and no infectious sign, with any cecum not being affected.

<u>Test Example 3</u>

Additional *in vivo* test for compound 10

An additional *in vivo* test was conducted on compound 10, which was shown to provide good results in Test Examples 1 and 2.

Specifically, the effectiveness of compound 10 on *Eimeria tenella,* and *Eimeria acervulina* in broiler chicks in a similar manner to that in Test Example 2.

In this test, OPG value) that is the number of oocysts existing in one gram of the faeces, was also determined on the sixth day after the infection. In the determination of OPG value, the faeces were taken and scrambled thoroughly, and two grams of an aliquot was diluted 20 times with distilled water. A Fuchs-Rosenthal hemocytometer was used to conduct the counting, and to determine OPG value per one gram of the faeces. OPG value enables us to determine the prognostic severity of the infection according to the number of value. In general, the higher is OPG value, the severer is the disease.

The results are shown Tables 34 and 35.

Table 34

| Anticoccidial effect on *Eimeria tenella* (broiler chicks) | | | | | | | | | | | |
|---|---|---|---|---|---|---|---|---|---|---|---|
| Comp.No | indose (ppm feed) | relative weight gain ratio (-1-8d) % | hemorrhagic stool excretion | | | | mortality (/) | OPG value | | cecum lesion index (8d) | estimation (×-◎) |
| | | | 4 | 5 | 6 | 7d | | 6d | 7d | | |
| Comp. 10 | 200 | 70.2(T) | - | - | - | - | 0/3 | 0 | 0 | 0 | ◎ (T)* |
| | 100 | 101.0 | - | + | + | ± | 0/3 | $1.0\times10^{5}$ | $6.3\times10^{3}$ | 1.7* | △* |
| | 50 | 54.8 | + | +++ | ++ | + | 0/3 | $2.9\times10^{5}$ | $9.8\times10^{4}$ | 4.0 | × |
| | 25 | 65.0 | + | +++ | ++ | - | 0/3 | $1.3\times10^{5}$ | $4.3\times10^{4}$ | 4.0 | × |
| infective control | 0 | 49.0 | ++ | +++ | ++ | ± | 1/3 | $1.9\times10^{5}$ | $1.3\times10^{5}$ | 4.0 | |
| normal control | 0 | 100 | - | - | - | - | 0/3 | 0 | 0 | 0 | |

* diarrhea (+)

Table 35

| Anticoccidial effect on *Eimeria acervulina* (broiler chicks) | | | | | | | | | |
|---|---|---|---|---|---|---|---|---|---|
| Comp. No | dose (ppm in feed) | relative weight gain ratio (-1-5d) % | excretion of mucos faeces | | | mortal -ity (/) | OPG value | | estimation (×-◎) |
| | | | 3 | 4 | 5d | | 4d | 5d | |
| Comp. 10 | 200 | 82.3(T) | - | - | - | 0/3 | 0 | 0 | ◎ (T)* |
| | 100 | 80.6 | - | + | ++ | 0/3 | $6.6x10^6$ | $2.8x10^6$ | △* |
| | 50 | 67.8 | - | ++ | +++ | 0/3 | $5.9x10^6$ | $1.7x10^6$ | x |
| | 25 | 51.6 | - | ++ | +++ | 0/3 | $1.9x10^7$ | $6.2x10^6$ | x |
| infective control | 0 | 54.9 | - | ++ | +++ | 0/3 | $1.7x10^7$ | $7.5x10^6$ | |
| normal control | 0 | 100 | - | - | - | 0/3 | 0 | 0 | |

* diarrhea (+)

Results:

When administered to both *Eimeria tenella,* and *Eimeria acervulina* in the form of feed at 100ppm, compound 10 explicitly provided improvements in the clinical conditions, the relative weight gain ratio, and the cecum lesion index (only *Eimeria tenella),* demonstrating that compound 10 exhibits a excellent anticoccidial effect.

EFFECTS OF THE INVENTION

As shown in the above test examples, the compounds of the present invention exhibit strong anticoccidial activities in chicken chicks, such as inhibition of the decrease in relative weight gain ratio, inhibition of hemorrhagic stool excretion, decrease in oocysts counts (OPG), improvement of cecum lesion index, and the like. Further, the compounds of the invention have a low toxicity as demonstrated by the data of mortality. Accordingly, the compounds of the invention are useful as compositions for preventing and treating coccidiosis in poultry such as chicken, turkey, ducks, or the like as well as livestock such as cattle, pig, or the like.

**Claims**

**1.** A pharmaceutical composition useful for animals except for humans, which comprises a compound of formula (I):

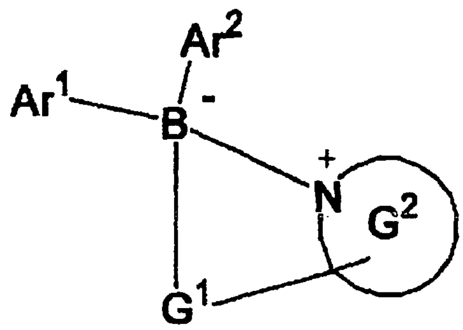

(I)

in which

$Ar^1$ and $Ar^2$ are independently a cyclic group which may be optionally substituted;
$G^1$ is a group of the formula: -A-, -A-$CR^1R^2$- or -A-$CR^3R^4$-$CR^5R^6$- wherein A is an oxygen atom or a sulfur atom, $R^1$-$R^6$ are the same or different, and are each a hydrogen atom, a halogen atom, hydroxy, an aliphatic hydrocarbon group which may be optionally substituted, a-O- (aliphatic hydrocarbon group which may be optionally substituted), an acyloxy, a phenyloxy which may be optionally substituted, an amino group which may be optionally substituted, a sulfo group which may be optionally substituted, or an aryl which may be optionally substituted, or $R^1$ and $R^2$, $R^3$ and $R^4$ and/or $R^5$ and $R^6$ are combined together to form oxo, a methylene which may be optionally substituted, or an imino which may be optionally substituted; and

a group of the formula:

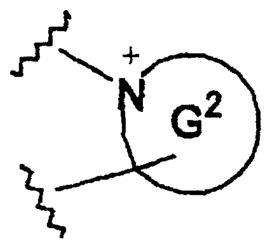

is an azacyclic group that may be optionally substituted;
provided that the smallest ring among rings constructed with $B^-$, $G^1$ and $G^2$ is a 5- or 6-membered ring; or a salt thereof or a hydrate of them.

**2.** The pharmaceutical composition of claim 1, in which $Ar^1$ and $Ar^2$ in formula (I) are the same, and are an aryl which may be optionally substituted.

**3.** The pharmaceutical composition of claim 1 or 2, in which $G^1$ in formula (I) is a group of the formula: -O-, -O-$CR^1R^2$- or -O-$CR^3R^4$-$CR^5R^6$- wherein $R^1$-$R^6$ are the same or different, and are each a hydrogen atom, a halogen atom, hydroxy, a lower alkyl which may be optionally substituted, a lower alkenyl which may be optionally substituted, a lower alkyloxy which may be optionally substituted, or a lower alkenyloxy which may be optionally substituted.

**4.** A pharmaceutical composition for use as an antiprotozoal agent, which comprises a compound of formula (I) in which all symbols are as defined in claim 1, or a salt thereof or a hydrate of them.

**5.** A pharmaceutical composition for use as an anticoccidial agent, which comprises a compound of formula (I) in which all symbols are as defined in claim 1, or a salt thereof or a hydrate of them.

**6.** A compound of formula (II):

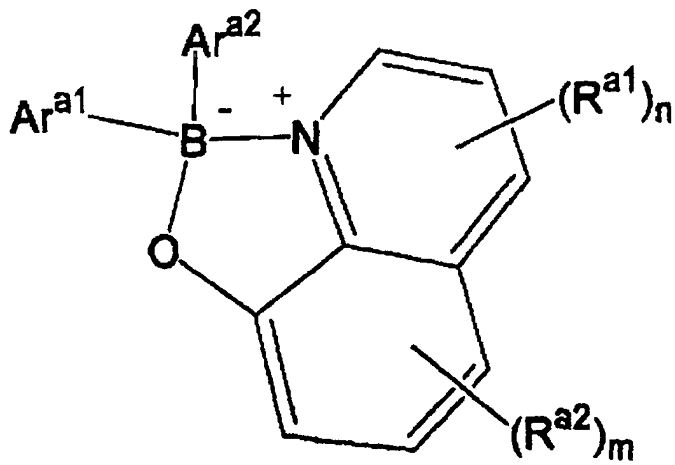

(II)

in which

$Ar^{a1}$ is an aryl which may be optionally substituted;
$Ar^{a2}$ is a fused aryl which may be optionally substituted;
$R^{a1}$ and $R^{a2}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted; and
n and m are the same or different, and are an integer of 0-3;

provided that when both n and m are 0, then a compound wherein both $Ar^{a1}$ and $Ar^{a2}$ are not naphthalen-2-yl substituted at the 1-position by naphthalen-1-yl; or a salt thereof or a hydrate of them.

7. A compound of formula (III):

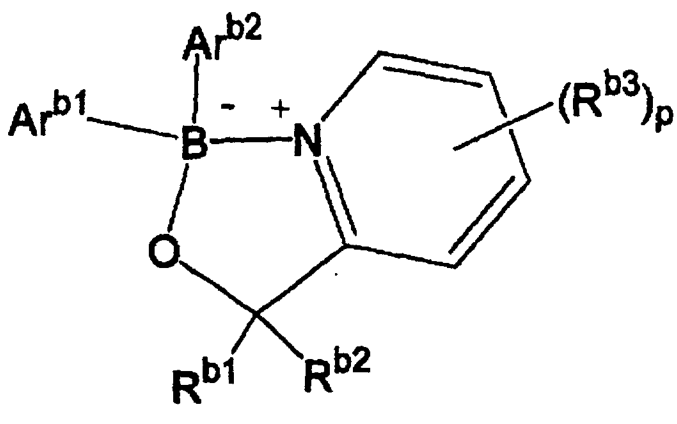

(III)

in which

$Ar^{b1}$ and $Ar^{b2}$ are the same or different, and are an aryl which may be optionally substituted;
$R^{b1}$ and $R^{b2}$ are independently a hydrogen atom, a halogen atom, a lower alkyl, a lower alkenyl, a halogenated lower alkyl, a halogenated lower alkenyl, an aralkyl which may be optionally substituted, hydroxy, a lower

alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{b1}$ and $R^{b2}$ are combined together to form an oxo, a methylene which may be optionally substituted or an imino which may be optionally substituted;

$R^{b3}$ is each a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be bonded with the adjacent group to form a fused ring; and

p is an integer of 0-4;

provided that when p is 0, both $R^{b1}$ and $R^{b2}$ are hydrogen, and $Ar^{b1}$ and $Ar^{b2}$ is a phenyl substituted with a halogen atom or a lower alkyl, then the phenyl of $Ar^{b1}$ and $Ar^{b2}$ is additionally substituted with a substituent other than a halogen atom or a lower alkyl; or a salt thereof or a hydrate of them.

**8.** A compound of formula (IV):

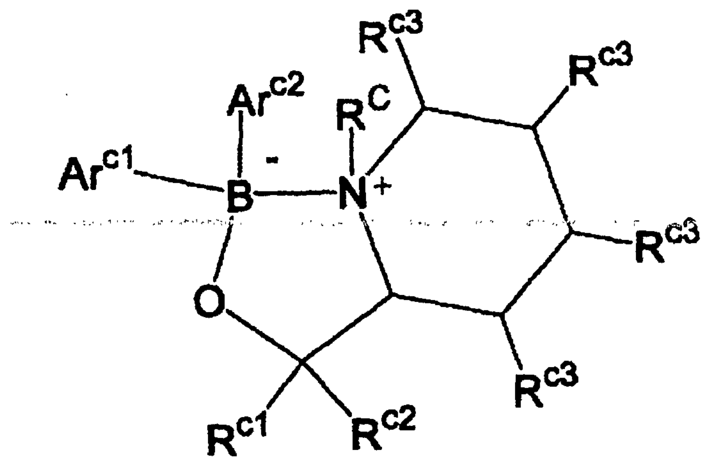

(IV)

in which

$Ar^{c1}$ and $Ar^{c2}$ are independently a cyclic group which may be optionally substituted;

$R^{c1}$ and $R^{c2}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{c1}$ and $R^{c2}$ are combined together to form an oxo, a methylene which may be optionally substituted or an imino which may be optionally substituted;

$R^{c3}$ is each a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be combined with the adjacent group to form a fused ring; and

$R^c$ is a hydrogen atom or a lower alkyl; or a salt thereof or a hydrate of them.

**9.** A compound of formula (V):

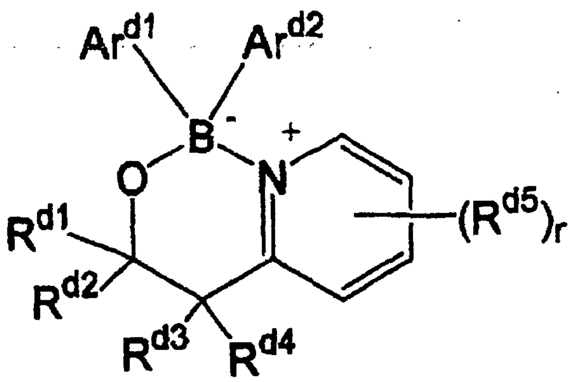

(V)

in which

$Ar^{d1}$ and $Ar^{d2}$ are the same or different, and are an aryl which may be optionally substituted;
$R^{d1}$, $R^{d2}$, $R^{d3}$, and $R^{d4}$ are independently a hydrogen atom, a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or $R^{d1}$ and $R^{d2}$ and/or $R^{d3}$ and $R^{d4}$ are combined together to form an oxo, a methylene which may be optionally substituted or an imino which may be optionally substituted;
$R^{d5}$ is each a halogen atom, a lower alkyl, a lower alkenyl, a halogenated lower alkyl, a halogenated lower alkenyl, an aralkyl which may be optionally substituted, hydroxy, a lower alkoxy, a lower alkenyloxy, a halogenated lower alkoxy, a halogenated lower alkenyloxy, an acyloxy, phenyloxy which may be optionally substituted, an aralkyloxy which may be optionally substituted, an amino group which may be optionally substituted, a sulfo group which may be optionally substituted, or an aryl which may be optionally substituted, or may be combined with the adjacent group to form a fused ring; and
r is an integer of 0-4;

provided that when r is 0, all of $R^{d1}$, $R^{d2}$, $R^{d3}$ and $^{d4}$ are hydrogen, and $Ar^{d1}$ and $Ar^{d2}$ are phenyl substituted with a halogen atom or a lower alkyl, then the phenyl of $Ar^{d1}$ and $Ar^{d2}$ is additionally substituted with a substituent other than a halogen atom or a lower alkyl; or a salt thereof or a hydrate of them.

**10.** A pharmaceutical composition useful for animals except for humans, which comprises a compound of any one of claims 6 to 9.

**11.** A pharmaceutical composition for use as an antiprotozoal agent, which comprises a compound of any one of claims 6 to 9.

**12.** A pharmaceutical composition for use as an anticoccidial agent, which comprises a compound of any one of claims 6 to 9.

## INTERNATIONAL SEARCH REPORT

International application No.
PCT/JP99/07139

A. CLASSIFICATION OF SUBJECT MATTER
Int.Cl[7] A61K31/69, A61P33/02, C07F5/02

According to International Patent Classification (IPC) or to both national classification and IPC

B. FIELDS SEARCHED

Minimum documentation searched (classification system followed by classification symbols)
Int.Cl[7] A61K31/69, A61P33/02, C07F5/02

Documentation searched other than minimum documentation to the extent that such documents are included in the fields searched

Electronic data base consulted during the international search (name of data base and, where practicable, search terms used)
CAPLUS(STN),REGISTRY(STN),MEDLINE(STN),EMBASE(STN)

C. DOCUMENTS CONSIDERED TO BE RELEVANT

| Category* | Citation of document, with indication, where appropriate, of the relevant passages | Relevant to claim No. |
|---|---|---|
| X<br>Y | TRUJILLO,Jose et al. X-ray crystallographic study of boroxazolidones obtained from L-ornithine, L-methionine, kainic acid and 2,6-pyridinedicarboxylic acid, J. Organomet.Chem., 1998, Vol.571, No.1, pp.21-29, especially, page 22 | 7,8<br>1-5,10-12 |
| X<br>Y | HOPFL,Herbert et al, Study of cyclic borinates obtained frompiperidine- and piperazine alcohols by spectroscopic methodsand X-ray crystallography, J. Organomet. Chem., 1998, Vol.553, No.1-2, pp.221-239, especially, pages 222,227 | 8<br>1-5,10-12 |
| X<br>Y | DOROKHOV,V.A.et al, Boron chelate complexes with some enaminones and diketones containing pyridine moiety and their mutual transformation in solutions, Izv. Akad. Nauk, Ser. Khim., 1996, No.3, pp.710-714, especially, page 712 | 7<br>1-5,10-12 |
| X<br>Y | Yuan,Guo-Zheng et al, Single site transarylation of 2,2'-dimetalized 1,1'-binaphthyl to aminochloroborates and synthesis of 2-binaphthyl boron compounds, Youji Huaxue, 1996, Vol.16, No.2, pp.139-144, especially, pages 139,140 | 6<br>1-5,10-12 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 February, 2000 (22.02.00) | 29 February, 2000 (29.02.00) |
| Name and mailing address of the ISA/<br>Japanese Patent Office | Authorized officer |
| Facsimile No. | Telephone No. |

Form PCT/ISA/210 (second sheet) (July 1992)

## INTERNATIONAL SEARCH REPORT

International application No.
PCT/JP99/07139

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | US, 5348947, A (PATEL,Bomi P.), 20 September, 1994 (20.09.94) (Family: none) the whole document | 7<br>1-5,10-12 |
| X<br>Y | US, 5348947, A (PATEL,Bomi P.), 20 September, 1994 (20.09.94) (Family: none) the whole document | 7<br>1-5,10-12 |
| X<br>Y | TORRES,Luis Alfonso et al, Rotating-bomb combustion calorimetry and the standard enthalpies of formation of two borinic esters, J. Chem. Thermodyn. Vol.1994, No.26, No.4, pp.337-43, especially, page 338 | 7,9<br>1-5,10-12 |
| X<br>Y | FARFAN,Norberto et al, Through-bond modulation of N .fwdarw.B ring formation shown by NMR and x-ray diffraction studies of borate derivatives of pyridyl alcohols, J. Chem. Soc., Perkin Trans. 2, 1992, No.4, pp.527-32, especially, page 527 | 7,9<br>1-5,10-12 |
| X<br>Y | LIU,Xiaolan et al, Structure studies of bis(substituted)-2-(substituted)-8-hydroxyquinolines, Youji Huaxue, 1991, Vol.11, No.4, pp.410-15, especially, pages 412,414 | 6<br>1-5,10-12 |
| X<br>Y | SHAN,Zixing et al, Synthesis of aromatic nitrogen-containingheterocyclic derivatives of asymmetric diarylborinic acids, Wuhan Daxue Xuebao, Ziran Kexueban, 1990, No.3, pp.67-72, especially, page 68 | 6,7<br>1-5,10-12 |
| X<br>Y | YUAN,Guozheng et al, Ligand substitution reaction of diarylboron chelates, Wuji Huaxue Xuebao, 1990, Vol.6, No.3, pp.314-18, especially, pages 314,315 | 6<br>1-5,10-12 |
| X<br>Y | YUAN,Guozheng et al, Studies on antitumor boron compounds. V. Fluorine- and methoxy-substituted diphenylboron chelates with N,O-bidentate ligands, Youji Huaxue, 1989, Vol.9, No.3, pp.226-9, especially, page 227 | 6<br>1-5,10-12 |
| X<br>Y | YUAN,Guozheng et al, Boron compounds. XX. Synthesis and conversion of ethanolamine-tri(2-furyl)borane, Youji Huaxue 1987, No.2, pp.146-9, especiallty, page 148 | 6<br>1-5,10-12 |
| X<br>Y | MOEHRLE,H.et al, Concurrent reaction of the phenol and the 1,3-dicarbonyl function under Mannich conditions, Pharmazie,1985, Vol.40, No.11, pp.767-71, especially, Page 767 | 6<br>1-5,10-12 |
| X<br>Y | MOEHRLE,H.et al, Reaction of functional epoxycarbonyl compounds opposed to hydrogen chloride, Pharmazie, 1985, Vol.40, No.6, pp.387-93, especially, page 389 | 6<br>1-5,10-12 |
| X<br>Y | MOEHRLE,H.et al, Real structures of drugs seemingly derivedfrom-(8-hydroxyquinol-5-yl)-3-phenylpropane-1, 3-dione, Pharmazie, 1985, Vol.40, No.5, pp.307-11, especially, page 309 | 6<br>1-5,10-12 |
| X<br>Y | LIN,Kai et al, Synthesis and antitumor activity of organyloxy-diarylborane chelates containing quinoline ring, Yiyao Gongye, 1985, Vol.16, No.11, pp.500-2, especially, page 501 | 6<br>1-5,10-12 |

Form PCT/ISA/210 (continuation of second sheet) (July 1992)

**INTERNATIONAL SEARCH REPORT**

International application No.
PCT/JP99/07139

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | BAILEY,P.J.et al, Boron-containing antibacterial agents: effects on growth and morphology of bacteria under various culture conditions, Antimicrob. Agents Chemother., 1980, Vol.17, No.4, pp.549-53, especially, Fig.1 | 6<br>1-5,10-12 |
| X<br>Y | MOEHRLE,H.et al, Conformation influences during the dehydrogenation of phenolic Mannich bases, Monatsh. Chem., 1974, Vol.105, No.6, pp.1151-63, especially, page 1156 | 6<br>1-5,10-12 |
| X<br>Y | TITKOV,Yu.B.et al, Luminescence method for determining molybdenum with hydroxyquinoline and sodium tetraphenylborate, Ukr. Khim. Zh., 1970, Vol.36, No.6, pp.613-15, especially, page 614 | 6<br>1-5,10-12 |
| X<br>Y | DOUGLASS,James E.et al, Some bis-amine complexes of boroniumions with bulky substituents of boron, J. Organomet. Chem., 1967, Vol.8, No.3, pp.421-6, especially, page 421 | 6<br>1-5,10-12 |
| X<br>Y | DE, 1670494, A (Chemie Grünenthal GmbH), 21 January, 1971 (21.01.71) (Family: none) page 2, line 6 to page 4 | 1-12 |
| X<br>Y | EP, 969531, A2 (Bayer Aktiengesellschaft), 05 January, 2000 (05.01.00), Par. Nos. [0046] to [0079]; Claim 10 & DE, 19829947, A1 | 6 |

Form PCT/ISA/210 (continuation of second sheet) (July 1992)